# EUROPEAN PATENT APPLICATION

(11) **EP 4 512 801 A1**
(43) Date of publication of application: **26.02.2025**
(21) Application number: 23831552.7
(22) Date of filing: 29.06.2023
(51) Int. Cl.: C07D 215/24, A61K 31/4704, A61P 5/14, A61P 5/16, A61P 27/02, C07D 215/38

(54) **3,4-DIHYDROQUINOLIN-2(1H)-ONE COMPOUND**

(30) Priority: 30.06.2022 JP 2022106016
(71) Applicant: Kissei Pharmaceutical Co., Ltd., Matsumoto-Shi, Nagano 399-8710 (JP)
(72) Inventor: MUTAI, Yosuke, Azumino-shi, Nagano 399-8304 (JP); ISHIKAWA, Takehiro, Azumino-shi, Nagano 399-8304 (JP); ITOU, Tatsuya, Azumino-shi, Nagano 399-8304 (JP); MATSUMOTO, Kyohei, Azumino-shi, Nagano 399-8304 (JP); TANADA, Fumiya, Azumino-shi, Nagano 399-8304 (JP)
(74) Representative: Isarpatent
(86) International application number: PCT/JP2023/024105
(87) International publication number: WO 2024/005113

(57) **Abstract**

An object of the present invention is to provide a novel compound which has thyroid-stimulating hormone receptor antagonist activity and is useful for treating thyroid-related diseases.

The present invention relates to a 3,4-dihydroquinolin-2(1H)-one compound represented by the following Formula (I) or a pharmacologically acceptable salt thereof. The compound of the present invention or a pharmacologically acceptable salt thereof have antagonist activity against a thyroid-stimulating hormone receptor and is useful as a therapeutic agent for thyroid-related diseases (for example, hyperthyroidism, Graves' disease, thyroid eye disease and thyroid cancer).

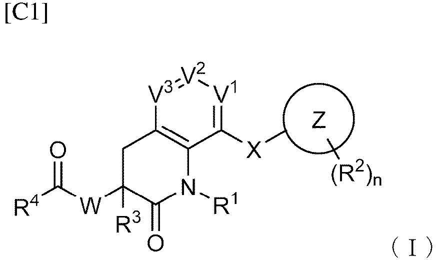

## Description

### [Technical Field]

The present invention relates to a 3,4-dihydroquinolin-2(1H)-one compound useful as a pharmaceutical. More specifically, the present invention relates to a 3,4-dihydroquinolin-2(1H)-one compound or a pharmacologically acceptable salt thereof, which have antagonist activity against a thyroid-stimulating hormone receptor (TSHR) and are useful as a therapeutic agent for thyroid-related diseases.

### [Background Art]

The thyroid hormones triiodothyronine (T3) and thyroxine (T4) play important roles in development, growth, and metabolism, and their synthesis and secretion are precisely regulated by the thyroid-stimulating hormone (TSH) secreted by the pituitary gland.

In hyperthyroidism, these thyroid hormones are excessively secreted for some reason, and the hormone action thereof causes various undesirable effects on the mind and body, such as goiter, tachycardia, high blood pressure, fatigue, weight loss, palpitation, sleep disorders, and menstrual irregularities.

There are various causes of hyperthyroidism, and among these, Graves' disease (Basedow's disease) is the most common. In Graves' disease, due to the autoimmune mechanism, one's own thyroid gland is recognized as a foreign body, and autoantibodies against TSHR present on thyroid follicular cells, called TSHR antibodies (TRAb), are produced. It is thought that this TRAb acts as an agonist on TSHR, and when TSHR is excessively stimulated, more thyroid hormones than necessary are secreted, and hyperthyroidism develops.

In addition, as a disease associated with Graves' disease, a thyroid eye disease is also known. The thyroid eye disease is an autoimmune inflammatory disease that exhibits various ocular symptoms, and is thought to be mainly caused by the agonist action of TRAb on TSHR in the orbital tissue. It often develops at roughly the same time as hyperthyroidism, but may not be associated with thyroid dysfunction.

Currently, antithyroid drugs such as thiamazole and propylthiouracil, which inhibit biosynthesis of thyroid hormones, are used for drug therapy for Graves' disease, but they have problems of a low remission rate, a long treatment period until remission is achieved, and a high frequency of side effects. Therefore, there is currently a need for a therapeutic agent for Graves' disease with a new mechanism of action.

Blocking TSHR or inhibiting signal transduction induced through TSHR inhibits thyroid hormone production, secretion and thyroid cell proliferation. Therefore, the TSHR antagonist is thought to have a therapeutic effect for Graves' disease and thyroid eye disease caused by the agonist action of TRAb on TSHR (PTL 1 and 2). NCGC00242364 is known as a TSHR antagonist, and it is found that it has a blood T4 concentration lowering effect in mice in which TSH-releasing hormone (TRH) has been administered and mice in which thyroid stimulating antibodies M22 has been administered (NPL 1).

Compounds having TSHR antagonist activity are described in PTL 1 to 3, and NPL 1.

However, the 3,4-dihydroquinolin-2(1H)-one compound of the present invention is not mentioned in any of PTL 1 to 3 and NPL 1.

### [Citation List]

### [Patent Literature]

[PTL 1] U.S. Patent Application Publication No. 2011/0172267
[PTL 2] U.S. Patent Application Publication No. 2012/0315217
[PTL 3] U.S. Patent Application Publication No. 2019/0134024

### [Non Patent Literature]

[NPL 1] Susanne Neumann et al., "Endocrinology" 2014, Vol. 155, No. 1, pp. 310-314

### [Summary of Invention]

### [Technical Problem]

An object of the present invention is to provide a novel compound which has TSHR antagonist activity and is useful for treating thyroid-related diseases.

### [Solution to Problem]

The present invention relates to a compound represented by the following Formula (I) or a pharmacologically acceptable salt thereof.

Specifically, the present invention relates to the following [1] to [15] and the like.
[1] A compound represented by Formula (I): 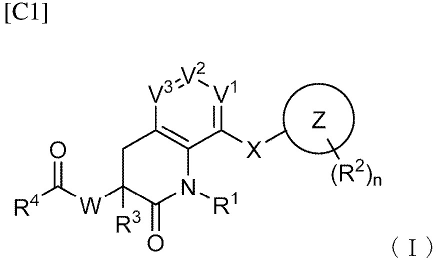 [wherein,
   the ring Z is C₆₋₁₀ aryl, 5- or 6-membered heteroaryl, C₃₋₈ cycloalkyl, or 3- to 8-membered heterocycloalkyl;
   X is -CHR^{X}- or -O-;
   R^{X} is a hydrogen atom or C₁₋₆ alkyl;
   V¹ is =CR^{V1}- or =N-;
   V² is =CR^{V2}- or =N-;
   V³ is =CR^{V3}- or =N-;
   R^{V1}, R^{V2}, and RV³ are each independently a hydrogen atom, a halogen atom, a hydroxy group, C₁₋₆ alkyl, C₁₋₆ alkoxy, halo C₁₋₆ alkyl, hydroxy C₁₋₆ alkyl, C₆₋₁₀ aryl C₁₋₆ alkyl, or C₆₋₁₀ aryl C₁₋₆ alkoxy;
   W is -CH₂- or -NH-;
   R¹ is a hydrogen atom or C₁₋₆ alkyl;
   R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
   n is an integer of 0 to 3;
   when n is 2 or 3, respective R²s may be the same as or different from each other;
   R³ is a hydrogen atom, a halogen atom or C₁₋₆ alkyl;
   R⁴ is C₁₋₆ alkyl, halo C₁₋₆ alkyl, -NR⁵R⁵', or C₃₋₈ cycloalkyl; and
   R⁵ and R⁵' are each independently a hydrogen atom or C₁₋₆ alkyl] or a pharmacologically acceptable salt thereof.
[2] The compound according to [1], which is represented by Formula (II): 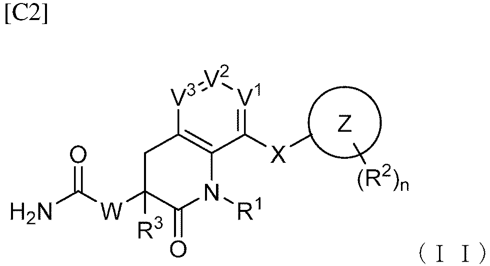 [wherein,
   the ring Z is C₆₋₁₀ aryl, 5- or 6-membered heteroaryl, C₃₋₈ cycloalkyl, or 3- to 8-membered heterocycloalkyl;
   X is -CHR^{X}- or -O-;
   R^{X} is a hydrogen atom or C₁₋₆ alkyl;
   V¹ is =CR^{V1}- or =N-;
   V² is =CR^{V2}- or =N-;
   V³ is =CR^{V3}- or =N-;
   R^{V1}, R^{V2}, and R^{V3} are each independently a hydrogen atom, a halogen atom, a hydroxy group, C₁₋₆ alkyl, C₁₋₆ alkoxy, halo C₁₋₆ alkyl, C₆₋₁₀ aryl C₁₋₆ alkyl, or C₆₋₁₀ aryl C₁₋₆ alkoxy;
   W is -CH₂- or -NH-;
   R¹ is a hydrogen atom or C₁₋₆ alkyl;
   R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
   n is an integer of 0 to 3;
   when n is 2 or 3, respective R²s may be the same as or different from each other; and
   R³ is a hydrogen atom, a halogen atom, or C₁₋₆ alkyl]
   or a pharmacologically acceptable salt thereof.
[3] The compound according to [1] or [2],
   wherein V¹ is =CR^{V1}-;
   V² is =CR^{V2}-;
   V³ is =CR^{V3}-; and
   R^{V1}, R^{V2} and R^{V3} have the same meanings as in [1] or [2];
   or a pharmacologically acceptable salt thereof.
[4] The compound according to any one of [1] to [3]:
   wherein the ring Z is C₆₋₁₀ aryl;
   or a pharmacologically acceptable salt thereof.
[5] The compound according to any one of [1] to [4], which is represented by Formula (III): 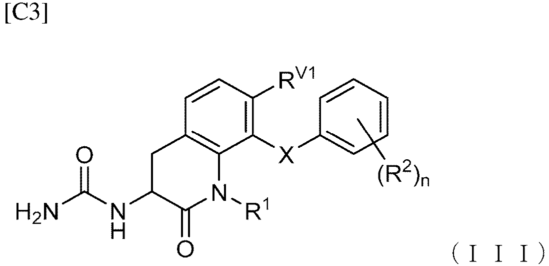 [wherein,
   X is -CHR^{X}- or -O-;
   R^{X} is a hydrogen atom or C₁₋₆ alkyl;
   R^{V1} is a hydrogen atom, a halogen atom, a hydroxy group, C₁₋₆ alkyl, C₁₋₆ alkoxy, halo C₁₋₆ alkyl, C₆₋₁₀ aryl C₁₋₆ alkyl, or C₆₋₁₀ aryl C₁₋₆ alkoxy;
   R¹ is a hydrogen atom or C₁₋₆ alkyl;
   R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
   n is an integer of 0 to 3;
   when n is 2 or 3, respective R²s may be the same as or different from each other]
   or a pharmacologically acceptable salt thereof.
[6] The compound according to any one of [1] to [5]:
   wherein R^{V1} is a hydrogen atom, a halogen atom, a hydroxy group, C₁₋₆ alkyl, C₁₋₆ alkoxy, or halo C₁₋₆ alkyl;
   R¹ is a C₁₋₆ alkyl; and
   R² is a halogen atom, C₁₋₆ alkyl, halo C₁₋₆ alkyl, C₁₋₆ alkoxy or halo C₁₋₆ alkoxy;
   or a pharmacologically acceptable salt thereof.
[7] The compound according to any one of [1] to [3], which is represented by Formula (IV): 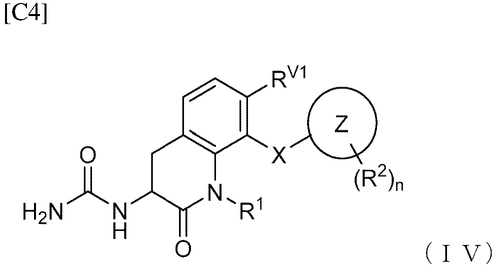 [wherein,
   the ring Z is C₆₋₁₀ aryl, 5- or 6-membered heteroaryl or C₃₋₈ cycloalkyl;
   X is -CHR^{X}- or -O-;
   R^{X} is a hydrogen atom or C₁₋₆ alkyl;
   R^{V1} is a hydrogen atom, a halogen atom, a hydroxy group, C₁₋₆ alkyl, halo C₁₋₆ alkyl or C₆₋₁₀ aryl C₁₋₆ alkoxy;
   R¹ is a C₁₋₆ alkyl;
   R² is a halogen atom, a cyano group, C₁₋₆ alkyl, halo C₁₋₆ alkyl, C₁₋₆ alkoxy or halo C₁₋₆ alkoxy;
   n is an integer of 0 to 3;
   when n is 2 or 3, respective R²s may be the same as or different from each other]
   or a pharmacologically acceptable salt thereof.
[8] The compound according to [7]:
   wherein the ring Z is C₆₋₁₀ aryl;
   or a pharmacologically acceptable salt thereof.
[9] The compound according to any one of [1] to [8]:
   wherein X is -O-;
   or a pharmacologically acceptable salt thereof.
[10] The compound according to any one of [1] to [9], which is selected from the group consisting of the following compounds: 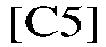 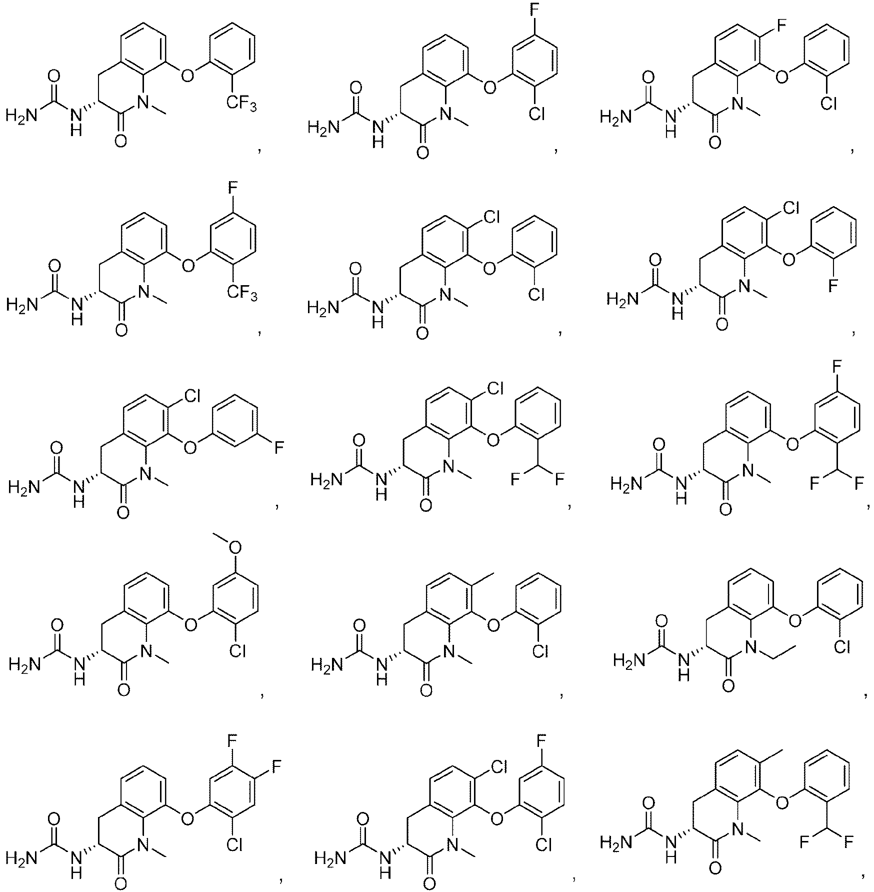 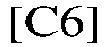 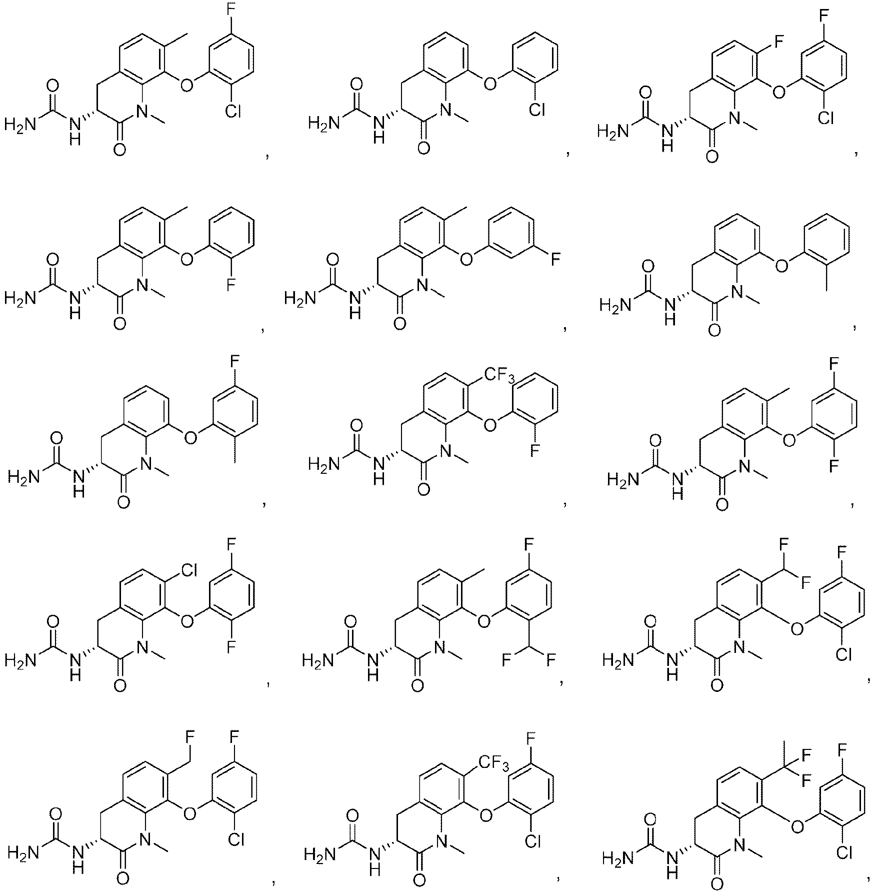 and 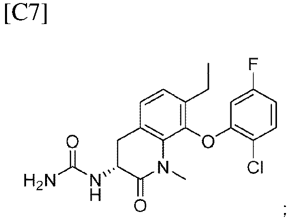 or a pharmacologically acceptable salt thereof.
[11] The compound according to any one of [1] to [8]:
   wherein X is -CHR^{X}-;
   R^{X} has the same meaning as in [7];
   or a pharmacologically acceptable salt thereof.
[12] The compound according to any one of [1] to [8] or [11], which is selected from the group consisting of the following compounds: 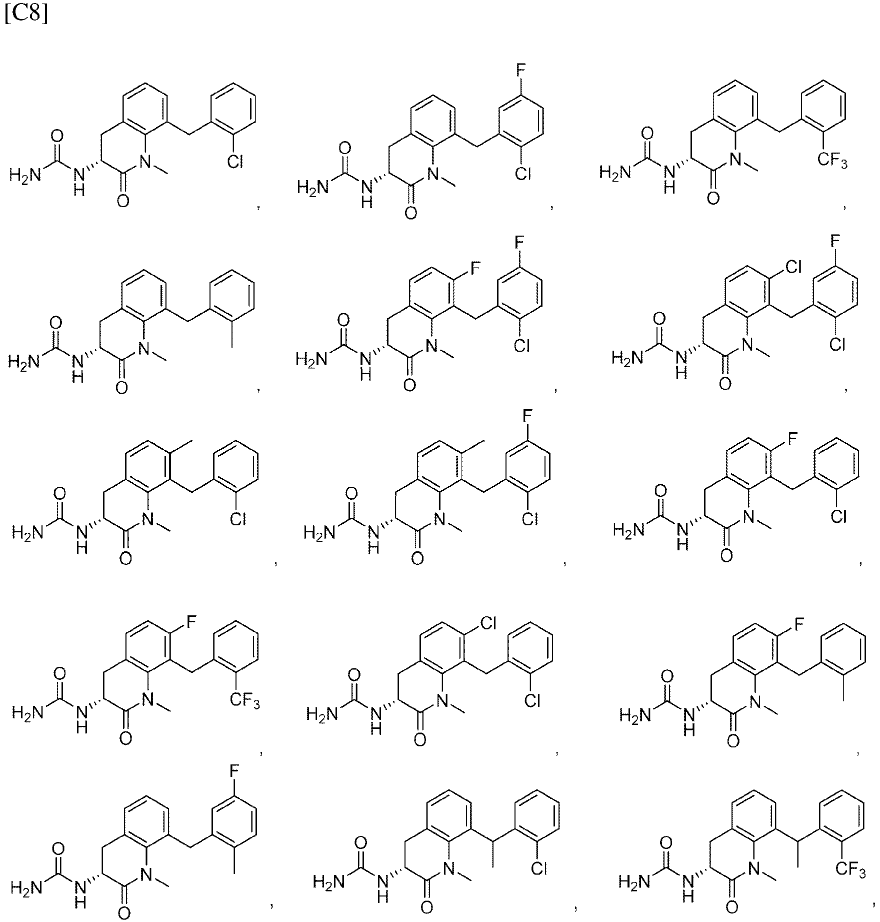 and 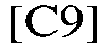 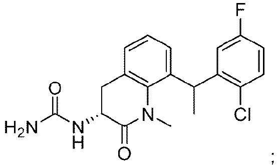 or a pharmacologically acceptable salt thereof.
[13] A pharmaceutical composition including the compound according to any one of [1] to [12] or a pharmacologically acceptable salt thereof, and a pharmaceutical additive.
[14] The pharmaceutical composition according to [13], which is a pharmaceutical composition for treating thyroid-related diseases.
[15] The pharmaceutical composition according to [14],
   wherein the thyroid-related diseases are hyperthyroidism, Graves' disease, or thyroid eye disease.

One embodiment of the present invention relates to a method of treating thyroid-related diseases including administering a required amount of the pharmaceutical composition according to [13] to a patient.

One embodiment of the present invention relates to a use of the compound according to any one of [1] to [12] or a pharmacologically acceptable salt thereof for producing a pharmaceutical composition for treating thyroid-related diseases.

One embodiment of the compound represented by Formula (I) is, for example, a compound represented by the following Formula (V): 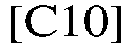 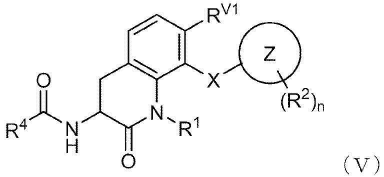 [wherein,
the ring Z is C₆₋₁₀ aryl, 5- or 6-membered heteroaryl or C₃₋₈ cycloalkyl;
X is -CHR^{X}- or -O-;
R^{X} is a hydrogen atom or C₁₋₆ alkyl;
R^{V1} is a hydrogen atom, a halogen atom, a hydroxy group, C₁₋₆ alkyl, halo C₁₋₆ alkyl, hydroxy C₁₋₆ alkyl or C₆₋₁₀ aryl C₁₋₆ alkoxy;
R¹ is a C₁₋₆ alkyl;
R² is a halogen atom, a cyano group, C₁₋₆ alkyl, halo C₁₋₆ alkyl, C₁₋₆ alkoxy or halo C₁₋₆ alkoxy;
n is an integer of 0 to 3;
when n is 2 or 3, respective R²s may be the same as or different from each other;
R⁴ is C₁₋₆ alkyl, halo C₁₋₆ alkyl, -NR⁵R⁵', or C₃₋₈ cycloalkyl; and
R⁵ and R⁵' are each independently a hydrogen atom or C₁₋₆ alkyl]

### [Advantageous Effects of Invention]

The compound of the present invention has excellent TSHR antagonist activity. Therefore, the compound of the present invention or a pharmacologically acceptable salt thereof are useful as a therapeutic agent for thyroid-related diseases.

### [Description of Embodiments]

Hereinafter, embodiments of the present invention will be described in more detail.

In the present invention, unless otherwise specified, respective terms have the following meanings.

"Halogen atom" refers to a fluorine atom, a chlorine atom, a bromine atom or an iodine atom.

"C₁₋₆ alkyl" refers to a linear or branched alkyl group having 1 to 6 carbon atoms. Examples thereof include methyl, ethyl, propyl, isopropyl, butyl, isobutyl, sec-butyl, and tert-butyl.

"C₁₋₆ alkoxy" refers to a linear or branched alkoxy group having 1 to 6 carbon atoms. Examples thereof include methoxy, ethoxy, propoxy, and isopropoxy.

"Hydroxy C₁₋₆ alkyl" refers to C₁₋₆ alkyl substituted with one or two hydroxy groups. Examples thereof include hydroxymethyl, 2-hydroxyethyl, 3-hydroxypropyl, and 2-hydroxypropan-2-yl.

"Halo C₁₋₆ alkyl" refers to C₁₋₆ alkyl substituted with 1 to 5 identical or different halogen atoms. Examples thereof include monofluoromethyl, 2-fluoroethyl, difluoromethyl, trifluoromethyl, 1,1-difluoroethyl, 2,2,2-trifluoroethyl, 3,3,3-trifluoropropyl, 4,4,4-trifluorobutyl, and pentafluoroethyl.

"Halo C₁₋₆ alkoxy" refers to C₁₋₆ alkoxy substituted with 1 to 5 identical or different halogen atoms. Examples thereof include monofluoromethoxy, difluoromethoxy, trifluoromethoxy, 1,1,2,2-tetrafluoroethoxy, and pentafluoroethoxy.

"C₆₋₁₀ aryl" refers to a phenyl group or a naphthyl group.

"5- or 6-membered heteroaryl" refers to a 5- or 6-membered aromatic heterocyclic group including 1 to 4 heteroatoms selected from among an oxygen atom, a nitrogen atom and a sulfur atom in the ring. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, and 1,2,4-oxadiazolyl.

"C₃₋₈ cycloalkyl" refers to a 3- to 8-membered saturated hydrocarbon group, and includes those having a partially crosslinked structure. Examples thereof include cyclopropyl, cyclobutyl, cyclopentyl, cyclohexyl, bicyclo[1.1.1]pentyl, and bicyclo[2.2.2]octyl.

"3- to 8-membered heterocycloalkyl" refers to a cycloalkyl group in which carbon atoms in the ring are substituted with 1 or 2 heteroatoms selected from among an oxygen atom, a nitrogen atom and a sulfur atom, and includes those having a partially crosslinked structure. Examples thereof include aziridinyl, azetidinyl, pyrrolidinyl, piperidinyl, piperazinyl, morpholinyl, thiomorpholinyl, azepanyl, azabicyclo[2.2.2]octyl, 2-oxa-5-azabicyclo[2.2.1]heptanyl, 6-oxa-3-azabicyclo[2.2.1]heptanyl, 8-oxa-3-azabicyclo[3.2.1]octanyl, 3-oxa-8-azabicyclo[3.2.1]octanyl, 2-azaspiro[3.3]heptanyl, 2-oxa-6-azaspiro[3.3]heptanyl, pyrrolidonyl, piperidonyl, oxiranyl, oxetyl, tetrahydrofuranyl, and tetrahydropyranyl.

"C₆₋₁₀ aryl C₁₋₆ alkoxy" refers to C₁₋₆ alkoxy substituted with one C₆₋₁₀ aryl. Examples thereof include benzyloxy.

"C₆₋₁₀ aryl C₁₋₆ alkyl" refers to C₁₋₆ alkyl substituted with one C₆₋₁₀ aryl. Examples thereof include benzyl.

The following abbreviations in the text, drawings and tables have the following meanings.
Boc: tert-butoxycarbonyl
Boc₂O: di-tert-butyl dicarbonate
DIPEA: N,N-diisopropylethylamine
DCM: dichloromethane
DMAP: 4-dimethylaminopyridine
DMF: N,N-dimethylformamide
DPPA: diphenylphosphoryl azide
HATU: O-(7-azabenzotriazol-1-yl)-N,N,N',N'-tetramethyluronium hexafluorophosphate
LDA: lithium diisopropylamide
MeCN: acetonitrile
MTBE: methyl tert-butyl ether
NMP: N-methylpyrrolidone
TEA: triethylamine
TFA: trifluoroacetic acid
THF: tetrahydrofuran
Xphos: 2-dicyclohexylphosphino-2',4',6'-triisopropylbiphenyl
10% Pd/C: 10% palladium on carbon (wetted with about 55% water)
10% Pt/C: 10% platinum carbon (wetted with about 55% water)
APS: aminopropylated silica gel
ODS: octadecylsilylated silica gel
Method A: column chromatography using a silica gel column connected to the bottom of an aminopropylated silica gel column
Ref. No.: Reference Example Number
Str.: structural formula
Ex. No.: Example Number
Phys. data: physical property values
IC₅₀: 50% inhibitory concentration
¹H-NMR: proton nuclear magnetic resonance spectrum
DMSO: dimethyl sulfoxide
DMSO-d6: dimethyl sulfoxide-d6
CDCl₃: chloroform-d1
MS: mass spectrometry
(the measured values in the table were measured by a multi-ionization method including an electrospray ionization method and an atmospheric pressure chemical ionization method)
cAMP: adenosine 3',5'-cyclic monophosphate
CHO: Chinese hamster ovary
FBS: fetal bovine serum
HEPES: 2-(4-(2-hydroxyethyl)-1-piperazinyl)ethanesulfonic acid
IBMX: 3-isobutyl-1-methylxanthine

In the compounds represented by Formulae (I) to (V), when one or more asymmetric carbon atoms are present, the present invention also includes a compound in which asymmetric carbon atoms are in the R configuration, a compound in which asymmetric carbon atoms are in the S configuration, and any combination of these compounds. In addition, the scope of the present invention also includes racemic compounds, racemic mixtures, single enantiomers and diastereomeric mixtures thereof.

In the compounds represented by Formulae (I) to (V), when cis-trans isomers are present, the present invention includes both of the cis-trans isomers.

In the compounds represented by Formulae (I) to (V), when tautomers are present, the present invention includes any of the tautomers.

In the present invention, the determination of stereochemistry can be performed by a method known in the art.

As necessary, the compound represented by Formulae (I) to (V) can be converted into a pharmacologically acceptable salt thereof according to a conventional method. Examples of such salts include acid addition salts and salts with bases.

Examples of acid addition salts include acid addition salts with mineral acids such as hydrochloric acid, hydrobromic acid, hydroiodic acid, sulfuric acid, nitric acid, and phosphoric acid, and acid addition salts with organic acids such as formic acid, acetic acid, trifluoroacetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, benzoic acid, glutamic acid, and aspartic acid.

Examples of salts with bases include salts with inorganic bases such as lithium salts, sodium salts, potassium salts, calcium salts, and magnesium salts, and salts with organic bases such as N-methyl-D-glucamine, N,N'-dibenzylethylenediamine, TEA, piperidine, morpholine, pyrrolidine, arginine, lysine, and choline.

Unless otherwise specified, for example, suffixes "hydrochloride," or "HCl" to chemical names or structural formulas for salts are not stoichiometric descriptions, but simply refer to salt forms.

When the compounds represented by Formulae (I) to (V) or a pharmacologically acceptable salt thereof are present as, for example, a crystal, the present invention also includes any crystal form. For example, pharmacologically acceptable salts also include solvates with pharmaceutically acceptable solvents such as water or ethanol, and cocrystals with appropriate cocrystal formers (Coformer).

In the compounds represented by Formulae (I) to (V), some atoms may be substituted with corresponding isotopes. The present invention also includes compounds substituted with these isotopes. Examples of isotopes include isotopes of a hydrogen atom, a carbon atom, a chlorine atom, a fluorine atom, an iodine atom, a nitrogen atom, an oxygen atom, and a sulfur atom represented by ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, and ³⁵S. As one embodiment, compounds in which some hydrogen atoms of compounds represented by Formulae (I) to (V) are substituted with ²H (D: deuterium atom) may be exemplified.

In the compounds represented by Formulae (I) to (V), compounds in which some atoms are substituted with isotopes can be produced by the same method as a production method to be described below using commercially available building blocks into which isotopes are introduced. In addition, methods described in the literature (for example, refer to Journal of Synthetic Organic Synthesis, Vol. 65, No. 12, pp. 1179-1190, 2007 and refer to RADIOISOTOPES, Vol. 56, No. 11, pp. 741-750, 2007) can be used for production.

The compounds represented by Formulae (I) to (V) of the present invention can be produced, for example, by methods shown in Schemes 1 to 8 or methods similar thereto, or by methods described in the literature or methods similar thereto. In the schemes, the compounds represented by Formulae (I) to (V) correspond to compounds represented by Formulae (I-1) to (I-7).

The compounds represented by Formulae (I) to (V) of the present invention can be produced by the following methods, but the following production methods are simply general production methods, and are not intended to limit the production methods.

In the reaction of each step, when raw substances and reagents are commercially available, commercially available products can be used.

In the reaction of each step, the reaction time varies depending on the raw substances used, solvent, reaction temperature and the like, but is generally 30 minutes to 3 days unless otherwise specified.

In the reaction of each step, the reaction temperature varies depending on the raw substances used, solvent and the like, but is generally -78°C to a reflux temperature unless otherwise specified.

In the reaction of each step, the pressure varies depending on the raw substances used, solvent, reaction temperature and the like, but is generally 1 to 20 atm unless otherwise specified.

In the reaction of each step, a microwave reaction device such as an Initiator (commercially available from Biotage) can be used. When the reaction is performed using a microwave reaction device, the reaction can be performed under varying conditions depending on the raw substances used, solvent, and model, pressure range: 1 to 30 bar, power range: 1 to 400 W, reaction temperature: room temperature to 300°C, and reaction time: 1 minute to 1 day.

Unless otherwise specified, the reaction in each step is performed without a solvent or using an appropriate solvent. Examples of appropriate solvents include solvents inert to the reaction. Specific examples of solvents used include solvents described in reference examples and examples corresponding to the steps and the following solvents. Two or more of the following solvents may be used in combination at an appropriate ratio. Alcohols:
methanol, ethanol, tert-butyl alcohol, 2-propanol, etc.;
ethers: diethyl ether, THF, 1,2-dimethoxyethane, 1,4-dioxane, cyclopentyl methyl ether, MTBE, etc.;
aromatic hydrocarbons: benzene, chlorobenzene, 1,2-dichlorobenzene, toluene, xylene, etc.;
saturated hydrocarbons: cyclohexane, n-hexane, n-pentane, etc.;
amides: DMF, N,N-dimethylacetamide, NMP, etc.;
halogenated hydrocarbons: DCM, 1,2-dichloroethane, carbon tetrachloride, etc.;
nitriles: MeCN, etc.;
sulfoxides: DMSO, etc.;
aromatic organic bases: pyridine, etc.;
acid anhydrides: acetic anhydride, etc.;
organic acids: formic acid, acetic acid, TFA, methanesulfonic acid, etc.;
esters: ethyl acetate, methyl acetate, isopropyl acetate, etc.;
ketones: acetone, methyl ethyl ketone, etc.; and
water.

In the reaction of each step, when a base is used, the reaction is performed using a base suitable for the reaction. Specific examples of bases used include bases described in reference examples and examples corresponding to the steps and the following bases. inorganic bases: sodium hydroxide, lithium hydroxide, potassium hydroxide, etc.;
basic salts: sodium carbonate, sodium bicarbonate, potassium carbonate, cesium carbonate, etc.; organic bases: TEA, DIPEA, diethylamine, pyridine, DMAP, 2,6-lutidine, 1,8-diazabicyclo[5.4.0]-7-undecene, imidazole, piperidine, etc.;
metal alkoxides: sodium ethoxide, sodium methoxide, potassium tert-butoxide, etc.;
alkali metal hydrides: sodium hydride, etc.;
metal amides: sodium amide, LDA, lithium bis(trimethylsilyl)amide, sodium bis(trimethylsilyl)amide, potassium bis(trimethylsilyl)amide, etc.;
organomagnesiums: isopropylmagnesium chloride, etc.;
organolithiums: methyllithium, n-butyllithium, sec-butyllithium, tert-butyllithium, etc.

In the reaction of each step, when an acid is used, the reaction is performed using an acid suitable for the reaction. Specific examples of acids used include acids described in reference examples and examples corresponding to the steps and the following acids.
inorganic acids: hydrochloric acid, sulfuric acid, nitric acid, hydrobromic acid, phosphoric acid, etc.;
organic acids: acetic acid, TFA, citric acid, methanesulfonic acid, p-toluenesulfonic acid, 10-camphorsulfonic acid, etc.; and
Lewis acids: boron trifluoride diethyl ether complex, zinc iodide, aluminum chloride, zinc chloride, titanium(IV) chloride, etc.

In the reaction of each step, when a condensing agent is used, the reaction is performed using a condensing agent suitable for the reaction. Specific examples of condensing agents used include condensing agents described in reference examples and examples corresponding to the steps and the following condensing agents.
carbodiimides: 1-ethyl-3-(3-dimethylaminopropyl)carbodiimide hydrochloride, N,N'-dicyclohexylcarbodiimide, etc.;
imidazoles: carbonyldiimidazole, etc.;
uronium salts, phosphonium salts: HATU, 1H-benzotriazol-1-yloxytris(dimethylamino)phosphonium hexafluorophosphate, etc.;
triazines: 4-(4,6-dimethoxy-1,3,5-triazine-2-yl)-4-methylmorpholinium chloride, etc.;
others: propylphosphonic anhydride (cyclic trimer), etc.

In the reaction of each step, when a reducing agent is used, the reaction is performed using a reducing agent suitable for the reaction. Specific examples of reducing agents used include reducing agents described in reference examples and examples corresponding to the steps and the following reducing agents.
metal hydrides: lithium aluminum hydride, lithium borohydride, sodium borohydride, sodium triacetoxyborohydride, sodium cyanoborohydride, diisobutylaluminum hydride, etc.;
boranes: BH₃-THF complex, picoline borane complex, decaborane, etc.

In the reaction of each step, when a carbonyl group introduction reagent is used, the reaction is performed using a carbonyl group introduction reagent suitable for the reaction. Specific examples of carbonyl group introduction reagents used include carbonyl group introduction reagents described in reference examples and examples corresponding to the steps and the following carbonyl group introduction reagents.
phosgenes: phosgene, diphosgene, triphosgene, etc.;
chloroformate esters: 4-nitrophenyl chloroformate, etc.;
imidazoles: carbonyldiimidazole, etc.

In each step, when a protecting group is required according to the type of functional groups, introduction and removal operations can be performed in an appropriate combination according to a conventional method. Regarding the type of protecting groups, protection, and deprotection, for example, the methods described in "Greene's Protective Groups in Organic Synthesis," fifth edition, Wiley-Interscience, 2014 edited by Peter G. M. Wuts may be referred to.

In each step, when a hydrolysis reaction is performed, the reaction can be performed in the presence of an acid or a base. Examples of acids and bases used include those exemplified above.

In each step, when a catalytic reduction reaction is performed, the reaction can be performed in the presence of hydrogen and a catalyst. Examples of catalysts used include palladium on carbon powder, a Perlman catalyst, platinum carbon powder, and Raney nickel. Here, as necessary, an acid may be used in the reaction.

In each step, when a reduction reaction is performed, the reaction can be performed in the presence of a reducing agent. Examples of reducing agents used include those exemplified above.

In each step, when a metal reduction reaction is performed, the reaction can be performed in the presence of a metal or the like. Examples of metals used or the like include iron powder, zinc powder, tin chloride, and titanium trichloride. Here, as necessary, an acid may be used in the reaction.

In each step, when an amidation reaction is performed, the reaction can be performed using a condensing agent in the presence or absence of a base. Examples of condensing agents and bases used include those exemplified above. Here, when a carbodiimide is used as the condensing agent, the reaction may be performed by adding additives such as 1-hydroxybenzotriazole and DMAP as necessary. In addition, the reaction can be performed using an acyl halide or an acid anhydride in the presence or absence of a base.

In each step, when a reductive amination reaction is performed, the reaction can be performed in the presence of a reducing agent. Examples of reducing agents used include those exemplified above. In addition, the reaction can be performed in the presence of hydrogen and a catalyst. Examples of catalysts used include palladium on carbon powder, a Perlman catalyst, platinum carbon powder, and Raney nickel.

In each step, when an aromatic nucleophilic substitution reaction is performed, the reaction can be performed in the presence of a base. Examples of bases include those exemplified above.

In each step, when a Negishi cross coupling reaction is performed, the reaction can be performed in the presence of an organozinc compound, a palladium catalyst and a ligand. Examples of palladium catalysts used include palladium(II) acetate and tris(dibenzylideneacetone)palladium(0). Examples of ligands include Xphos, 2-dicyclohexylphosphino-2',6'-dimethoxybiphenyl, and tris(2-methylphenyl)phosphine.

In each step, when a Suzuki-Miyaura cross-coupling reaction is performed, the reaction can be performed in the presence of a palladium catalyst and a base. Examples of palladium catalysts used include bis(di-tert-butyl(4-dimethylaminophenyl)phosphine)dichloropalladium(II), and tetrakis(triphenylphosphine)palladium(0). Examples of bases used include those exemplified above.

In each step, when a Curtius rearrangement reaction is performed, the reaction can be performed in the presence of an azide source. Examples of azide sources used include sodium azide and DPPA.

In each step, when a carbamate formation reaction or a urea formation reaction is performed, the reaction can be performed using a carbonyl group introduction reagent in the presence or absence of a base. Examples of carbonyl group introduction reagents and bases used include those exemplified above.

The compound represented by Formula (I-1) can be produced, for example, according to the method described in Scheme 1. 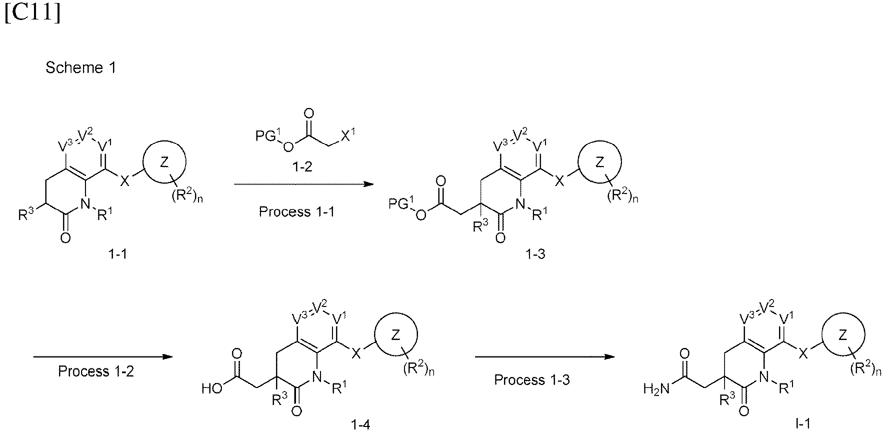 The symbols in the formulae have the same meanings as above. X¹ is a chlorine atom, a bromine atom, or an iodine atom. PG¹ is a protecting group.

### Process 1-1

Compound (1-3) can be produced by reacting Compound (1-1) with Compound (1-2) in the presence of a base.

### Process 1-2

Compound (1-4) can be produced by removing the protecting group of Compound (1-3).

### Process 1-3

Compound (I-1) can be produced by an amidation reaction of Compound (1-4).

The compound represented by Formula (I-2) can be produced, for example, according to the method described in Scheme 2.

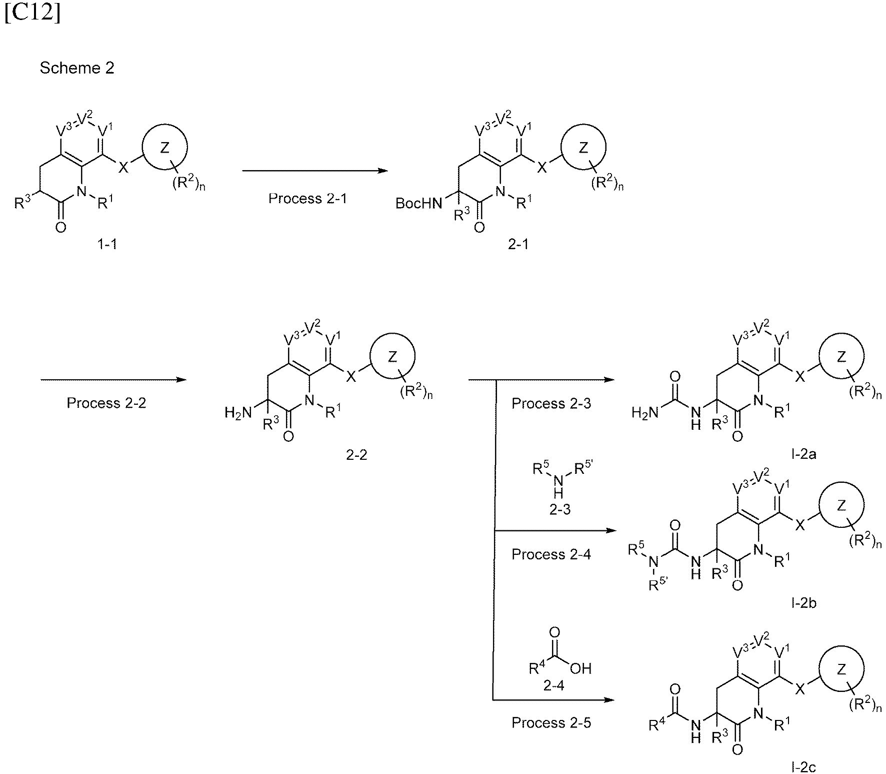

The symbols in the formulae have the same meanings as above.

### Process 2-1

Compound (2-1) can be produced by reacting Compound (1-1) with DPPA and Boc₂O in the presence of a base.

### Process 2-2

Compound (2-2) can be produced by removing the Boc group of Compound (2-1).

### Process 2-3

Compound (I-2a) can be produced by reacting Compound (2-2) with trimethylsilyl isocyanate. As another method, Compound (I-2) can be produced by reacting Compound (2-2) with potassium cyanate or sodium cyanate in the presence of an acid.

### Process 2-4

Compound (I-2b) can be produced by a urea formation reaction of Compound (2-2) and Compound (2-3).

### Process 2-5

Compound (I-2c) can be produced by an amidation reaction of Compound (2-2) and Compound (2-4).

The compound represented by Formula (3-10) can be produced, for example, according to the method described in Scheme 3. 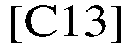 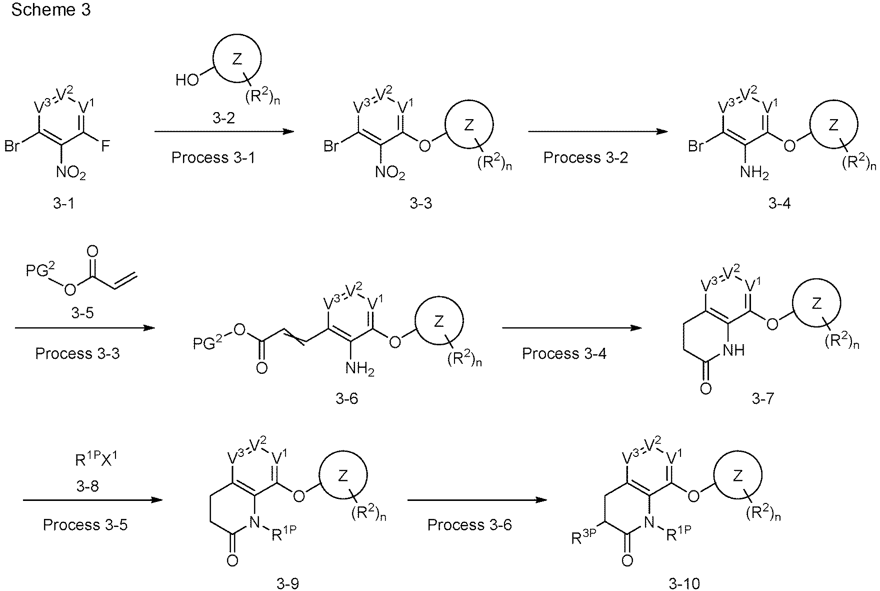 The symbols in the formulae have the same meanings as above. PG² is a protecting group, R^{1P} is C₁₋₆ alkyl, and R^{3P} is a halogen atom or C₁₋₆ alkyl.

### Process 3-1

Compound (3-3) can be produced by an aromatic nucleophilic substitution reaction of Compound (3-1) and Compound (3-2).

### Process 3-2

Compound (3-4) can be produced by a metal reduction reaction of Compound (3-3).

### Process 3-3

Compound (3-6) can be produced by reacting Compound (3-4) with Compound (3-5) in the presence of a palladium catalyst.

### Process 3-4

Compound (3-7) can be produced by a catalytic reduction reaction of Compound (3-6). As another method, Compound (3-7) can be produced by reacting Compound (3-6) with sodium borohydride in the presence of cobalt(II) chloride and copper(II) sulfate and then reacting it with a base.

### Process 3-5

Compound (3-9) can be produced by reacting Compound (3-7) with Compound (3-8) in the presence of a base.

### Process 3-6

Compound (3-10) can be produced by reacting Compound (3-9) with a halogenating agent or an alkylating agent in the presence or absence of a base. As the halogenating agent, N-fluorobenzenesulfonimide, chlorosuccinimide, benzenesulfonyl chloride or the like can be used. As the alkylating agent, methyl iodide, ethyl iodide or the like can be used.

The compound represented by Formula (I-3) can be produced, for example, according to the method described in Scheme 4. 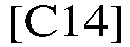 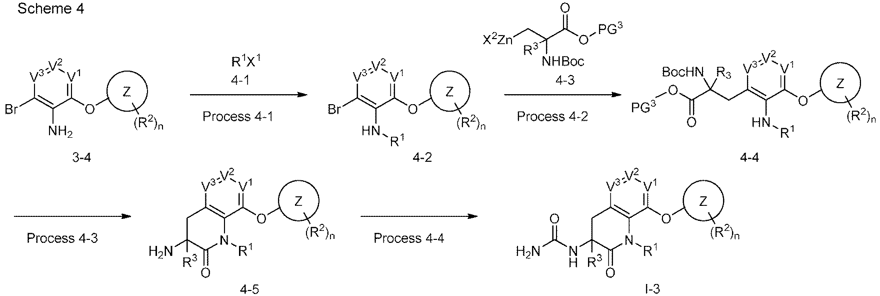 The symbols in the formulae have the same meanings as above. X² is a bromine atom or an iodine atom, and PG³ is a protecting group.

### Process 4-1

Compound (4-2) can be produced by reacting Compound (3-4) with Compound (4-1) in the presence of a base.

### Process 4-2

Compound (4-4) can be produced by a Negishi cross coupling reaction of Compound (4-2) and Compound (4-3).

### Process 4-3

Compound (4-5) can be produced by reacting Compound (4-4) with an acid.

### Process 4-4

Compound (I-3) can be produced by reacting Compound (4-5) with trimethylsilyl isocyanate. As another method, Compound (I-3) can be produced by reacting Compound (4-5) with potassium cyanate or sodium cyanate in the presence of an acid.

The compound represented by Formula (5-4) can be produced, for example, according to the method described in Scheme 5. 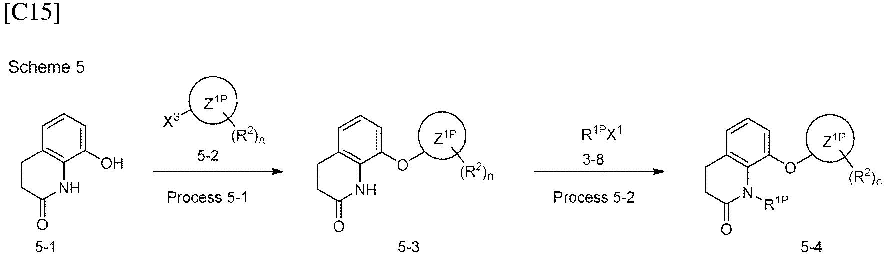 The symbols in the formulae have the same meanings as above. Z^{1P} is 6-membered heteroaryl containing at least one nitrogen atom, and X³ is a fluorine atom or a chlorine atom.

### Process 5-1

Compound (5-3) can be produced by an aromatic nucleophilic substitution reaction of Compound (5-1) and Compound (5-2).

### Process 5-2

Compound (5-4) can be produced by reacting Compound (5-3) with Compound (3-8) in the presence of a base.

The compound represented by Formula (I-4) can be produced, for example, according to the method described in Scheme 6.

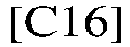

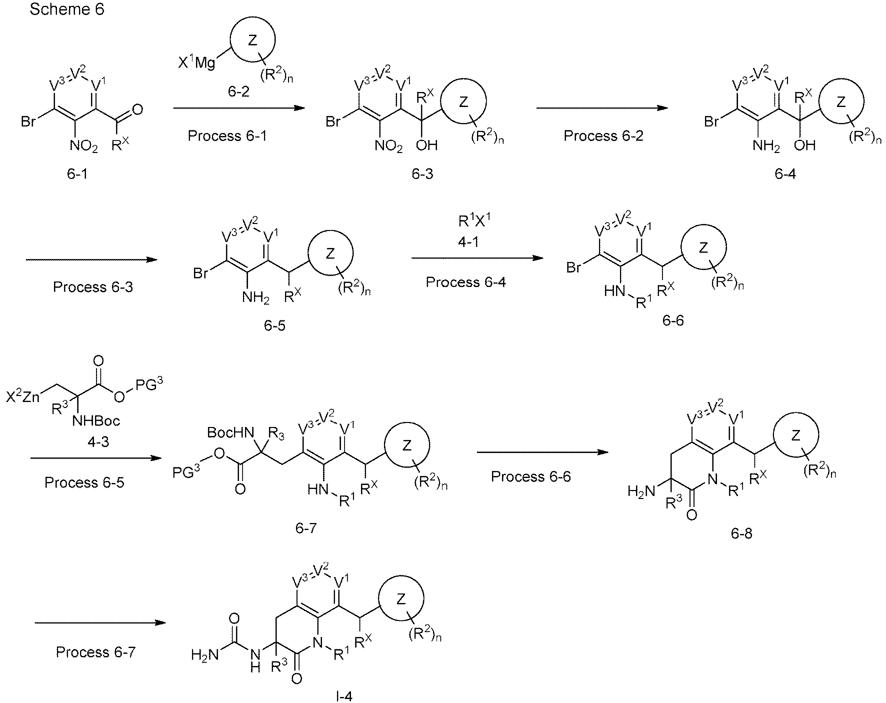

The symbols in the formulae have the same meanings as above.

### Process 6-1

Compound (6-3) can be produced by reacting Compound (6-1) with Grignard reagent (6-2).

### Process 6-2

Compound (6-4) can be produced by a metal reduction reaction of Compound (6-3).

### Process 6-3

Compound (6-5) can be produced by reacting Compound (6-4) with triethylsilane in the presence of an acid.

### Process 6-4

Compound (6-6) can be produced by reacting Compound (6-5) with Compound (4-1) in the presence of a base.

### Process 6-5

Compound (6-7) can be produced by a Negishi cross coupling reaction of Compound (6-6) and Compound (4-3).

### Process 6-6

Compound (6-8) can be produced by reacting Compound (6-7) with an acid.

### Process 6-7

Compound (I-4) can be produced by reacting Compound (6-8) with trimethylsilyl isocyanate. As another method, Compound (I-4) can be produced by reacting Compound (6-8) with potassium cyanate or sodium cyanate in the presence of an acid.

The compound represented by Formula (I-6) can be produced, for example, according to the method described in Scheme 7. 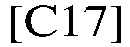 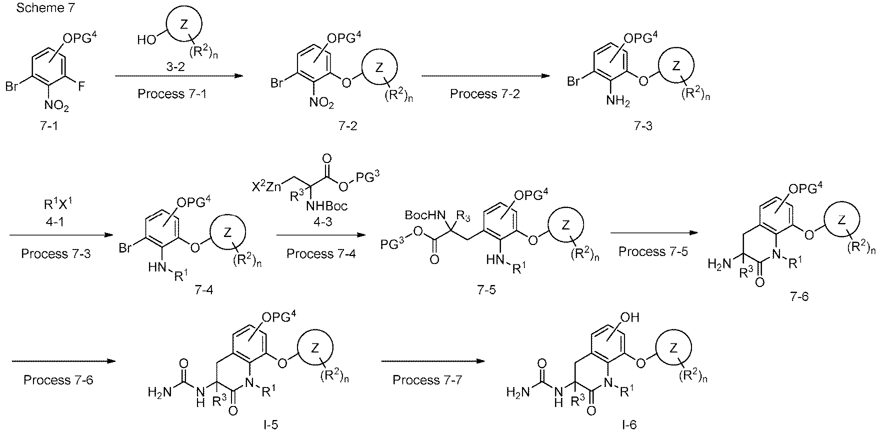 The symbols in the formulae have the same meanings as above. PG⁴ is a protecting group.

### Process 7-1

Compound (7-2) can be produced by an aromatic nucleophilic substitution reaction of Compound (7-1) and Compound (3-2).

### Process 7-2

Compound (7-3) can be produced by a metal reduction reaction of Compound (7-2).

### Process 7-3

Compound (7-4) can be produced by reacting Compound (7-3) with Compound (4-1) in the presence of a base.

### Process 7-4

Compound (7-5) can be produced by a Negishi cross coupling reaction of Compound (7-4) and Compound (4-3).

### Process 7-5

Compound (7-6) can be produced by reacting Compound (7-5) with an acid.

### Process 7-6

Compound (I-5) can be produced by reacting Compound (7-6) with trimethylsilyl isocyanate. As another method, Compound (I-5) can be produced by reacting Compound (7-6) with potassium cyanate or sodium cyanate in the presence of an acid.

### Process 7-7

Compound (I-6) can be produced by removing the protecting group of Compound (I-5).

The compound represented by Formula (I-7) can be produced, for example, according to the method described in Scheme 8.

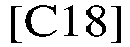

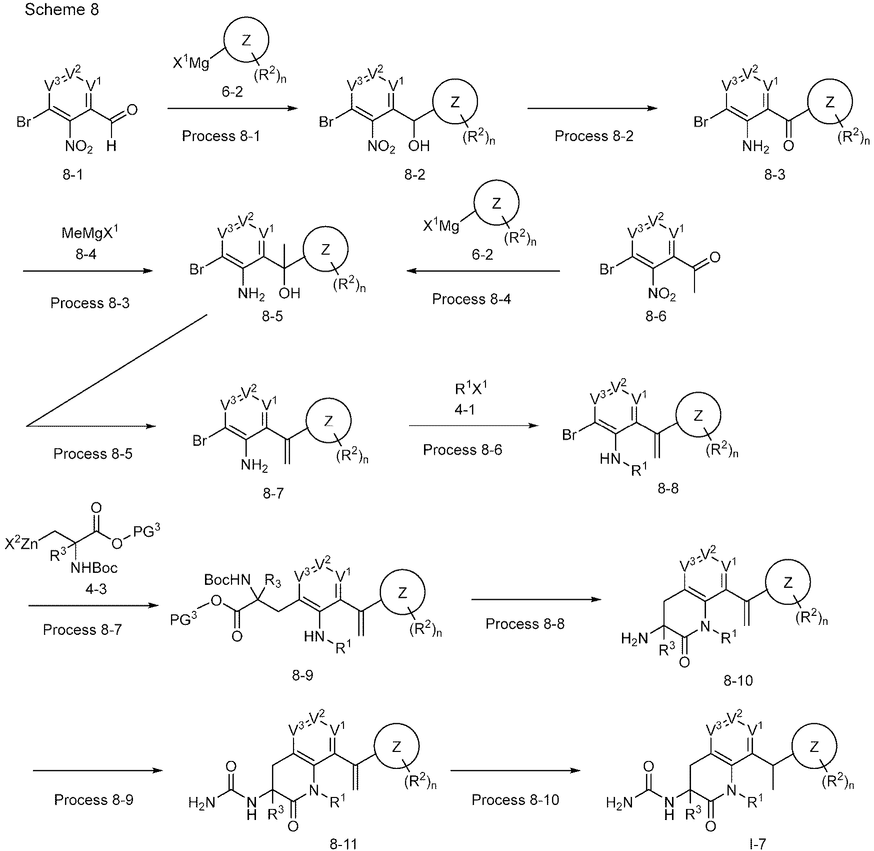

The symbols in the formulae have the same meanings as above.

### Process 8-1

Compound (8-2) can be produced by reacting Compound (8-1) with Grignard reagent (6-2).

### Process 8-2

Compound (8-3) can be produced by an oxidation reaction of Compound (8-2).

### Process 8-3

Compound (8-5) can be produced by reacting Compound (8-3) with Grignard reagent (8-4).

### Process 8-4

Compound (8-5) can be produced by reacting Compound (8-6) with Grignard reagent (6-2).

### Process 8-5

Compound (8-7) can be produced by reacting Compound (8-5) with triethylsilane in the presence of an acid.

### Process 8-6

Compound (8-8) can be produced by reacting Compound (8-7) with Compound (4-1) in the presence of a base.

### Process 8-7

Compound (8-9) can be produced by a Negishi cross coupling reaction of Compound (8-8) and Compound (4-3).

### Process 8-8

Compound (8-10) can be produced by reacting Compound (8-9) with an acid.

### Process 8-9

Compound (8-11) can be produced by reacting Compound (8-10) with trimethylsilyl isocyanate. As another method, Compound (8-11) can be produced by reacting Compound (8-10) with potassium cyanate or sodium cyanate in the presence of an acid.

### Process 8-10

Compound (I-7) can be produced by a catalytic reduction reaction of Compound (8-11).

The above schemes are examples of methods for producing the compounds represented by Formulae (I) to (V) or their intermediates during production. The schemes shown above can be variously modified into schemes that can be easily understood by those skilled in the art.

The compounds represented by Formulae (I) to (V) and their intermediates during production can be isolated and purified as necessary by isolation and purification methods well known to those skilled in the art such as solvent extraction, crystallization, recrystallization, chromatography, and preparative high-performance liquid chromatography.

The compound of the present invention can be used as a therapeutic agent for thyroid-related diseases because it has excellent TSHR antagonist activity. In the present invention, the thyroid-related diseases include, for example, hyperthyroidism, Graves' disease, thyroid eye disease and thyroid cancer. Preferably, the compound of the present invention can be used as a therapeutic agent for hyperthyroidism, Graves' disease, or thyroid eye disease (refer to Endocrinology, 2014, 155 (1), pp. 310-314). More preferably, the compound of the present invention can be used as a therapeutic agent for hyperthyroidism or Graves' disease.

In addition, in one embodiment, hyperthyroidism includes, for example, hyperthyroidism caused by any of Graves' disease, thyroiditis, Plummer's disease, toxic multinodular goiter, TSH-producing pituitary adenoma, hyperemesis gravidarum, ovarian goiter, gestational trophoblastic tumor, and germ cell tumor. Preferably, the compound of the present invention can be used as a therapeutic agent for hyperthyroidism caused by Graves' disease.

In addition, in one embodiment, the thyroid-related diseases are diseases and symptoms associated with abnormalities in thyroid hormone levels. The diseases and symptoms associated with abnormalities in thyroid hormone levels include, for example, diseases and symptoms caused by TRAb.

In the present invention, "treatment" includes the meaning of "prevention." For example, the treatment of hyperthyroidism, Graves' disease or thyroid eye disease includes the meaning of "prevention of relapse/recurrence" and "maintenance of remission." In addition, in one embodiment, the compound of the present invention can be used to prevent the development of thyroid eye disease in patients with Graves' disease.

In the present invention, "antagonist" refers to a drug that inhibits or blocks the function of a target protein, regardless of its binding site. For example, "antagonist" includes the meaning of "allosteric antagonist" and "negative allosteric modulator (NAM)."

The therapeutic effect of the compound of the present invention on thyroid-related diseases can be confirmed according to methods well known in the related art. Examples of methods for confirming the effect of hyperthyroidism or Graves' disease in model animals include methods described in Endocrinology 2007, 148(5), pp. 2335-2344 and methods similar thereto.

The pharmaceutical composition of the present invention may be used in various dosage forms depending on the usage. Examples of such dosage forms include powdered drugs, granules, fine granules, dry syrups, tablets, capsules, injections, liquid drugs, ointments, suppositories, patches, eye drops, and enema agents.

The pharmaceutical composition of the present invention includes a compound represented by Formulae (I) to (V) or a pharmacologically acceptable salt thereof as an active ingredient.

The pharmaceutical composition of the present invention is prepared using a compound represented by Formulae (I) to (V) or a pharmacologically acceptable salt thereof, and at least one pharmaceutical additive. These pharmaceutical compositions can also be prepared by being appropriately mixed, diluted or dissolved with appropriate pharmaceutical additives such as an excipient, a disintegrant, a binder, a lubricant, a diluent, a buffer, an isotonizing agent, a preservative, a wetting agent, an emulsifying agent, a dispersing agent, a stabilizer, and a dissolution aid according to a pharmaceutically known method depending on a dosage form.

When the pharmaceutical composition of the present invention is used for treatment, the dosage of the compound represented by Formulae (I) to (V) or a pharmacologically acceptable salt thereof is appropriately determined depending on the age, sex, weight, degree of disease and treatment of patients and the like. The daily dosage may be administered separately once, twice, three times or four times a day.

In the case of oral administration, the dosage for adults can be set, for example, in a range of 0.1 to 5,000 mg/day. In one embodiment, in the case of oral administration, the dosage can be set in a range of 1 to 1,500 mg/day, and preferably in a range of 1 to 500 mg/day.

In the case of parenteral administration, the dosage for adults can be set to, for example, 0.01 to 5,000 mg/day. In one embodiment, in the case of parenteral administration, the dosage can be set in a range of 0.1 to 1,500 mg/day and preferably in a range of 0.1 to 500 mg/day.

In one embodiment, the pharmaceutical composition of the present invention can also be used in combination with drugs other than TSHR antagonists. Examples of other drugs that can be used in combination in the treatment of thyroid-related diseases include antithyroid drugs (for example, thiamazole, propylthiouracil, etc.), inorganic iodine, lithium carbonate, and thyroid hormone formulations.

When the compound represented by Formulae (I) to (V) or a pharmacologically acceptable salt thereof are used in combination with other drugs, a formulation containing these active ingredients together or a formulation in which each of these active ingredients is separately formulated can be administered. When the components are formulated separately, the formulations can be administered separately or simultaneously. In addition, the dosage of the compound represented by Formulae (I) to (V) or a pharmacologically acceptable salt thereof can be appropriately reduced depending on the dosage of other drugs used in combination.

The compounds represented by Formulae (I) to (V) that are appropriately converted into prodrugs may be used. For example, the prodrugs of the compounds represented by Formulae (I) to (V) can be produced by introducing a prodrug-constituting group using a corresponding prodrug formation reagent such as a halide and performing purification. Examples of prodrug-constituting groups include groups described in "Development of Medicine" (Hirokawa Shoten, 1990), Vol. 7, pp. 163-198.

### [Examples]

Hereinafter, the present invention will be described in more detail with reference to reference examples, examples and test examples, but the present invention is not limited to the contents thereof.

The compounds described in the following examples, except for commercially available reagents, were named using ChemDraw Professional (PerkinElmer), MarvinSketch (ChemAxon) and the like.

### Reference Example A-1

### 2-bromo-6-phenoxyaniline

Potassium carbonate (10.1 g) was added to a mixture of 1-bromo-3-fluoro-2-nitrobenzene (4.01 g), phenol (1.89 g) and DMF (40 mL), and the mixture was stirred at 80°C for 6 hours. The reaction mixture was cooled to room temperature and water was then added. The mixture was extracted with ethyl acetate. The extract was washed with water, then dried with anhydrous sodium sulfate and concentrated under reduced pressure. The residue was purified by APS column chromatography (elution solvent: n-hexane/ethyl acetate=80/20 to 50/50) to obtain 1-bromo-2-nitro-3-phenoxybenzene (5.58 g).

Iron powder (3.05 g) was added to a mixture of the obtained Compound (5.58 g), ethanol (43 mL) and acetic acid (43 mL), and the mixture was stirred at 91°C for 11 hours. The reaction mixture was cooled to room temperature and then filtered through Celite, and the filtrate was concentrated under reduced pressure. The residue was purified by silica gel column chromatography (elution solvent: n-hexane/ethyl acetate=100/0 to 94/6) to obtain a title compound (4.68 g).

### Reference Example A-2

### 8-phenoxy-1,2,3,4-tetrahydroquinolin-2-one

A mixture of Reference Example A-1 (2.13 g), ethyl acrylate (2.42 g), palladium(II) acetate (0.181 g), tris(2-methylphenyl)phosphine (0.491 g), TEA (4.08 g) and MeCN (40 mL) was refluxed for 5 hours. The reaction mixture was cooled to room temperature and water was then added. The mixture was extracted with DCM, and the extract was then concentrated under reduced pressure. The residue was purified by silica gel column chromatography (elution solvent: n-hexane/ethyl acetate=100/0 to 70/30) to obtain ethyl (2E)-3-(2-amino-3-phenoxyphenyl) prop-2-enoate (2.08 g).

A mixture of the obtained Compound (2.08 g), 10% Pd/C (0.400 g) and methanol (30 mL) was stirred at room temperature under a hydrogen atmosphere for 12 hours. The reaction mixture was filtered through Celite, and the filtrate was concentrated under reduced pressure. The residue was suspended in water, and insoluble substances were collected by filtration. The obtained solid was dried under reduced pressure to obtain a title compound (1.53 g).

### Reference Example A-3

### 1-methyl-8-phenoxy-1,2,3,4-tetrahydroquinolin-2-one

Sodium hydride (about 60%) (0.022 g) was added to a mixture of Reference Example A-2 (0.100 g), methyl iodide (0.119 g) and THF (2 mL) under ice cooling, and the mixture was stirred at room temperature for 1 hour. Ice water was added to the reaction mixture under ice cooling. The mixture was extracted with ethyl acetate, and the extract was concentrated under reduced pressure. The residue was purified by silica gel column chromatography (elution solvent: n-hexane/ethyl acetate=74/26 to 57/43) to obtain a title compound (0.102 g).

### Reference Example A-4

### 2-bromo-6-(2-fluorophenoxy)aniline

Potassium carbonate (1.57 g) was added to a mixture of 1-bromo-3-fluoro-2-nitrobenzene (1.00 g), 2-fluorophenol (0.561 g) and DMF (10 mL), and the mixture was stirred at 100°C for 1 hour. The reaction mixture was cooled to room temperature, water was then added, and the mixture was stirred at room temperature for 15 minutes. Insoluble substances were collected by filtration, and the obtained solid was dissolved in ethyl acetate. The mixture was washed with water and a saturated saline solution, then dried with anhydrous magnesium sulfate, and concentrated under reduced pressure.

Iron powder (0.762 g) was added to a mixture of the residue, ethanol (12 mL) and acetic acid (12 mL), and the mixture was stirred at 90°C for 1 hour. The reaction mixture was cooled to room temperature and then filtered through Celite, and the filtrate was concentrated under reduced pressure. Ethyl acetate and a saturated sodium bicarbonate aqueous solution were added to the residue, and the mixture was filtered through Celite. The filtrate was extracted with ethyl acetate. The extract was washed with a saturated saline solution, dried with anhydrous magnesium sulfate, and then concentrated under reduced pressure to obtain a title compound (1.32 g).

### Reference Example A-5

### 8-(2-fluorophenoxy)-1-methyl-1,2,3,4-tetrahydroquinolin-2-one

A mixture of Reference Example A-4 (1.23 g), benzyl acrylate (1.41 g), palladium(II) acetate (0.098 g), tris(2-methylphenyl)phosphine (0.265 g), TEA (1.77 g) and MeCN (10 mL) was stirred at 135°C under microwave irradiation for 30 minutes. The reaction mixture was cooled to room temperature and then concentrated under reduced pressure. The residue was purified by silica gel column chromatography (elution solvent: n-hexane/ethyl acetate=90/10 to 40/60) to obtain benzyl 3-(2-amino-3-(2-fluorophenoxy)phenyl) prop-2-enoate (1.12 g).

A mixture of the obtained Compound (1.12 g), 10% Pd/C (0.100 g) and DMF (10 mL) was stirred at 60°C under a hydrogen atmosphere for 1 hour. The reaction mixture was stirred at 80°C under a hydrogen atmosphere for 1 hour. Ethanol (10 mL) was added to the reaction mixture, and the mixture was stirred at 70°C under a hydrogen atmosphere for 1 hour. The reaction mixture was cooled to room temperature and then filtered through Celite, and the filtrate was concentrated under reduced pressure. A mixture of the residue, 10% Pd/C (0.100 g) and acetic acid (10 mL) was stirred at 80°C under a hydrogen atmosphere for 2 hours. Concentrated sulfuric acid (0.020 mL) was added to the reaction mixture, and the mixture was stirred at 80°C under a hydrogen atmosphere for 2 hours. The reaction mixture was cooled to room temperature and then filtered through Celite, and the filtrate was concentrated under reduced pressure. Ethyl acetate and a saturated sodium bicarbonate aqueous solution were added to the residue. The mixture was extracted with ethyl acetate, and the extract was concentrated under reduced pressure. The residue was purified by silica gel column chromatography (elution solvent: n-hexane/ethyl acetate=67/33 to 37/63) to obtain 8-(2-fluorophenoxy)-1,2,3,4-tetrahydroquinolin-2-one (0.638 g).

Sodium hydride (about 60%) (0.119 g) and methyl iodide (0.704 g) were added to a mixture of the obtained Compound (0.638 g) and DMF (10 mL) at room temperature, and the mixture was stirred at room temperature for 30 minutes. Water and diethyl ether were added to the reaction mixture under ice cooling. The mixture was extracted with diethyl ether. The extract was washed with water and then concentrated under reduced pressure. The residue was purified by silica gel column chromatography (elution solvent: n-hexane/ethyl acetate=83/17 to 18/82) to obtain a title compound (0.672 g).

### Reference Example A-6

### 2-bromo-6-(3-fluorophenoxy)aniline

Potassium carbonate (0.471 g) was added to a mixture of 1-bromo-3-fluoro-2-nitrobenzene (0.500 g), 3-fluorophenol (0.255 g) and DMF (5 mL), and the mixture was stirred at 100°C for 1 hour. The reaction mixture was cooled to room temperature, and water and diethyl ether were then added. The mixture was extracted with diethyl ether. The extract was washed with water, then dried with anhydrous magnesium sulfate, and concentrated under reduced pressure.

Iron powder (0.381 g) was added to a mixture of the residue, ethanol (6 mL) and acetic acid (6 mL), and the mixture was stirred at 90°C for 1 hour. The reaction mixture was cooled to room temperature and then filtered through Celite, and the filtrate was concentrated under reduced pressure. Ethyl acetate and a saturated sodium bicarbonate aqueous solution were added to the residue, and the mixture was filtered through Celite. The filtrate was extracted with ethyl acetate. The extract was washed with a saturated saline solution, dried with anhydrous magnesium sulfate, and then concentrated under reduced pressure to obtain a title compound (0.681 g).

### Reference Example A-7

### 8-(3-fluorophenoxy)-1-methyl-1,2,3,4-tetrahydroquinolin-2-one

A mixture of Reference Example A-6 (0.641 g), benzyl acrylate (0.737 g), palladium(II) acetate (0.051 g), tris(2-methylphenyl)phosphine (0.138 g), TEA (0.920 g) and MeCN (10 mL) was stirred at 130°C under microwave irradiation for 1 hour. The reaction mixture was cooled to room temperature and then concentrated under reduced pressure. The residue was purified by silica gel column chromatography (elution solvent: n-hexane/ethyl acetate=90/10 to 59/41) to obtain benzyl 3-(2-amino-3-(3-fluorophenoxy)phenyl) prop-2-enoate (0.382 g).

A mixture of the obtained Compound (0.382 g), 10% Pd/C (0.040 g) and acetic acid (5 mL) was stirred at 80°C under a hydrogen atmosphere for 2 hours. The reaction mixture was cooled to room temperature and then filtered through Celite. A mixture of the filtrate and 10% Pd/C (0.040 g) was stirred at 80°C under a hydrogen atmosphere for 1 hour and then at 100°C for 3 hours. The reaction mixture was cooled to room temperature and then filtered through Celite, and the filtrate was concentrated under reduced pressure. Ethyl acetate and a saturated sodium bicarbonate aqueous solution were added to the residue. The mixture was extracted with ethyl acetate, and the extract was concentrated under reduced pressure. The residue was purified by silica gel column chromatography (elution solvent: n-hexane/ethyl acetate=67/33 to 24/76) to obtain 8-(3-fluorophenoxy)-1,2,3,4-tetrahydroquinolin-2-one (0.135 g).

Sodium hydride (about 60%) (0.025 g) was added to a mixture of the obtained Compound (0.135 g), methyl iodide (0.149 g) and DMF (2 mL) under ice cooling, and the mixture was stirred at room temperature for 30 minutes. Water and diethyl ether were added to the reaction mixture under ice cooling. The mixture was extracted with diethyl ether, and the extract was then washed with water and concentrated under reduced pressure. The residue was purified by silica gel column chromatography (elution solvent: n-hexane/ethyl acetate=90/10 to 31/69) to obtain a title compound (0.156 g).

### Reference Example A-8

Reference Example A-8 was synthesized in the same method as in Reference Example A-6 except that 3-(trifluoromethyl)phenol was used in place of 3-fluorophenol.

### Reference Example A-9

### 1-methyl-8-(3-(trifluoromethyl)phenoxy)-1,2,3,4-tetrahydroquinolin-2-one

A mixture of Reference Example A-8 (1.27 g), benzyl acrylate (1.24 g), palladium(II) acetate (0.086 g), tris(2-methylphenyl)phosphine (0.233 g), TEA (1.55 g) and MeCN (10 mL) was stirred at 130°C under microwave irradiation for 1 hour. The reaction mixture was cooled to room temperature and then concentrated under reduced pressure. The residue was purified by silica gel column chromatography (elution solvent: n-hexane/ethyl acetate=100/0 to 50/50) to obtain benzyl 3-(2-amino-3-(3-(trifluoromethyl)phenoxy)phenyl) prop-2-enoate (1.48 g).

A mixture of the obtained Compound (1.48 g), 10% Pd/C (0.382 g) and acetic acid (18 mL) was stirred at 80°C under a hydrogen atmosphere for 6 hours. The reaction mixture was cooled to room temperature and then filtered through Celite, and the filtrate was concentrated under reduced pressure. Ethyl acetate and a saturated sodium bicarbonate aqueous solution were added to the residue. The mixture was extracted with ethyl acetate. The extract was washed with water and then concentrated under reduced pressure. The residue was purified by silica gel column chromatography (elution solvent: n-hexane/ethyl acetate=60/40 to 33/67) to obtain 8-(3-(trifluoromethyl)phenoxy)-1,2,3,4-tetrahydroquinolin-2-one (0.661 g).

Sodium hydride (about 60%) (0.086 g) was added to a mixture of the obtained Compound (0.661 g), methyl iodide (0.305 g) and DMF (5 mL) under ice cooling, and the mixture was stirred at room temperature for 30 minutes. Water and diethyl ether were added to the reaction mixture under ice cooling. The mixture was extracted with diethyl ether, and the extract was then washed with water and concentrated under reduced pressure. The residue was purified by silica gel column chromatography (elution solvent: n-hexane/ethyl acetate=80/20 to 38/62) to obtain a title compound (0.607 g).

### Reference Example A-10

### 2-bromo-6-(3-chlorophenoxy)aniline

Potassium carbonate (0.937 g) was added to a mixture of 1-bromo-3-fluoro-2-nitrobenzene (0.746 g), 3-chlorophenol (0.436 g) and DMF (10 mL) and the mixture was stirred at 100°C for 1 hour. The reaction mixture was cooled to room temperature, and water and diethyl ether were then added. The mixture was extracted with diethyl ether, and the extract was washed with water. The extract was dried with anhydrous magnesium sulfate and then concentrated under reduced pressure.

Iron powder (0.947 g) was added to a mixture of the residue, ethanol (12 mL) and acetic acid (12 mL), and the mixture was stirred at 90°C for 1 hour. The reaction mixture was cooled to room temperature and then filtered through Celite, and the filtrate was concentrated under reduced pressure. A 2 mol/L sodium hydroxide aqueous solution was added to a mixture of the residue and ethyl acetate under ice cooling. The mixture was filtered through Celite, and the filtrate was extracted with ethyl acetate. The extract was washed with a saturated saline solution, dried with anhydrous magnesium sulfate, and then concentrated under reduced pressure to obtain a title compound (0.963 g).

### Reference Example A-11

### 8-(3-chlorophenoxy)-1-methyl-1,2,3,4-tetrahydroquinolin-2-one

A mixture of Reference Example A-10 (0.963 g), benzyl acrylate (1.05 g), palladium(II) acetate (0.072 g), tris(2-methylphenyl)phosphine (0.196 g), TEA (1.80 g) and MeCN (10 mL) was stirred at 130°C under microwave irradiation for 1 hour. The reaction mixture was cooled to room temperature and then concentrated under reduced pressure. The residue was purified by silica gel column chromatography (elution solvent: n-hexane/ethyl acetate=100/0 to 0/100) to obtain benzyl 3-(2-amino-3-(3-chlorophenoxy)phenyl) prop-2-enoate (0.955 g).

Cobalt(II) chloride pentahydrate (0.002 g), copper(II) sulfate (0.012 g) and sodium borohydride (0.105 g) were added to a mixture of the obtained Compound (0.955 g), methanol (5 mL) and THF (5 mL) at room temperature. The reaction mixture was stirred at room temperature for 10 minutes, a 2 mol/L sodium hydroxide aqueous solution (3.77 mL) was then added, and the mixture was stirred at 50°C for 30 minutes. The reaction mixture was cooled to room temperature and 2 mol/L hydrochloric acid (3.77 mL) was then added. The reaction mixture was concentrated under reduced pressure. Water was added to the residue, and the mixture was extracted with ethyl acetate. The extract was dried with anhydrous magnesium sulfate and then concentrated under reduced pressure.

Methyl iodide (0.693 g) and sodium hydride (about 60%) (0.107 g) were added to a mixture of the residue and DMF (10 mL) at room temperature, and the mixture was stirred at room temperature for 30 minutes. Water was added to the reaction mixture under ice cooling, and the mixture was extracted with diethyl ether. The extract was washed with water and then concentrated under reduced pressure. The residue was purified by silica gel column chromatography (elution solvent: n-hexane/ethyl acetate=90/10 to 0/100) to obtain a title compound (0.345 g).

### Reference Example A-12

Reference Example A-12 was synthesized in the same method as in Reference Example A-10 except that 1-bromo-3,4-difluoro-2-nitrobenzene was used in place of 1-bromo-3-fluoro-2-nitrobenzene and phenol was used in place of 3-chlorophenol.

### Reference Example A-13

### 7-fluoro-1-methyl-8-phenoxy-1,2,3,4-tetrahydroquinolin-2-one

A mixture of Reference Example A-12 (1.20 g), benzyl acrylate (1.38 g), palladium(II) acetate (0.095 g), tris(2-methylphenyl)phosphine (0.259 g), TEA (2.15 g) and MeCN (10 mL) was stirred at 130°C under microwave irradiation for 1 hour. The reaction mixture was cooled to room temperature and then concentrated under reduced pressure. The residue was purified by silica gel column chromatography (elution solvent: n-hexane/ethyl acetate=92/8 to 43/57) to obtain benzyl 3-(2-amino-4-fluoro-3-phenoxyphenyl) prop-2-enoate (1.03 g).

Cobalt(II) chloride pentahydrate (0.002 g), copper(II) sulfate (0.013 g) and sodium borohydride (0.229 g) were added to a mixture of the obtained Compound (0.998 g), methanol (7 mL) and THF (7 mL) at room temperature. The reaction mixture was stirred at room temperature for 10 minutes and 2 mol/L hydrochloric acid (0.5 mL) was then added. The reaction mixture was stirred at room temperature for 10 minutes. A 2 mol/L sodium hydroxide aqueous solution (0.5 mL) and DCM were added to the reaction mixture. The reaction mixture was stirred at room temperature for 10 minutes and at 50°C for 30 minutes. Concentrated hydrochloric acid (1 mL) was added to the reaction mixture at room temperature, and the mixture was stirred for 10 minutes. Ethyl acetate and water were added to the mixture. The mixture was extracted with ethyl acetate, and the extract was concentrated under reduced pressure. The residue was purified by silica gel column chromatography (elution solvent: n-hexane/ethyl acetate=65/35 to 26/74) to obtain 7-fluoro-8-phenoxy-1,2,3,4-tetrahydroquinolin-2-one (0.554 g).

Sodium hydride (about 60%) (0.103 g) was added to a mixture of the obtained Compound (0.554 g), methyl iodide (0.458 g) and DMF (6 mL), and the mixture was stirred at room temperature for 30 minutes. Water and diethyl ether were added to the reaction mixture under ice cooling. The mixture was extracted with diethyl ether, and the extract was washed with water. The extract was dried with anhydrous magnesium sulfate and then concentrated under reduced pressure. The residue was purified by silica gel column chromatography (elution solvent: n-hexane/ethyl acetate=70/30 to 35/65) to obtain a title compound (0.606 g).

### Reference Example A-14

Reference Example A-14 was synthesized in the same method as in Reference Example A-10 except that 1-chloro-2-fluoro-3-nitrobenzene was used in place of 1-bromo-3-fluoro-2-nitrobenzene, and phenol was used in place of 3-chlorophenol.

### Reference Example A-15

### 7-chloro-1-methyl-8-phenoxy-1,2,3,4-tetrahydroquinolin-2-one

Pyridine (0.630 g) and 9-fluorenylmethyl chloroformate (1.65 g) were added to a mixture of Reference Example A-14 (1.17 g) and DCM (20 mL) under ice cooling, and the mixture was stirred at room temperature for 30 minutes. 1 mol/L hydrochloric acid was added to the reaction mixture under ice cooling. The mixture was extracted with DCM, and the extract was concentrated under reduced pressure. The residue was purified by silica gel column chromatography (elution solvent: n-hexane/ethyl acetate=100/0 to 44/56) to obtain N-(3-chloro-2-phenoxyphenyl) carbamate (9H-fluoren-9-yl)methyl (1.01 g).

A mixture of the obtained Compound (1.01 g), n-butyl acrylate (0.589 g), palladium(II) acetate (0.052 g), potassium peroxodisulfate (0.683 g), TFA (16 mL) and DCM (4 mL) was stirred at room temperature for 14 hours. The reaction mixture was filtered through Celite, and the filtrate was concentrated under reduced pressure. A saturated sodium bicarbonate aqueous solution was added to a mixture of the residue and ethyl acetate under ice cooling. The mixture was extracted with ethyl acetate, and the extract was then washed with water, and concentrated under reduced pressure. The residue was purified by silica gel column chromatography (elution solvent: n-hexane/ethyl acetate=100/0 to 43/57) to obtain butyl 3-(4-chloro-2-((((9H-fluoren-9-yl)methoxy)carbonyl)amino)-3-phenoxyphenyl) prop-2-enoate (0.271 g).

Piperidine (0.203 g) was added to a mixture of the obtained Compound (0.271 g) and DCM (2 mL), and the mixture was stirred at room temperature for 30 minutes. The reaction mixture was concentrated under reduced pressure, and the residue was purified by silica gel column chromatography (elution solvent: n-hexane/ethyl acetate=100/0 to 68/32) to obtain butyl 3-(2-amino-4-chloro-3-phenoxyphenyl) prop-2-enoate (0.196 g).

Cobalt(II) chloride pentahydrate (0.374 mg), copper(II) sulfate (2.71 mg) and sodium borohydride (0.024 g) were added to a mixture of the obtained Compound (0.196 g), methanol (5 mL) and THF (5 mL), and the mixture was stirred at room temperature for 10 minutes. A 2 mol/L sodium hydroxide aqueous solution (0.850 mL) was added to the reaction mixture, and the mixture was stirred at 50°C for 30 minutes. The mixture was cooled to room temperature, acetic acid (0.170 g) was then added, and the mixture was concentrated under reduced pressure. Acetic acid (2 mL) was added to the residue, the mixture was stirred at 50°C for 30 minutes, and the mixture was then concentrated under reduced pressure. A saturated sodium bicarbonate aqueous solution was added to a mixture of the residue and DCM. The mixture was extracted with DCM, and the extract was concentrated under reduced pressure. The residue was purified by silica gel column chromatography (elution solvent: n-hexane/ethyl acetate=70/30 to 37/63) to obtain 7-chloro-8-phenoxy-1,2,3,4-tetrahydroquinolin-2-one (0.060 g).

Methyl iodide (0.047 g) and sodium hydride (about 60%) (0.011 g) were added to a mixture of the obtained Compound (0.060 g) and DMF (1 mL) under ice cooling, and the mixture was stirred at room temperature for 30 minutes. Water and diethyl ether were added to the reaction mixture under ice cooling. The mixture was extracted with diethyl ether. The extract was washed with water and then concentrated under reduced pressure. The residue was purified by silica gel column chromatography (elution solvent: n-hexane/ethyl acetate=95/5 to 32/68) to obtain a title compound (0.061 g).

### Reference Example A-16

### 1-methyl-8-((3-(trifluoromethyl)pyridin-2-yl)oxy)-1,2,3,4-tetrahydroquinolin-2-one

Potassium carbonate (0.508 g) was added to a mixture of 8-hydroxy-1,2,3,4-tetrahydroquinolin-2-one (0.200 g), NMP (5 mL) and 2-chloro-3-(trifluoromethyl)pyridine (0.289 g), and the mixture was stirred at 110°C for 8 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure. The residue was purified by Method A (elution solvent: n-hexane/ethyl acetate=60/40 to 0/100) to obtain 8-((3-(trifluoromethyl)pyridin-2-yl)oxy)-1,2,3,4-tetrahydroquinolin-2-one (0.240 g).

Sodium hydride (about 60%) (0.047 g) was added to a mixture of the obtained Compound (0.240 g), DMF (2 mL) and methyl iodide (0.166 g) under ice cooling, and the mixture was stirred at room temperature for 1 hour. A saturated ammonium chloride aqueous solution, ethyl acetate and n-hexane were added to the reaction mixture under ice cooling. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=90/10 to 60/40) to obtain a title compound (0.240 g).

### Reference Example B-1

### 2-bromo-N-methyl-6-(2-(trifluoromethyl)phenoxy)aniline

Potassium carbonate (1.26 g) was added to a mixture of 1-bromo-3-fluoro-2-nitrobenzene (1.00 g), 2-(trifluoromethyl)phenol (0.737 g) and NMP (10 mL), and the mixture was stirred at 110°C for 3 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure.

Ammonium chloride (6.08 g) and iron powder (1.27 g) were added to a mixture of the residue, ethanol (15 mL) and water (5 mL), and the mixture was stirred at 80°C for 1 hour. The reaction mixture was cooled to room temperature and then filtered through Celite. Water was added to the filtrate, and the mixture was extracted with ethyl acetate. The extract was washed with a saturated saline solution, then dried with anhydrous sodium sulfate, and concentrated under reduced pressure. The residue was purified by silica gel column chromatography (elution solvent: n-hexane/ethyl acetate=98/2 to 80/20) to obtain 2-bromo-6-(2-(trifluoromethyl)phenoxy)aniline (1.50 g).

Methyllithium (1.12 mol/L in diethyl ether) (0.887 mL) was added to a mixture of the obtained Compound (0.300 g) and THF (3 mL) at -78°C, and the mixture was stirred at the same temperature for 1 hour. A mixture of methyl iodide (0.154 g) and THF (2 mL) was added to the reaction mixture at -78°C and the mixture was then stirred at the same temperature for 10 minutes and for 2 hours under ice cooling. A saturated ammonium chloride aqueous solution was added to the reaction mixture under ice cooling. The mixture was extracted with ethyl acetate, and the extract was washed with a saturated saline solution. The extract was dried with anhydrous sodium sulfate and then concentrated under reduced pressure. The residue was purified by Method A (elution solvent: n-hexane/ethyl acetate=98/2 to 80/20) to obtain a title compound (0.250 g).

### Reference Example B-2

### (3R)-3-amino-1-methyl-8-(2-(trifluoromethyl)phenoxy)-1,2,3,4-tetrahydroquinolin-2-one

Zinc (0.104 g) was added to a mixture of methyl (S)-2-((tert-butoxycarbonyl)amino)-3-iodopropanate (0.285 g) and DMF (4 mL), and the mixture was stirred at room temperature for 2 hours. Reference Example B-1 (0.250 g), palladium(II) acetate (0.008 g) and Xphos (0.034 g) were added to the reaction mixture, and the mixture was stirred at room temperature for 13 hours. A saturated ammonium chloride aqueous solution and ethyl acetate were added to the reaction mixture. The mixture was filtered through Celite, and the filtrate was extracted with ethyl acetate. The extract was washed with water and a saturated saline solution, dried with anhydrous sodium sulfate, and then concentrated under reduced pressure.

TFA (1.65 g) was added to a mixture of the residue and DCM (5 mL) under ice cooling, and the mixture was stirred at room temperature for 3 hours. A 5 mol/L sodium hydroxide aqueous solution (3 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=49/49/2 to 0/98/2 to 0/86/14) to obtain a title compound (0.084 g).

### Reference Example B-3

### 2-bromo-6-(2-methoxyphenoxy)-N-methylaniline

Potassium carbonate (0.769 g) was added to a mixture of 1-bromo-3-fluoro-2-nitrobenzene (0.612 g), 2-methoxyphenol (0.345 g) and DMF (10 mL), and the mixture was stirred at 100°C for 1 hour. The reaction mixture was cooled to room temperature, and water and diethyl ether were then added. The mixture was extracted with diethyl ether, and the extract was washed with water. The extract was dried with anhydrous magnesium sulfate and then concentrated under reduced pressure.

Iron powder (0.777 g) was added to a mixture of the residue, ethanol (6 mL) and acetic acid (3 mL), and the mixture was stirred at 90°C for 1 hour. The reaction mixture was cooled to room temperature and then filtered through Celite, and the filtrate was concentrated under reduced pressure. A 2 mol/L sodium hydroxide aqueous solution was added to a mixture of the residue and ethyl acetate under ice cooling. The mixture was filtered through Celite, and the filtrate was extracted with ethyl acetate. The extract was washed with a saturated saline solution, dried with anhydrous magnesium sulfate and then concentrated under reduced pressure.

Methyllithium (1.06 mol/L in diethyl ether) (2.72 mL) was added to a mixture of the residue and THF (10 mL) at -78°C, and the mixture was stirred at the same temperature for 30 minutes. A mixture of methyl iodide (0.375 g) and THF (1 mL) was added to the reaction mixture at -78°C, and the mixture was then stirred at the same temperature for 10 minutes and under ice cooling for 30 minutes. A saturated ammonium chloride aqueous solution was added to the reaction mixture under ice cooling, and the mixture was extracted with ethyl acetate. The extract was washed with water and then concentrated under reduced pressure. The residue was purified by silica gel column chromatography (elution solvent: n-hexane/ethyl acetate=100/0 to 55/45) to obtain a title compound (0.544 g).

### Reference Example B-4

### (3R)-3-amino-8-(2-methoxyphenoxy)-1-methyl-1,2,3,4-tetrahydroquinolin-2-one

Under an argon atmosphere, zinc (0.140 g) was added to a mixture of methyl (S)-2-((tert-butoxycarbonyl)amino)-3-iodopropanate (0.384 g) and DMF (5 mL), and the mixture was stirred at room temperature for 2 hours. Reference Example B-3 (0.300 g), palladium(II) acetate (0.011 g) and Xphos (0.046 g) were added to the reaction mixture, and the mixture was stirred at room temperature for 14 hours. A saturated ammonium chloride aqueous solution and ethyl acetate were added to the reaction mixture. The mixture was filtered through Celite, and the filtrate was extracted with ethyl acetate. The extract was washed with water and dried with anhydrous magnesium sulfate and then concentrated under reduced pressure.

A mixture of the residue and hydrogen chloride (4 mol/L in 1,4-dioxane) (4 mL) was stirred at room temperature for 30 minutes. The reaction mixture was concentrated under reduced pressure. A saturated sodium bicarbonate aqueous solution was added to a mixture of the residue and ethyl acetate. The mixture was extracted with ethyl acetate, and the extract was concentrated under reduced pressure. The residue was purified by APS column chromatography (elution solvent: n-hexane/ethyl acetate/methanol=50/50/0 to 0/100/0 to 0/50/50) to obtain a title compound (0.173 g).

### Reference Example B-5

### 3-(3-bromo-2-(methylamino)phenoxy)benzonitrile

Potassium carbonate (1.27 g) was added to a mixture of 1-bromo-3-fluoro-2-nitrobenzene (1.01 g), 3-hydroxybenzonitrile (0.548 g) and DMF (10 mL) and the mixture was stirred at 100°C for 1 hour. The reaction mixture was cooled to room temperature and water was then added. Insoluble substances were collected by filtration, and the obtained solid was washed with DMF/water (1/2) and water and then dried under reduced pressure to obtain 3-(3-bromo-2-nitrophenoxy)benzonitrile (1.33 g).

Ammonium chloride (5.57 g) and iron powder (1.16 g) were added to a mixture of the obtained Compound (1.33 g), ethanol (15 mL) and water (5 mL), and the mixture was stirred at 80°C for 1 hour. The reaction mixture was cooled to room temperature and then filtered through Celite. Water was added to the filtrate, the mixture was then extracted with ethyl acetate, and the extract was washed with a saturated saline solution. The extract was dried with anhydrous magnesium sulfate and then concentrated under reduced pressure. Toluene was added to the residue and then concentrated under reduced pressure to obtain 3-(2-amino-3-bromophenoxy)benzonitrile (1.22 g).

Under an argon atmosphere, methyllithium (1.06 mol/L in diethyl ether) (4.2 mL) was added to a mixture of the obtained Compound (1.17 g) and THF (12 mL) at -78°C and the mixture was stirred at the same temperature for 1 hour. A mixture of methyl iodide (0.687 g) and THF (7 mL) was added to the reaction mixture at -78°C and the mixture was then stirred at the same temperature for 10 minutes and for 2 hours under ice cooling. A saturated ammonium chloride aqueous solution was added to the reaction mixture under ice cooling. The mixture was extracted with DCM, and the extract was concentrated under reduced pressure. The residue was purified by silica gel column chromatography (elution solvent: n-hexane/ethyl acetate=100/0 to 85/15) to obtain a title compound (1.24 g).

### Reference Example B-6

### 3-(((3R)-3-amino-1-methyl-2-oxo-1,2,3,4-tetrahydroquinolin-8-yl)oxy)benzonitrile

Under an argon atmosphere, zinc (0.527 g) was added to a mixture of methyl (S)-2-((tert-butoxycarbonyl)amino)-3-iodopropanate (1.44 g) and DMF (8 mL), and the mixture was stirred at room temperature for 2 hours. A mixture of Reference Example B-5 (1.11 g) and DMF (3 mL), palladium(II) acetate (0.042 g) and Xphos (0.174 g) were added to the reaction mixture, and the mixture was stirred at room temperature for 13 hours. A saturated ammonium chloride aqueous solution and ethyl acetate were added to the reaction mixture. The mixture was filtered through Celite, and the filtrate was extracted with ethyl acetate. The extract was washed with water and dried with anhydrous magnesium sulfate and then concentrated under reduced pressure.

TFA (8.33 g) was added to a mixture of the residue and DCM (11 mL) under ice cooling, and the mixture was stirred at room temperature for 2 hours. A 5 mol/L sodium hydroxide aqueous solution (13.2 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=50/50/0 to 0/100/0 to 0/85/15) to obtain a title compound (0.570 g).

### Reference Example B-7

Reference Example B-7 was synthesized in the same method as in Reference Example B-3 except that 3-methoxyphenol was used in place of 2-methoxyphenol.

### Reference Example B-8

### (3R)-3-amino-8-(3-methoxyphenoxy)-1-methyl-1,2,3,4-tetrahydroquinolin-2-one

Under an argon atmosphere, zinc (0.143 g) was added to a mixture of, methyl (S)-2-((tert-butoxycarbonyl)amino)-3-iodopropanate (0.432 g) and DMF (5 mL), and the mixture was stirred at room temperature for 1 hour. A mixture of Reference Example B-7 (0.270 g) and DMF (0.5 mL), palladium(II) acetate (0.010 g) and Xphos (0.042 g) were added to the reaction mixture, and the mixture was stirred at room temperature for 14 hours. A saturated ammonium chloride aqueous solution and diethyl ether were added to the reaction mixture. The mixture was filtered through Celite, and the filtrate was extracted with diethyl ether. The extract was washed with water and dried with anhydrous magnesium sulfate and then concentrated under reduced pressure.

A mixture of the residue and hydrogen chloride (4 mol/L in 1,4-dioxane) (3 mL) was stirred under ice cooling for 30 minutes. The reaction mixture was concentrated under reduced pressure. A saturated sodium bicarbonate aqueous solution was added to a mixture of the residue and ethyl acetate. The mixture was extracted with ethyl acetate, and the extract was concentrated under reduced pressure. The residue was purified by APS column chromatography (elution solvent: n-hexane/ethyl acetate/methanol=30/70/0 to 0/100/0 to 0/70/30) to obtain a title compound (0.167 g).

### Reference Example B-9

Reference Example B-9 was synthesized in the same method as in Reference Example B-3 except that 2-ethylphenol was used in place of 2-methoxyphenol.

### Reference Example B-10

Reference Example B-10 was synthesized in the same method as in Reference Example B-8 except that Reference Example B-9 was used in place of Reference Example B-7.

### Reference Example B-11

Reference Example B-11 was synthesized in the same method as in Reference Example B-3 except that 3-ethylphenol was used in place of 2-methoxyphenol.

### Reference Example B-12

### (3R)-3-amino-8-(3-ethylphenoxy)-1-methyl-1,2,3,4-tetrahydroquinolin-2-one

Under an argon atmosphere, zinc (0.143 g) was added to a mixture of methyl (S)-2-((tert-butoxycarbonyl)amino)-3-iodopropanate (0.432 g) and DMF (5 mL), and the mixture was stirred at room temperature for 2 hours. A mixture of Reference Example B-11 (0.268 g) and DMF (1 mL), palladium(II) acetate (0.010 g) and Xphos (0.042 g) were added to the reaction mixture, and the mixture was stirred at room temperature for 16 hours. A saturated ammonium chloride aqueous solution and ethyl acetate were added to the reaction mixture. The mixture was extracted with ethyl acetate, and the extract was washed with water. The extract was dried with anhydrous magnesium sulfate and concentrated under reduced pressure.

A mixture of the residue and hydrogen chloride (4 mol/L in 1,4-dioxane) (4 mL) was stirred at room temperature for 30 minutes. The reaction mixture was concentrated under reduced pressure. A saturated sodium bicarbonate aqueous solution was added to a mixture of the residue and ethyl acetate. The mixture was extracted with ethyl acetate, and the extract was washed with water and then concentrated under reduced pressure. The residue was purified by APS column chromatography (elution solvent: n-hexane/ethyl acetate/methanol=30/70/0 to 0/100/0 to 0/70/30) to obtain a title compound (0.110 g).

### Reference Example B-13

### 2-bromo-6-(2-chloro-5-fluorophenoxy)-N-methylaniline

Potassium carbonate (0.377 g) was added to a mixture of 1-bromo-3-fluoro-2-nitrobenzene (0.300 g), 2-chloro-5-fluorophenol (0.220 g) and DMF (10 mL), and the mixture was stirred at 100°C for 1 hour. The reaction mixture was cooled to room temperature, and water and diethyl ether were then added. The mixture was extracted with diethyl ether, and the extract was washed with water. The extract was dried with anhydrous magnesium sulfate and then concentrated under reduced pressure.

Ammonium chloride (1.46 g) and iron powder (0.777 g) were added to a mixture of the residue, ethanol (9 mL) and water (3 mL), and the mixture was stirred at 90°C for 1 hour. The reaction mixture was cooled to room temperature and then filtered through Celite. The filtrate was extracted with ethyl acetate, and the extract was then concentrated under reduced pressure. The residue was purified by silica gel column chromatography (elution solvent: n-hexane/ethyl acetate=100/0 to 75/25) to obtain 2-bromo-6-(2-chloro-5-fluorophenoxy)aniline (0.417 g).

Methyllithium (1.06 mol/L in diethyl ether) (1.37 mL) was added to a mixture of the obtained Compound (0.417 g) and THF (7 mL) at -78°C, and the mixture was stirred at the same temperature for 30 minutes. Methyl iodide (0.224 g) was added to the reaction mixture at -78°C, and the mixture was then stirred at the same temperature for 10 minutes and under ice cooling for 30 minutes. A saturated ammonium chloride aqueous solution was added to the reaction mixture under ice cooling. The mixture was extracted with ethyl acetate, and the extract was then washed with water, and concentrated under reduced pressure. The residue was purified by silica gel column chromatography (elution solvent: n-hexane/ethyl acetate=100/0 to 77/23) to obtain a title compound (0.436 g).

### Reference Example B-14

### (3R)-3-amino-8-(2-chloro-5-fluorophenoxy)-1-methyl-1,2,3,4-tetrahydroquinolin-2-one

Under an argon atmosphere, zinc (0.215 g) was added to a mixture of methyl (S)-2-((tert-butoxycarbonyl)amino)-3-iodopropanate (0.650 g) and DMF (7 mL), and the mixture was stirred at room temperature for 2 hours. A mixture of Reference Example B-13 (0.435 g) and DMF (1 mL), palladium(II) acetate (0.015 g) and Xphos (0.063 g) were added to the reaction mixture, and the mixture was stirred at room temperature for 16 hours. A saturated ammonium chloride aqueous solution and ethyl acetate were added to the reaction mixture. The mixture was extracted with ethyl acetate, and the extract was washed with water. The extract was dried with anhydrous magnesium sulfate and concentrated under reduced pressure.

A mixture of the residue and hydrogen chloride (4 mol/L in 1,4-dioxane) (4 mL) was stirred at room temperature for 30 minutes. The reaction mixture was concentrated under reduced pressure. A saturated sodium bicarbonate aqueous solution was added to a mixture of the residue and ethyl acetate. The mixture was extracted with ethyl acetate, and the extract was then washed with water, and concentrated under reduced pressure. The residue was purified by APS column chromatography (elution solvent: n-hexane/ethyl acetate/methanol=30/70/0 to 0/100/0 to 0/71/29) to obtain a title compound (0.198 g).

### Reference Example B-15

### 2-bromo-N-methyl-6-(2-isopropylphenoxy)aniline

Potassium carbonate (0.648 g) was added to a mixture of 1-bromo-3-fluoro-2-nitrobenzene (0.516 g), 2-isopropylphenol (0.351 g) and DMF (5 mL), and the mixture was stirred at 100°C for 1 hour. Water and toluene were added to the reaction mixture under water cooling. The mixture was extracted with toluene, and the extract was washed with water. The extract was concentrated under reduced pressure to obtain 1-bromo-3-(2-isopropylphenoxy)-2-nitrobenzene (1.01 g).

Ammonium chloride (3.00 g) and iron powder (0.638 g) were added to a mixture of the obtained Compound (0.774 g), ethanol (8 mL) and water (2.7 mL), and the mixture was stirred at 80°C for 2 hours. The reaction mixture was cooled with water and then filtered through Celite. Water was added to the filtrate and the mixture was then extracted with ethyl acetate. The extract was concentrated under reduced pressure, the residue was purified by silica gel column chromatography (elution solvent: n-hexane/ethyl acetate=100/0 to 70/30) to obtain 2-bromo-6-(2-isopropylphenoxy)aniline (0.746 g).

Methyllithium (1.04 mol/L in diethyl ether) (2.5 mL) was added to a mixture of the obtained Compound (0.672 g) and THF (7 mL) at -78°C, and the mixture was stirred at the same temperature for 1 hour. A mixture of methyl iodide (0.374 g) and THF (4 mL) was added to the reaction mixture at -78°C, and the mixture was then stirred under water cooling for 1 hour. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.787 g).

### Reference Example B-16

### (3R)-3-amino-1-methyl-8-(2-isopropylphenoxy)-1,2,3,4-tetrahydroquinolin-2-one

Under an argon atmosphere, zinc (0.312 g) was added to a mixture of Reference Example B-15 (0.685 g) and DMF (6 mL), and the mixture was stirred at room temperature for 2 hours. methyl (S)-2-((tert-butoxycarbonyl)amino)-3-iodopropanate (0.845 g), palladium(II) acetate (0.026 g) and Xphos (0.104 g) were added to the reaction mixture, and the mixture was stirred at room temperature for 14 hours. A saturated ammonium chloride aqueous solution, water and ethyl acetate were added to the reaction mixture. The mixture was filtered through Celite, and the filtrate was extracted with ethyl acetate. The extract was concentrated under reduced pressure. Ethyl acetate, water and toluene were added to the residue. The mixture was extracted with ethyl acetate, and the extract was dried with anhydrous magnesium sulfate and then concentrated under reduced pressure.

TFA (4.88 g) was added to a mixture of the residue and DCM (7 mL) under ice cooling, and the mixture was stirred at room temperature for 1.5 hours. A 5 mol/L sodium hydroxide aqueous solution (8.5 mL) was added to the reaction mixture under ice cooling. The mixture was extracted with DCM, and the organic layer was concentrated under reduced pressure. The residue was purified by APS column chromatography (elution solvent: n-hexane/ethyl acetate/methanol=50/50/0 to 0/100/0 to 0/80/20) to obtain a title compound (0.357 g).

### Reference Example B-17

### 2-bromo-6-(2-(difluoromethyl)phenoxy)-N-methylaniline

Potassium carbonate (0.660 g) was added to a mixture of 1-bromo-3-fluoro-2-nitrobenzene (0.522 g), 2-hydroxybenzaldehyde (0.290 g) and DMF (5 mL), and the mixture was stirred at 100°C for 1 hour. The reaction mixture was cooled to room temperature, and water, ethyl acetate and toluene were then added. A saturated ammonium chloride aqueous solution and 2 mol/L hydrochloric acid were added to the mixture. The mixture was extracted with ethyl acetate and the extract was washed with water. The extract was concentrated under reduced pressure, and the residue was purified by silica gel column chromatography (elution solvent: n-hexane/ethyl acetate=100/0 to 55/45) to obtain 2-(3-bromo-2-nitrophenoxy)benzaldehyde (0.686 g).

Bis(2-methoxyethyl)aminosulfur trifluoride (0.937 g) was added to a mixture of the obtained Compound (0.682 g), DCM (3.4 mL) and ethanol (0.020 g), and the mixture was stirred at 50°C for 2 hours. The reaction mixture was added to a saturated sodium bicarbonate aqueous solution cooled with ice. The mixture was extracted with DCM, and the extract was concentrated under reduced pressure. The residue was purified by silica gel column chromatography (elution solvent: n-hexane/ethyl acetate=100/0 to 50/50) to obtain 1-bromo-3-(2-(difluoromethyl)phenoxy)-2-nitrobenzene (0.674 g).

Ammonium chloride (2.30 g) and iron powder (0.480 g) were added to a mixture of the obtained Compound (0.588 g), ethanol (6 mL) and water (2 mL) and the mixture was stirred at 80°C for 1 hour. The reaction mixture was cooled to room temperature and then filtered through Celite. Water was added to the filtrate, and the mixture was then extracted with ethyl acetate. The extract was concentrated under reduced pressure, and the residue was purified by silica gel column chromatography (elution solvent: n-hexane/ethyl acetate=100/0 to 70/30) to obtain 2-bromo-6-(2-(difluoromethyl)phenoxy)aniline (0.488 g).

Methyllithium (1.04 mol/L in diethyl ether) (1.6 mL) was added to a mixture of the obtained Compound (0.482 g) and THF (5 mL) at -78°C and the mixture was stirred at the same temperature for 1 hour. A mixture of methyl iodide (0.261 g) and THF (3 mL) was added to the reaction mixture at -78°C and then stirred under ice cooling for 1 hour. A saturated ammonium chloride aqueous solution was added to the reaction mixture under ice cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.555 g).

### Reference Example B-18

Reference Example B-18 was synthesized in the same method as in Reference Example B-16 except that Reference Example B-17 was used in place of Reference Example B-15.

### Reference Example B-19

Reference Example B-19 was synthesized in the same method as in Reference Example B-13 except that 1-bromo-3,4-difluoro-2-nitrobenzene was used in place of 1-bromo-3-fluoro-2-nitrobenzene, and 2-chlorophenol was used in place of 2-chloro-5-fluorophenol.

### Reference Example B-20

Reference Example B-20 was synthesized in the same method as in Reference Example B-12 except that Reference Example B-19 was used in place of Reference Example B-11.

### Reference Example B-21

Reference Example B-21 was synthesized in the same method as in Reference Example B-13 except that 2-chloro-4-fluorophenol was used in place of 2-chloro-5-fluorophenol.

### Reference Example B-22

### (3R)-3-amino-8-(2-chloro-4-fluorophenoxy)-1-methyl-1,2,3,4-tetrahydroquinolin-2-one

Under an argon atmosphere, zinc (0.218 g) was added to a mixture of methyl (S)-2-((tert-butoxycarbonyl)amino)-3-iodopropanate (0.659 g) and DMF (7 mL), and the mixture was stirred at room temperature for 1 hour. A mixture of Reference Example B-21 (0.441 g) and DMF (1 mL), palladium(II) acetate (0.015 g) and Xphos (0.064 g) were added to the reaction mixture, and the mixture was stirred at room temperature for 16 hours. A saturated ammonium chloride aqueous solution and ethyl acetate were added to the reaction mixture. The mixture was extracted with ethyl acetate and the extract was washed with water. The extract was dried with anhydrous magnesium sulfate and then concentrated under reduced pressure.

TFA (3.04 g) was added to a mixture of the residue and DCM (7 mL) under ice cooling, and the mixture was stirred at room temperature for 2 hours. A 5 mol/L sodium hydroxide aqueous solution (1.8 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=40/60/0 to 0/100/0 to 0/35/65) to obtain a title compound (0.295 g).

### Reference Example B-23

### 2-bromo-6-(5-fluoro-2-(trifluoromethyl)phenoxy)-N-methylaniline

Potassium carbonate (0.188 g) was added to a mixture of 1-bromo-3-fluoro-2-nitrobenzene (0.150 g), 5-fluoro-2-(trifluoromethyl)phenol (0.135 g) and NMP (2 mL) and the mixture was stirred at 110°C for 6 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure.

Ammonium chloride (0.912 g) and iron powder (0.190 g) were added to a mixture of the residue, ethanol (3 mL) and water (1 mL) and the mixture was stirred at 80°C for 2 hours. The reaction mixture was cooled to room temperature and water was then added. The mixture was extracted with DCM, and the extract was concentrated under reduced pressure. The residue was purified by Method A (elution solvent: n-hexane/ethyl acetate=98/2 to 85/15) to obtain 2-bromo-6-(5-fluoro-2-(trifluoromethyl)phenoxy)aniline (0.120 g).

Methyllithium (1.06 mol/L in diethyl ether) (0.356 mL) was added to a mixture of the obtained Compound (0.120 g) and THF (1 mL) at -78°C, and the mixture was stirred at the same temperature for 1 hour. A mixture of methyl iodide (0.058 g) and THF (1 mL) was added to the reaction mixture at -78°C, and the mixture was then stirred at the same temperature for 10 minutes and under ice cooling for 1 hour. A saturated ammonium chloride aqueous solution was added to the reaction mixture under ice cooling. The mixture was extracted with DCM, and the extract was concentrated under reduced pressure. The residue was purified by Method A (elution solvent: n-hexane/ethyl acetate=98/2 to 85/15) to obtain a title compound (0.094 g).

### Reference Example B-24

Reference Example B-24 was synthesized in the same method as in Reference Example B-2 except that Reference Example B-23 was used in place of Reference Example B-1.

### Reference Example B-25

### 6-bromo-3-chloro-2-(2-chlorophenoxy)-N-methylaniline

Potassium carbonate (0.188 g) was added to a mixture of 1-bromo-4-chloro-3-fluoro-2-nitrobenzene (0.150 g), 2-chlorophenol (0.076 g) and NMP (2 mL), and the mixture was stirred at 110°C for 1.5 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure.

Ammonium chloride (0.788 g) and iron powder (0.165 g) were added to a mixture of the residue, ethanol (3 mL) and water (1 mL), and the mixture was stirred at 80°C for 1.5 hours. The reaction mixture was cooled to room temperature and water was then added. The mixture was extracted with DCM, and the extract was concentrated under reduced pressure. The residue was purified by Method A (elution solvent: n-hexane/ethyl acetate=98/2 to 85/15) to obtain 6-bromo-3-chloro-2-(2-chlorophenoxy)aniline (0.110 g).

Methyllithium (1.06 mol/L in diethyl ether) (0.343 mL) was added to a mixture of the obtained Compound (0.110 g) and THF (1 mL) at -78°C, and the mixture was stirred at the same temperature for 1 hour. A mixture of methyl iodide (0.056 g) and THF (1 mL) was added to the reaction mixture at -78°C, and the mixture was then stirred at the same temperature for 10 minutes and at room temperature for 1 hour. A saturated ammonium chloride aqueous solution was added to the reaction mixture under ice cooling. The mixture was extracted with DCM, and the extract was then concentrated under reduced pressure. The residue was purified by Method A (elution solvent: n-hexane/ethyl acetate=98/2 to 85/15) to obtain a title compound (0.100 g).

### Reference Example B-26

Reference Example B-26 was synthesized in the same method as in Reference Example B-2 except that Reference Example B-25 was used in place of Reference Example B-1.

### Reference Example B-27

Reference Example B-27 was synthesized in the same method as in Reference Example B-25 except that 2-fluorophenol was used in place of 2-chlorophenol.

### Reference Example B-28

Reference Example B-28 was synthesized in the same method as in Reference Example B-2 except that Reference Example B-27 was used in place of Reference Example B-1.

### Reference Example B-29

### 2-bromo-6-(3-(difluoromethoxy)phenoxy)-N-methylaniline

A mixture of potassium hydroxide (5.62 g) and water (25 mL) was added to a mixture of 3-(benzyloxy)phenol (1.01 g) and MeCN (25 mL) at room temperature. Diethyl (bromodifluoromethyl)phosphonate (2.69 g) was added to the mixture under ice cooling, and the mixture was stirred at room temperature for 10 minutes. The mixture was extracted with DCM, and the extract was then washed with water and concentrated under reduced pressure.

Ethanol (10 mL) and 10% Pd/C (0.204 g) were added to the residue, and the mixture was stirred at room temperature under a hydrogen atmosphere for 13 hours. The reaction mixture was filtered through Celite, and the filtrate was concentrated under reduced pressure.

Potassium carbonate (0.794 g) was added to a mixture of the residue, 1-bromo-3-fluoro-2-nitrobenzene (0.630 g) and DMF (6 mL), and the mixture was stirred at 100°C for 1 hour. The reaction mixture was cooled to room temperature, and water, ethyl acetate and toluene were then added. The mixture was extracted with ethyl acetate, and the extract was then washed with water, and concentrated under reduced pressure. The residue was purified by silica gel column chromatography (elution solvent: n-hexane/ethyl acetate=100/0 to 70/30) to obtain 1-bromo-3-(3-(difluoromethoxy)phenoxy)-2-nitrobenzene (0.871 g).

Ammonium chloride (3.21 g) and iron powder (0.671 g) were added to a mixture of the obtained Compound (0.866 g), ethanol (9 mL) and water (3 mL), and the mixture was stirred at 80°C for 2.5 hours. The reaction mixture was cooled with water and then filtered through Celite. Water was added to the filtrate, the mixture was then extracted with ethyl acetate, and the extract was concentrated under reduced pressure. The residue was purified by silica gel column chromatography (elution solvent: n-hexane/ethyl acetate=100/0 to 50/50) to obtain 2-bromo-6-(3-(difluoromethoxy)phenoxy)aniline (0.758 g).

Methyllithium (1.04 mol/L in diethyl ether) (2.6 mL) was added to a mixture of the obtained Compound (0.751 g) and THF (8 mL) at -78°C, and the mixture was stirred at the same temperature for 1 hour. A mixture of methyl iodide (0.387 g) and THF (5 mL) was added to the reaction mixture at -78°C, and the mixture was then stirred under water cooling for 1 hour. A saturated ammonium chloride aqueous solution and water were added to the reaction mixture under water cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.687 g).

### Reference Example B-30

Reference Example B-30 was synthesized in the same method as in Reference Example B-2 except that Reference Example B-29 was used in place of Reference Example B-1.

### Reference Example B-31

Reference Example B-31 was synthesized in the same method as in Reference Example B-23 except that 1-bromo-4-chloro-3-fluoro-2-nitrobenzene was used in place of 1-bromo-3-fluoro-2-nitrobenzene and 3-methoxyphenol was used in place of 5-fluoro-2-(trifluoromethyl)phenol.

### Reference Example B-32

Reference Example B-32 was synthesized in the same method as in Reference Example B-2 except that Reference Example B-31 was used in place of Reference Example B-1.

### Reference Example B-33

Reference Example B-33 was synthesized in the same method as in Reference Example B-23 except that 1-bromo-4-chloro-3-fluoro-2-nitrobenzene was used in place of 1-bromo-3-fluoro-2-nitrobenzene, and 3-fluorophenol was used in place of 5-fluoro-2-(trifluoromethyl)phenol.

### Reference Example B-34

Reference Example B-34 was synthesized in the same method as in Reference Example B-2 except that Reference Example B-33 was used in place of Reference Example B-1.

### Reference Example B-35

### 6-bromo-3-chloro-2-(2-(difluoromethyl)phenoxy)-N-methylaniline

Potassium carbonate (0.217 g) was added to a mixture of 1-bromo-4-chloro-3-fluoro-2-nitrobenzene (0.200 g), 2-hydroxybenzaldehyde (0.096 g) and NMP (2 mL), and the mixture was stirred at 110°C for 3 hours. The reaction mixture was cooled to room temperature, and 1 mol/L hydrochloric acid, ethyl acetate and n-hexane were then added. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain 2-(3-bromo-6-chloro-2-nitrophenoxy)benzaldehyde (0.160 g).

Bis(2-methoxyethyl)aminosulfur trifluoride (0.199 g) was added to a mixture of the obtained Compound (0.160 g) and DCM (3 mL) under ice cooling, and the mixture was stirred at 50°C for 2 hours. Bis(2-methoxyethyl)aminosulfur trifluoride (0.496 g) was added to the reaction mixture, and the mixture was stirred at 50°C for 3 hours. A saturated sodium bicarbonate aqueous solution was added to the reaction mixture under ice cooling. The mixture was extracted with DCM, and the extract was concentrated under reduced pressure. The residue was purified by Method A (elution solvent: n-hexane/ethyl acetate=98/2 to 85/15) to obtain 1-bromo-4-chloro-3-(2-(difluoromethyl)phenoxy)-2-nitrobenzene (0.130 g).

Ammonium chloride (0.459 g) and iron powder (0.096 g) were added to a mixture of the obtained Compound (0.130 g), ethanol (3 mL) and water (1 mL), and the mixture was stirred at 80°C for 2 hours. The reaction mixture was cooled to room temperature and water was then added. The mixture was extracted with DCM, and the extract was concentrated under reduced pressure. The residue was purified by Method A (elution solvent: n-hexane/ethyl acetate=98/2 to 85/15) to obtain 6-bromo-3-chloro-2-(2-(difluoromethyl)phenoxy)aniline (0.110 g).

Methyllithium (1.06 mol/L in diethyl ether) (0.327 mL) was added to a mixture of the obtained Compound (0.110 g) and THF (1 mL) at -78°C, and the mixture was stirred at the same temperature for 1 hour. A mixture of methyl iodide (0.054 g) and THF (1 mL) was added to the reaction mixture at -78°C, and the mixture was then stirred at the same temperature for 10 minutes and under ice cooling for 1 hour. A saturated ammonium chloride aqueous solution was added to the reaction mixture under ice cooling. The mixture was extracted with DCM, and the extract was then concentrated under reduced pressure. The residue was purified by Method A (elution solvent: n-hexane/ethyl acetate=98/2 to 85/15) to obtain a title compound (0.085 g).

### Reference Example B-36

Reference Example B-36 was synthesized in the same method as in Reference Example B-2 except that Reference Example B-35 was used in place of Reference Example B-1.

### Reference Example B-37

### 2-bromo-6-(2-(difluoromethyl)-5-fluorophenoxy)-N-methylaniline

Potassium carbonate (1.30 g) was added to a mixture of 1-bromo-3-fluoro-2-nitrobenzene (1.04 g), 4-fluoro-2-hydroxybenzaldehyde (0.701 g) and DMF (10 mL), and the mixture was stirred at 100°C for 4 hours. The reaction mixture was cooled to room temperature, and water, ethyl acetate and MTBE were then added. The mixture was extracted with ethyl acetate, and the extract was then washed with water, and concentrated under reduced pressure. The residue was purified by silica gel column chromatography (elution solvent: n-hexane/ethyl acetate=100/0 to 60/40) to obtain 2-(3-bromo-2-nitrophenoxy)-4-fluorobenzaldehyde (0.455 g).

Bis(2-methoxyethyl)aminosulfur trifluoride (0.587 g) was added to a mixture of the obtained Compound (0.361 g), DCM (4 mL) and ethanol (0.012 g), and the mixture was stirred at 50°C for 2.5 hours. The reaction mixture was added to a 5 mol/L sodium hydroxide aqueous solution cooled with ice. The mixture was extracted with DCM, and the extract was concentrated under reduced pressure. The residue was purified by silica gel column chromatography (elution solvent: n-hexane/ethyl acetate=100/0 to 50/50) to obtain 1-bromo-3-(2-(difluoromethyl)-5-fluorophenoxy)-2-nitrobenzene (0.359 g).

Ammonium chloride (1.06 g) and iron powder (0.223 g) were added to a mixture of the obtained Compound (0.286 g), ethanol (3 mL) and water (1 mL), and the mixture was stirred at 80°C for 1 hour. The reaction mixture was cooled with water and then filtered through Celite. Water was added to the filtrate, the mixture was then extracted with ethyl acetate, and the extract was concentrated under reduced pressure. The residue was purified by silica gel column chromatography (elution solvent: n-hexane/ethyl acetate=100/0 to 60/40) to obtain 2-bromo-6-(2-(difluoromethyl)-5-fluorophenoxy)aniline (0.256 g).

Methyllithium (1.04 mol/L in diethyl ether) (1.0 mL) was added to a mixture of the obtained Compound (0.247 g) and THF (3 mL) at -78°C, and the mixture was stirred at the same temperature for 1 hour. A mixture of methyl iodide (0.148 g) and THF (2 mL) was added to the reaction mixture at -78°C, and the mixture was then stirred under water cooling for 18 hours. A saturated ammonium chloride aqueous solution and water were added to the reaction mixture under water cooling. The mixture was extracted with DCM, and the extract was concentrated under reduced pressure. The residue was purified by silica gel column chromatography (elution solvent: n-hexane/ethyl acetate=100/0 to 65/35), and then APS column chromatography (elution solvent: n-hexane/ethyl acetate=100/0 to 80/20) to obtain a title compound (0.150 g).

### Reference Example B-38

### (3R)-3-amino-8-(2-(difluoromethyl)-5-fluorophenoxy)-l-methyl-1,2,3,4-tetrahydroquinolin-2-one

Under an argon atmosphere, zinc (0.054 g) was added to a mixture of methyl (S)-2-((tert-butoxycarbonyl)amino)-3-iodopropanate (0.145 g) and DMF (1 mL), and the mixture was stirred at room temperature for 2 hours. A mixture of Reference Example B-37 (0.126 g) and DMF (2 mL), palladium(II) acetate (0.005 g) and Xphos (0.017 g) were added to the reaction mixture, and the mixture was stirred at room temperature for 15 hours. A saturated ammonium chloride aqueous solution, water and ethyl acetate were added to the reaction mixture. The mixture was filtered through Celite, and the filtrate was extracted with ethyl acetate. The extract was washed with water and concentrated under reduced pressure.

TFA (0.829 g) was added to a mixture of the residue and DCM (2 mL) under ice cooling, and the mixture was stirred at room temperature for 3 hours. A 5 mol/L sodium hydroxide aqueous solution (1.45 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=50/50/0 to 0/100/0 to 0/80/20) to obtain a title compound (0.025 g).

### Reference Example B-39

Reference Example B-39 was synthesized in the same method as in Reference Example B-37 except that 5-fluoro-2-hydroxybenzaldehyde was used in place of 4-fluoro-2-hydroxybenzaldehyde.

### Reference Example B-40

Reference Example B-40 was synthesized in the same method as in Reference Example B-38 except that Reference Example B-39 was used in place of Reference Example B-37.

### Reference Example B-41

Reference Example B-41 was synthesized in the same method as in Reference Example B-13 except that 2-chloro-5-methoxyphenol was used in place of 2-chloro-5-fluorophenol.

### Reference Example B-42

Reference Example B-42 was synthesized in the same method as in Reference Example B-22 except that Reference Example B-41 was used in place of Reference Example B-21.

### Reference Example B-43

Reference Example B-43 was synthesized in the same method as in Reference Example B-13 except that 1-bromo-3-fluoro-4-methyl-2-nitrobenzene was used in place of 1-bromo-3-fluoro-2-nitrobenzene and 2-chlorophenol was used in place of 2-chloro-5-fluorophenol.

### Reference Example B-44

Reference Example B-44 was synthesized in the same method as in Reference Example B-22 except that Reference Example B-43 was used in place of Reference Example B-21.

### Reference Example B-45

Reference Example B-45 was synthesized in the same method as in Reference Example B-13 except that 1-(benzyloxy)-4-bromo-2-fluoro-3-nitrobenzene was used in place of 1-bromo-3-fluoro-2-nitrobenzene, and 2-chlorophenol was used in place of 2-chloro-5-fluorophenol.

### Reference Example B-46

Reference Example B-46 was synthesized in the same method as in Reference Example B-22 except that Reference Example B-45 was used in place of Reference Example B-21.

### Reference Example B-47

Reference Example B-47 was synthesized in the same method as in Reference Example B-13 except that 2-chlorophenol was used in place of 2-chloro-5-fluorophenol, and ethyl iodide was used in place of methyl iodide.

### Reference Example B-48

Reference Example B-48 was synthesized in the same method as in Reference Example B-22 except that Reference Example B-47 was used in place of Reference Example B-21.

### Reference Example B-49

### 2-bromo-6-(2-chloro-4,5-difluorophenoxy)-N-methylaniline

Potassium carbonate (0.377 g) was added to a mixture of 1-bromo-3-fluoro-2-nitrobenzene (0.300 g), 2-chloro-4,5-difluorophenol (0.236 g) and NMP (3 mL), and the mixture was stirred at 110°C for 2 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure.

Ammonium chloride (1.82 g) and iron powder (0.381 g) were added to a mixture of the residue, ethanol (3 mL) and water (1 mL), and the mixture was stirred at 80°C for 2 hours. The reaction mixture was cooled to room temperature and water was then added. The mixture was extracted with DCM, and the extract was then concentrated under reduced pressure. The residue was purified by silica gel column chromatography (elution solvent: n-hexane/ethyl acetate=98/2 to 85/15) to obtain 2-bromo-6-(2-chloro-4,5-difluorophenoxy)aniline (0.160 g).

Methyllithium (1.04 mol/L in diethyl ether) (0.506 mL) was added to a mixture of the obtained Compound (0.160 g) and THF (1 mL) at -78°C, and the mixture was stirred at the same temperature for 1 hour. A mixture of methyl iodide (0.081 g) and THF (1 mL) was added to the reaction mixture at -78°C, and the mixture was then stirred at the same temperature for 10 minutes and under ice cooling for 1 hour. A saturated ammonium chloride aqueous solution was added to the reaction mixture under ice cooling. The mixture was extracted with DCM, and the extract was then concentrated under reduced pressure. The residue was purified by APS column chromatography (elution solvent: n-hexane/ethyl acetate=98/2 to 85/15) to obtain a title compound (0.135 g).

### Reference Example B-50

Reference Example B-50 was synthesized in the same method as in Reference Example B-2 except that Reference Example B-49 was used in place of Reference Example B-1.

### Reference Example B-51

Reference Example B-51 was synthesized in the same method as in Reference Example B-49 except that 1-bromo-3,4-difluoro-2-nitrobenzene was used in place of 1-bromo-3-fluoro-2-nitrobenzene, and 2-(trifluoromethyl)phenol was used in place of 2-chloro-4,5-difluorophenol.

### Reference Example B-52

### (3R)-3-amino-7-fluoro-1-methyl-8-(2-(trifluoromethyl)phenoxy)-1,2,3,4-tetrahydroquinolin-2-one

Zinc (0.126 g) was added to a mixture of methyl (S)-2-((tert-butoxycarbonyl)amino)-3-iodopropanate (0.347 g) and DMF (2 mL), and the mixture was stirred at room temperature for 1 hour. Reference Example B-51 (0.320 g), palladium(II) acetate (0.010 g) and Xphos (0.042 g) were added to the reaction mixture, and the mixture was stirred at room temperature for 15 hours. A saturated ammonium chloride aqueous solution and ethyl acetate were added to the reaction mixture. The mixture was filtered through Celite, and the filtrate was extracted with ethyl acetate. The extract was washed with water and a saturated saline solution, dried with anhydrous sodium sulfate, and then concentrated under reduced pressure.

TFA (2.00 g) was added to a mixture of the residue and DCM (2 mL) under ice cooling, and the mixture was stirred at room temperature for 4 hours. A 2 mol/L sodium hydroxide aqueous solution (8.8 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=59/39/2 to 0/98/2), and then silica gel column chromatography (elution solvent: ethyl acetate/methanol=98/2 to 80/20) to obtain a title compound (0.099 g).

### Reference Example B-53

Reference Example B-53 was synthesized in the same method as in Reference Example B-49 except that 1-bromo-4-chloro-3-fluoro-2-nitrobenzene was used in place of 1-bromo-3-fluoro-2-nitrobenzene, and 2-chloro-5-fluorophenol was used in place of 2-chloro-4,5-difluorophenol.

### Reference Example B-54

Reference Example B-54 was synthesized in the same method as in Reference Example B-2 except that Reference Example B-53 was used in place of Reference Example B-1.

### Reference Example B-55

### 6-bromo-2-(2-(difluoromethyl)phenoxy)-N,3-dimethylaniline

Potassium carbonate (0.364 g) was added to a mixture of 1-bromo-3-fluoro-4-methyl-2-nitrobenzene (0.306 g), 2-hydroxybenzaldehyde (0.167 g) and DMF (3 mL), and the mixture was stirred at 100°C for 2 hours. The reaction mixture was cooled to room temperature, and water, ethyl acetate and toluene were then added. The mixture was extracted with ethyl acetate, and the extract was then washed with water, and concentrated under reduced pressure. The residue was purified by silica gel column chromatography (elution solvent: n-hexane/ethyl acetate=100/0 to 70/30) to obtain 2-(3-bromo-6-methyl-2-nitrophenoxy)benzaldehyde (0.306 g).

Bis(2-methoxyethyl)aminosulfur trifluoride (0.866 g) and ethanol (0.018 g) were added to a mixture of the obtained Compound (0.306 g) and DCM (3 mL), and the mixture was stirred at room temperature for 14 hours. A potassium carbonate aqueous solution cooled with ice was added to the reaction mixture. The mixture was extracted with DCM, and the extract was concentrated under reduced pressure. The residue was purified by silica gel column chromatography (elution solvent: n-hexane/ethyl acetate=100/0 to 65/35) to obtain 1-bromo-3-(2-(difluoromethyl)phenoxy)-4-methyl-2-nitrobenzene (0.298 g).

Ammonium chloride (1.75 g) and iron powder (0.365 g) were added to a mixture of the obtained Compound (0.298 g), ethanol (4.5 mL) and water (1.5 mL), and the mixture was stirred at 80°C for 1.5 hours. The reaction mixture was cooled to room temperature and then filtered through Celite. Water was added to the filtrate, the mixture was then extracted with ethyl acetate, and the extract was concentrated under reduced pressure. The residue was purified by silica gel column chromatography (elution solvent: n-hexane/ethyl acetate=100/0 to 70/30) to obtain 6-bromo-2-(2-(difluoromethyl)phenoxy)-3-methylaniline (0.237 g).

Methyllithium (1.04 mol/L in diethyl ether) (0.9 mL) was added to a mixture of the obtained Compound (0.237 g) and THF (2 mL) at -78°C, and the mixture was stirred at the same temperature for 1 hour. A mixture of methyl iodide (0.143 g) and THF (1 mL) was added to the reaction mixture at -78°C, and the mixture was then stirred under ice cooling for 2 hours. A saturated ammonium chloride aqueous solution and water were added to the reaction mixture under water cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.239 g).

### Reference Example B-56

Reference Example B-56 was synthesized in the same method as in Reference Example B-6 except that Reference Example B-55 was used in place of Reference Example B-5.

### Reference Example B-57

### 6-bromo-2-(2-chloro-5-fluorophenoxy)-N,3-dimethylaniline

Potassium carbonate (0.360 g) was added to a mixture of 1-bromo-3-fluoro-4-methyl-2-nitrobenzene (0.305 g), 2-chloro-5-fluorophenol (0.200 g) and DMF (10 mL), and the mixture was stirred at 100°C for 2 hours. The reaction mixture was cooled to room temperature, and water, ethyl acetate and toluene were then added. The mixture was extracted with ethyl acetate. The extract was washed with water and then concentrated under reduced pressure. The residue was purified by silica gel column chromatography (elution solvent: n-hexane/ethyl acetate=100/0 to 55/45) to obtain 1-bromo-3-(2-chloro-5-fluorophenoxy)-4-methyl-2-nitrobenzene (0.439 g).

Ammonium chloride (1.74 g) and iron powder (0.362 g) were added to a mixture of the obtained Compound (0.439 g), ethanol (4.5 mL) and water (1.5 mL), and the mixture was stirred at 80°C for 1.5 hours. The reaction mixture was cooled to room temperature and then filtered through Celite. Water was added to the filtrate, the mixture was then extracted with ethyl acetate, and the extract was concentrated under reduced pressure. The residue was purified by silica gel column chromatography (elution solvent: n-hexane/ethyl acetate=100/0 to 70/30) to obtain 6-bromo-2-(2-chloro-5-fluorophenoxy)-3-methylaniline (0.355 g).

Methyllithium (1.04 mol/L in diethyl ether) (1.3 mL) was added to a mixture of the obtained Compound (0.355 g) and THF (3 mL) at -78°C, and the mixture was stirred at the same temperature for 1 hour. A mixture of methyl iodide (0.214 g) and THF (1.5 mL) was added to the reaction mixture at -78°C, and the mixture was then stirred under ice cooling for 2 hours. A saturated ammonium chloride aqueous solution and water were added to the reaction mixture under water cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.349 g).

### Reference Example B-58

### (3R)-3-amino-8-(2-chloro-5-fluorophenoxy)-1,7-dimethyl-1,2,3,4-tetrahydroquinolin-2-one

Under an argon atmosphere, zinc (0.141 g) was added to a mixture of methyl (S)-2-((tert-butoxycarbonyl)amino)-3-iodopropanate (0.393 g) and DMF (3 mL), and the mixture was stirred at room temperature for 1.5 hours. A mixture of Reference Example B-57 (0.338 g) and DMF (1 mL), palladium(II) acetate (0.012 g) and Xphos (0.047 g) were added to the reaction mixture, and the mixture was stirred at room temperature for 13 hours. A saturated ammonium chloride aqueous solution, water and ethyl acetate were added to the reaction mixture. The mixture was filtered through Celite, and the filtrate was extracted with ethyl acetate. The extract was washed with water and concentrated under reduced pressure.

TFA (2.24 g) was added to a mixture of the residue and DCM (4 mL) under ice cooling, and the mixture was stirred at room temperature for 3 hours. A 5 mol/L sodium hydroxide aqueous solution (3.9 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=50/50/0 to 0/100/0 to 0/80/20) to obtain a title compound (0.135 g).

### Reference Example B-59

Reference Example B-59 was synthesized in the same method as in Reference Example B-49 except that 2-chlorophenol was used in place of 2-chloro-4,5-difluorophenol.

### Reference Example B-60

Reference Example B-60 was synthesized in the same method as in Reference Example B-52 except that Reference Example B-59 was used in place of Reference Example B-51.

### Reference Example B-61

### 2-bromo-6-(cyclohexyloxy)-N-methylaniline

Sodium hydride (about 60%) (0.280 g) was added to a mixture of 1-bromo-3-fluoro-2-nitrobenzene (0.770 g), cyclohexanol (0.386 g) and DMF (5 mL), and the mixture was stirred at room temperature for 1 hour. Water, ethyl acetate and n-hexane were added to the reaction mixture. The mixture was extracted with ethyl acetate, and the extract was washed with water and a saturated saline solution. The extract was dried with anhydrous sodium sulfate and then concentrated under reduced pressure.

Ammonium chloride (4.68 g) and iron powder (0.977 g) were added to a mixture of the residue, ethanol (3 mL) and water (1 mL) at room temperature, and the mixture was stirred at 80°C for 1.5 hours. The reaction mixture was cooled to room temperature and water was then added. The mixture was extracted with DCM, and the extract was concentrated under reduced pressure. The residue was purified by silica gel column chromatography (elution solvent: n-hexane/ethyl acetate=100/0 to 70/30) to obtain 2-bromo-6-(cyclohexyloxy)aniline (0.458 g).

Methyllithium (1.04 mol/L in diethyl ether) (1.8 mL) was added to a mixture of the obtained Compound (0.458 g) and THF (8 mL) at -78°C, and the mixture was stirred at the same temperature for 1 hour. A mixture of methyl iodide (0.385 g) and THF (2 mL) was added to the reaction mixture at -78°C, and the mixture was then stirred at the same temperature for 10 minutes and at room temperature for 1 hour. A saturated ammonium chloride aqueous solution and ethyl acetate were added to the reaction mixture under ice cooling. Methyllithium (1.04 mol/L in diethyl ether) (1.8 mL) was added to the reaction mixture at room temperature, and the mixture was stirred at the same temperature for 1 hour. The mixture was extracted with ethyl acetate, and the extract was washed with a saturated saline solution. The extract was dried with anhydrous sodium sulfate and then concentrated under reduced pressure. The residue was purified by APS column chromatography (elution solvent: n-hexane/ethyl acetate=100/0 to 80/20) to obtain a title compound (0.456 g).

### Reference Example B-62

### (3R)-3-amino-8-(cyclohexyloxy)-1-methyl-1,2,3,4-tetrahydroquinolin-2-one

Under an argon atmosphere, zinc (0.231 g) was added to a mixture of methyl (S)-2-((tert-butoxycarbonyl)amino)-3-iodopropanate (0.634 g) and DMF (10 mL), and the mixture was stirred at room temperature for 2 hours. Reference Example B-61 (0.456 g), palladium(II) acetate (0.018 g) and Xphos (0.077 g) were added to the reaction mixture, and the mixture was stirred at room temperature for 14 hours. The reaction mixture was stirred at 80°C for 1 hour. The reaction mixture was cooled to room temperature, and a saturated ammonium chloride aqueous solution and ethyl acetate were then added. The mixture was filtered through Celite, and the filtrate was extracted with ethyl acetate. The extract was washed with water and a saturated saline solution, dried with anhydrous sodium sulfate, and then concentrated under reduced pressure.

TFA (3.66 g) was added to a mixture of the residue and DCM (5 mL) under ice cooling, and the mixture was stirred at room temperature for 1 hour. A 5 mol/L sodium hydroxide aqueous solution (10 mL) was added to the reaction mixture under ice cooling. The mixture was extracted with DCM, and the extract was concentrated under reduced pressure. The residue was purified by APS column chromatography (elution solvent: ethyl acetate/methanol=100/0 to 50/50) to obtain a title compound (0.016 g).

### Reference Example B-63

### 2-bromo-6-(cyclopentyloxy)-N-methylaniline

Sodium hydride (about 60%) (0.091 g) was added to a mixture of cyclopentanol (0.196 g) and DMF (4 mL), and the mixture was stirred at room temperature for 30 minutes. 1-bromo-3-fluoro-2-nitrobenzene (0.500 g) was added to the reaction mixture, and the mixture was stirred at room temperature for 30 minutes. A saturated ammonium chloride aqueous solution, ethyl acetate and n-hexane were added to the reaction mixture under ice cooling. The mixture was extracted with ethyl acetate, and the extract was then washed with water, and concentrated under reduced pressure. The residue was purified by silica gel column chromatography (elution solvent: n-hexane/ethyl acetate=100/0 to 70/30), and then APS column chromatography (elution solvent: n-hexane/ethyl acetate=100/0 to 70/30) to obtain 1-bromo-3-(cyclopentyloxy)-2-nitrobenzene (0.462 g).

Ammonium chloride (2.16 g) and iron powder (0.450 g) were added to a mixture of the obtained Compound (0.462 g), ethanol (5 mL) and water (2 mL), and the mixture was stirred at 90°C for 2 hours. The reaction mixture was cooled to room temperature and water was then added. The mixture was extracted with DCM, and the extract was then concentrated under reduced pressure. The residue was purified by silica gel column chromatography (elution solvent: n-hexane/ethyl acetate=98/2 to 0/100) to obtain 2-bromo-6-(cyclopentyloxy)aniline (0.382 g).

Methyllithium (1.04 mol/L in diethyl ether) (1.4 mL) was added to a mixture of the obtained Compound (0.382 g) and THF (3 mL) at -78°C, and the mixture was stirred at the same temperature for 1 hour. A mixture of methyl iodide (0.284 g) and THF (1 mL) was added to the reaction mixture at -78°C, and the mixture was stirred at the same temperature for 15 minutes and under ice cooling for 1 hour. A saturated ammonium chloride aqueous solution was added to the reaction mixture under ice cooling. The mixture was extracted with DCM, and the extract was then concentrated under reduced pressure. The residue was purified by silica gel column chromatography (elution solvent: n-hexane/ethyl acetate=98/2 to 90/10) to obtain a title compound (0.218 g).

### Reference Example B-64

Reference Example B-64 was synthesized in the same method as in Reference Example B-6 except that Reference Example B-63 was used in place of Reference Example B-5.

### Reference Example B-65

### 6-bromo-2-(2-(difluoromethyl)phenoxy)-3-fluoro-N-methylaniline

Potassium carbonate (1.17 g) was added to a mixture of 1-bromo-3,4-difluoro-2-nitrobenzene (1.01 g), 2-hydroxybenzaldehyde (0.540 g) and DMF (10 mL), and the mixture was stirred at 100°C for 1 hour. The reaction mixture was cooled to room temperature, and water, ethyl acetate and toluene were then added. The mixture was extracted with ethyl acetate, and the extract was then washed with water, and concentrated under reduced pressure.

Bis(2-methoxyethyl)aminosulfur trifluoride (3.76 g) was added to a mixture of the residue, DCM (10 mL) and ethanol (0.078 g), and the mixture was stirred at 40°C for 3.5 hours. The reaction mixture was added to a sodium hydroxide aqueous solution cooled with ice. The mixture was extracted with DCM, and the extract was concentrated under reduced pressure. Ethyl acetate, MTBE and water were added to the residue. The mixture was extracted with ethyl acetate, and the extract was then concentrated under reduced pressure. The residue was purified by silica gel column chromatography (elution solvent: n-hexane/ethyl acetate=100/0 to 60/40) to obtain 1-bromo-3-(2-(difluoromethyl)phenoxy)-4-fluoro-2-nitrobenzene (1.07 g).

Ammonium chloride (3.93 g) and iron powder (0.821 g) were added to a mixture of the obtained Compound (1.07 g), ethanol (10 mL) and water (3 mL), and the mixture was stirred at 80°C for 1.5 hours. The reaction mixture was cooled to room temperature and then filtered through Celite. Water was added to the filtrate, the mixture was then extracted with ethyl acetate, and the extract was concentrated under reduced pressure. The residue was purified by silica gel column chromatography (elution solvent: n-hexane/ethyl acetate=100/0 to 70/30) to obtain 6-bromo-2-(2-(difluoromethyl)phenoxy)-3-fluoroaniline (0.963 g).

Under an argon atmosphere, methyllithium (1.04 mol/L in diethyl ether) (3.6 mL) was added to a mixture of the obtained Compound (0.957 g) and THF (12 mL) at -78°C, and the mixture was stirred at the same temperature for 30 minutes. Methyl iodide (0.573 g) was added to the reaction mixture at -78°C, and the mixture was then stirred at the same temperature for 10 minutes and under ice cooling for 30 minutes. A saturated ammonium chloride aqueous solution and ethyl acetate were added to the reaction mixture under ice cooling. The mixture was extracted with ethyl acetate, and the extract was then washed with water, and concentrated under reduced pressure. The residue was purified by APS column chromatography (elution solvent: n-hexane/ethyl acetate=100/0 to 95/5) to obtain a title compound (0.915 g).

### Reference Example B-66

Reference Example B-66 was synthesized in the same method as in Reference Example B-22 except that Reference Example B-65 was used in place of Reference Example B-21.

### Reference Example B-67

### 6-bromo-2-(2-chloro-5-fluorophenoxy)-3-fluoro-N-methylaniline

Potassium carbonate (1.17 g) was added to a mixture of 1-bromo-3,4-difluoro-2-nitrobenzene (1.02 g), 2-chloro-5-fluorophenol (0.651 g) and DMF (10 mL), and the mixture was stirred at 100°C for 1 hour. The reaction mixture was cooled to room temperature, and water, ethyl acetate and toluene were then added. The mixture was extracted with ethyl acetate, and the extract was then washed with water, and concentrated under reduced pressure.

Ammonium chloride (5.72 g) and iron powder (1.20 g) were added to a mixture of the residue, ethanol (15 mL) and water (5 mL), and the mixture was stirred at 80°C for 1.5 hours. The reaction mixture was cooled to room temperature and then filtered through Celite. Water was added to the filtrate, and the mixture was extracted with ethyl acetate. The extract was concentrated under reduced pressure. The residue was purified by silica gel column chromatography (elution solvent: n-hexane/ethyl acetate=100/0 to 60/40) to obtain 6-bromo-2-(2-chloro-5-fluorophenoxy)-3-fluoroaniline (0.916 g).

Methyllithium (1.04 mol/L in diethyl ether) (3.6 mL) was added to a mixture of the obtained Compound (0.908 g) and THF (10 mL) at -78°C, and the mixture was stirred at the same temperature for 1 hour. A mixture of methyl iodide (0.539 g) and THF (5 mL) was added to the reaction mixture at -78°C, and the mixture was then stirred under water cooling for 1 hour. A saturated ammonium chloride aqueous solution and water were added to the reaction mixture under water cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.633 g).

### Reference Example B-68

Reference Example B-68 was synthesized in the same method as in Reference Example B-22 except that Reference Example B-67 was used in place of Reference Example B-21.

### Reference Example B-69

Reference Example B-69 was synthesized in the same method as in Reference Example B-13 except that 1-bromo-3-fluoro-4-methyl-2-nitrobenzene was used in place of 1-bromo-3-fluoro-2-nitrobenzene, and 2-fluorophenol was used in place of 2-chloro-5-fluorophenol.

### Reference Example B-70

### (3R)-3-amino-8-(2-fluorophenoxy)-1,7-dimethyl-1,2,3,4-tetrahydroquinolin-2-one

Under an argon atmosphere, zinc (0.086 g) was added to a mixture of methyl (S)-2-((tert-butoxycarbonyl)amino)-3-iodopropanate (0.257 g) and DMF (4 mL), and the mixture was stirred at room temperature for 2 hours. A mixture of Reference Example B-69 (0.186 g) and DMF (1 mL), palladium(II) acetate (0.007 g) and Xphos (0.029 g) were added to the reaction mixture, and the mixture was stirred at room temperature for 16 hours. A saturated ammonium chloride aqueous solution and diethyl ether were added to the reaction mixture. The mixture was filtered through Celite, and the filtrate was extracted with diethyl ether. The extract was washed with water and dried with anhydrous magnesium sulfate and then concentrated under reduced pressure.

TFA (1.37 g) was added to a mixture of the residue and DCM (4 mL) under ice cooling, and the mixture was stirred at room temperature for 3 hours. A 5 mol/L sodium hydroxide aqueous solution (2.5 mL) was added to the reaction mixture under ice cooling. The mixture was extracted with DCM, and the extract was concentrated under reduced pressure. The residue was purified by APS column chromatography (elution solvent: n-hexane/ethyl acetate/methanol=40/60/0 to 0/100/0 to 0/60/40) to obtain a title compound (0.031 g).

### Reference Example B-71

Reference Example B-71 was synthesized in the same method as in Reference Example B-13 except that 1-bromo-3-fluoro-4-methyl-2-nitrobenzene was used in place of 1-bromo-3-fluoro-2-nitrobenzene, and 3-fluorophenol was used in place of 2-chloro-5-fluorophenol.

### Reference Example B-72

Reference Example B-72 was synthesized in the same method as in Reference Example B-22 except that Reference Example B-71 was used in place of Reference Example B-21.

### Reference Example B-73

### 2-bromo-N-methyl-6-(2-methylphenoxy)aniline

Potassium carbonate (0.377 g) was added to a mixture of 1-bromo-3-fluoro-2-nitrobenzene (0.300 g), 2-methylphenol (0.147 g) and DMF (3 mL), and the mixture was stirred at 110°C for 2 hours. The reaction mixture was cooled to room temperature and ethyl acetate, n-hexane and water were then added. The mixture was extracted with ethyl acetate, and the extract was washed with water and a saturated saline solution. The extract was dried with anhydrous sodium sulfate and then concentrated under reduced pressure.

Ammonium chloride (1.46 g) and iron powder (0.381 g) were added to a mixture of the residue, ethanol (3 mL) and water (1 mL), and the mixture was stirred at 80°C for 2 hours. The reaction mixture was cooled to room temperature and water was then added. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(2-methylphenoxy)aniline (0.370 g).

Methyllithium (3.1 mol/L in diethoxymethane) (0.472 mL) was added to a mixture of the obtained Compound (0.370 g) and THF (3 mL) at -78°C, and the mixture was stirred at the same temperature for 1 hour. A THF (1 mL) solution containing methyl iodide (0.227 g) was added to the reaction mixture at -78°C, and the mixture was then stirred at the same temperature for 10 minutes and for 2 hours under ice cooling. A saturated ammonium chloride aqueous solution was added to the reaction mixture under ice cooling. The mixture was extracted with DCM, and the extract was concentrated under reduced pressure. The residue was purified by APS column chromatography (elution solvent: n-hexane/ethyl acetate=98/2 to 85/15) to obtain a title compound (0.390 g).

### Reference Example B-74

### (3R)-3-amino-1-methyl-8-(2-methylphenoxy)-1,2,3,4-tetrahydroquinolin-2-one

Under an argon atmosphere, zinc (0.187 g) was added to a mixture of methyl (S)-2-((tert-butoxycarbonyl)amino)-3-iodopropanate (0.642 g) and DMF (3 mL), and the mixture was stirred at room temperature for 1 hour. Reference Example B-73 (0.380 g), palladium(II) acetate (0.015 g) and Xphos (0.062 g) were added to the reaction mixture, and the mixture was stirred at 40°C for 3 hours. The reaction mixture was cooled to room temperature, and a saturated ammonium chloride aqueous solution and ethyl acetate were then added. The mixture was filtered through Celite, and the filtrate was extracted with ethyl acetate. The extract was washed with water and a saturated saline solution, dried with anhydrous sodium sulfate, and then concentrated under reduced pressure.

TFA (2.97 g) was added to a mixture of the residue and DCM (3 mL) under ice cooling, and the mixture was stirred at room temperature for 15 hours. A 5 mol/L sodium hydroxide aqueous solution (5.2 mL) was added to the reaction mixture under ice cooling. The mixture was extracted with DCM, and the extract was concentrated under reduced pressure. The residue was purified by Method A (elution solvent: ethyl acetate/methanol=98/2 to 80/20) to obtain a title compound (0.290 g).

### Reference Example B-75

Reference Example B-75 was synthesized in the same method as in Reference Example B-73 except that 5-fluoro-2-methylphenol was used in place of 2-methylphenol.

### Reference Example B-76

Reference Example B-76 was synthesized in the same method as in Reference Example B-74 except that Reference Example B-75 was used in place of Reference Example B-73.

### Reference Example B-77

Reference Example B-77 was synthesized in the same method as in Reference Example B-73 except that 1-bromo-3-fluoro-4-methyl-2-nitrobenzene was used in place of 1-bromo-3-fluoro-2-nitrobenzene, and 2,5-difluorophenol was used in place of 2-methylphenol.

### Reference Example B-78

### (3R)-3-amino-8-(2,5-difluorophenoxy)-1,7-dimethyl-1,2,3,4-tetrahydroquinolin-2-one

Under an argon atmosphere, a mixture of methyl (S)-2-((tert-butoxycarbonyl)amino)-3-iodopropanate (0.537 g) and THF (0.7 mL) was added to a mixture of zinc (0.156 g) and DMF (1.4 mL), and the mixture was stirred at room temperature for 1 hour. A mixture of Reference Example B-77 (0.357 g) and THF (1.4 mL), palladium(II) acetate (0.012 g) and Xphos (0.052 g) were added to the reaction mixture, and the mixture was stirred at 40°C for 2 hours. A saturated ammonium chloride aqueous solution, water, ethyl acetate and n-hexane were added to the reaction mixture under ice cooling. The mixture was filtered through Celite, and the filtrate was extracted with ethyl acetate/n-hexane (1/1). The extract was washed with water and a saturated saline solution and dried with anhydrous magnesium sulfate and then concentrated under reduced pressure.

Methanesulfonic acid (0.314 g) was added to a mixture of the residue, toluene (1.7 mL) and ethanol (1.2 mL), and the mixture was stirred at 80°C for 30 minutes. A 5 mol/L sodium hydroxide aqueous solution (0.66 mL) was added to the reaction mixture under ice cooling. The mixture was extracted with DCM, and the extract was concentrated under reduced pressure. The residue was purified by Method A (elution solvent: ethyl acetate/methanol=98/2 to 80/20) to obtain a title compound (0.275 g).

### Reference Example C-1

### tert-butyl 6-bromo-2-(2-fluorophenoxy)-3-(trifluoromethyl)benzoate

A mixture of 6-bromo-2-fluoro-3-(trifluoromethyl)benzoic acid (1.00 g), Boc₂O (1.52 g), DMAP (0.043 g) and tert-butyl alcohol (10 mL) was stirred at room temperature for 13 hours. The reaction mixture was stirred at 50°C for 2 hours. The reaction mixture was cooled to room temperature and water and ethyl acetate were then added. The mixture was extracted with ethyl acetate, and the extract was washed with 1 mol/L hydrochloric acid and a saturated saline solution. The extract was dried with anhydrous sodium sulfate and concentrated under reduced pressure.

Potassium carbonate (0.403 g) was added to a mixture of the residue, 2-fluorophenol (0.163 g) and NMP (5 mL), and the mixture was stirred at 110°C for 4 hours. The reaction mixture was cooled to room temperature, and water, ethyl acetate and n-hexane were then added. The mixture was extracted with ethyl acetate, and the extract was washed with water and a saturated saline solution. The extract was dried with anhydrous sodium sulfate and concentrated under reduced pressure. The residue was purified by silica gel column chromatography (elution solvent: n-hexane/ethyl acetate=98/2 to 85/15) to obtain a title compound (0.560 g).

### Reference Example C-2

### tert-butyl N-(6-bromo-2-(2-fluorophenoxy)-3-(trifluoromethyl)phenyl)-N-methylcarbamate

A mixture of Reference Example C-1 (0.560 g) and hydrogen chloride (4 mol/L in 1,4-dioxane) (3 mL) was stirred at room temperature for 1 hour. The reaction mixture was stirred at 60°C for 3 hours. The reaction mixture was stirred at 70°C for 14 hours. Hydrogen chloride (4 mol/L in 1,4-dioxane) (2 mL) was added to the reaction mixture, and the mixture was stirred at 80°C for 6 hours. The reaction mixture was cooled to room temperature and then poured into water. The mixture was extracted with ethyl acetate, and the extract was washed with a saturated saline solution. The extract was dried with anhydrous sodium sulfate and then concentrated under reduced pressure.

DIPEA (0.171 g) and DPPA (0.365 g) were added to a mixture of the residue, toluene (3 mL) and tert-butyl alcohol (2 mL), and the mixture was stirred at 100°C for 3 hours. DPPA (0.912 g) and DIPEA (0.428 g) were added to the reaction mixture, and the mixture was stirred at 100°C for 3 hours. The reaction mixture was cooled to room temperature and a saturated sodium bicarbonate aqueous solution was then added. The mixture was extracted with ethyl acetate, and the extract was washed with a saturated saline solution. The extract was dried with anhydrous magnesium sulfate and concentrated under reduced pressure. The residue was purified by silica gel column chromatography (elution solvent: n-hexane/ethyl acetate=100/0 to 60/40), and then APS column chromatography (elution solvent: n-hexane/ethyl acetate=100/0 to 60/40) to obtain tert-butyl N-(6-bromo-2-(2-fluorophenoxy)-3-(trifluoromethyl)phenyl)carbamate (0.768 g).

Sodium hydride (about 60%) (0.066 g) was added to a mixture of the obtained Compound (0.497 g), DMF (10 mL) and methyl iodide (0.235 g) under ice cooling, and the mixture was stirred at room temperature for 1 hour. A saturated ammonium chloride aqueous solution, ethyl acetate and n-hexane were added to the reaction mixture under ice cooling. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=100/0 to 30/70) to obtain a title compound (0.248 g).

### Reference Example C-3

### (3R)-3-amino-8-(2-fluorophenoxy)-1-methyl-7-(trifluoromethyl)-1,2,3,4-tetrahydroquinolin-2-one

Under an argon atmosphere, zinc (0.077 g) was added to a mixture of methyl (S)-2-((tert-butoxycarbonyl)amino)-3-iodopropanate (0.211 g) and DMF (2 mL), and the mixture was stirred at room temperature for 2 hours. Reference Example C-2 (0.248 g), palladium(II) acetate (0.006 g) and Xphos (0.025 g) were added to the reaction mixture, and the mixture was stirred at room temperature for 2 hours. A saturated ammonium chloride aqueous solution and ethyl acetate were added to the reaction mixture. The mixture was filtered through Celite, and the filtrate was extracted with ethyl acetate. The extract was washed with water and a saturated saline solution, dried with anhydrous sodium sulfate, and then concentrated under reduced pressure.

TFA (1.22 g) was added to a mixture of the residue and DCM (2 mL) under ice cooling, and the mixture was stirred at room temperature for 15 hours. A 5 mol/L sodium hydroxide aqueous solution (5 mL) was added to the reaction mixture under ice cooling. The mixture was extracted with DCM, and the extract was concentrated under reduced pressure. The residue was purified by APS column chromatography (elution solvent: ethyl acetate/methanol=100/0 to 50/50) to obtain a title compound (0.026 g).

### Reference Example D-1

### 2-bromo-6-((2-chlorophenyl)methyl)-N-methylaniline

Isopropylmagnesium chloride (2 mol/L in THF) (2.4 mL) was added to a mixture of 1-chloro-2-iodobenzene (1.12 g) and THF (5 mL) at -40°C, and the mixture was stirred under ice cooling for 30 minutes. A mixture of 3-bromo-2-nitrobenzaldehyde (0.540 g) and THF (5 mL) was added to the reaction mixture at -40°C, and the mixture was stirred at the same temperature for 10 minutes under ice cooling for 2 hours. A saturated ammonium chloride aqueous solution was added to the reaction mixture under ice cooling. The mixture was extracted with ethyl acetate, and the extract was washed with a saturated saline solution. The extract was dried with anhydrous sodium sulfate and then concentrated under reduced pressure. The residue was purified by silica gel column chromatography (elution solvent: n-hexane/ethyl acetate=95/5 to 70/30) to obtain (3-bromo-2-nitrophenyl)(2-chlorophenyl)methanol (0.800 g).

Ammonium chloride (3.12 g) and iron powder (0.652 g) were added to a mixture of the obtained Compound (0.800 g), ethanol (6 mL) and water (2 mL), and the mixture was stirred at 80°C for 1.5 hours. The reaction mixture was cooled to room temperature and then filtered through Celite. Water was added to the filtrate, the mixture was then extracted with DCM, and the extract was concentrated under reduced pressure. The residue was purified by silica gel column chromatography (elution solvent: n-hexane/ethyl acetate=95/5 to 70/30) to obtain (2-amino-3-bromophenyl)(2-chlorophenyl)methanol (0.670 g).

TFA (2.44 g) and triethylsilane (0.498 g) were added to a mixture of the obtained Compound (0.670 g) and DCM (5 mL), and the mixture was stirred at room temperature for 13 hours. A 5 mol/L sodium hydroxide aqueous solution (4.3 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(2-chlorobenzyl)aniline (0.480 g).

Methyllithium (3.1 mol/L in diethoxymethane) (0.574 mL) was added to a mixture of the obtained Compound (0.480 g) and THF (5 mL) at -78°C, and the mixture was stirred at the same temperature for 1 hour. Methyl iodide (0.276 g) was added to the reaction mixture at -78°C, and the mixture was then stirred at the same temperature for 10 minutes and under ice cooling for 1 hour. A saturated ammonium chloride aqueous solution was added to the reaction mixture under ice cooling. The mixture was extracted with DCM, and the extract was concentrated under reduced pressure. The residue was purified by silica gel column chromatography (elution solvent: n-hexane/ethyl acetate=98/2 to 85/15) to obtain a title compound (0.460 g).

### Reference Example D-2

### (3R)-3-amino-8-((2-chlorophenyl)methyl)-1-methyl-1,2,3,4-tetrahydroquinolin-2-one

Under an argon atmosphere, zinc (0.213 g) was added to a mixture of methyl (S)-2-((tert-butoxycarbonyl)amino)-3-iodopropanate (0.731 g) and DMF (5 mL), and the mixture was stirred at room temperature for 1 hour. Reference Example D-1 (0.460 g), palladium(II) acetate (0.017 g) and Xphos (0.071 g) were added to the reaction mixture, and the mixture was stirred at 40°C for 6 hours. The reaction mixture was cooled to room temperature and a saturated ammonium chloride aqueous solution and ethyl acetate were then added. The mixture was filtered through Celite, and the filtrate was extracted with ethyl acetate. The extract was washed with water and a saturated saline solution, dried with anhydrous sodium sulfate, and then concentrated under reduced pressure.

TFA (3.38 g) was added to a mixture of the residue and DCM (5 mL), and the mixture was stirred at room temperature for 2 hours. A 5 mol/L sodium hydroxide aqueous solution (6.0 mL) was added to the reaction mixture under ice cooling. The mixture was extracted with DCM, and the extract was concentrated under reduced pressure. The residue was purified by Method A (elution solvent: ethyl acetate/methanol=98/2 to 80/20) to obtain a title compound (0.120 g).

### Reference Example D-3

### 2-bromo-6-((2-chloro-5-fluorophenyl)methyl)-N-methylaniline

Isopropylmagnesium chloride (2 mol/L in THF) (2.2 mL) was added to a mixture of 1-chloro-4-fluoro-2-iodobenzene (1.13 g) and THF (4.7 mL) at -40°C, and the mixture was stirred under ice cooling for 30 minutes. A mixture of 3-bromo-2-nitrobenzaldehyde (0.506 g) and THF (4.7 mL) was added to the reaction mixture at -40°C, and the mixture was stirred at the same temperature for 10 minutes and under ice cooling for 1.5 hours. A saturated ammonium chloride aqueous solution and water were added to the reaction mixture under ice cooling. The mixture was extracted with ethyl acetate, and the extract was washed with a saturated saline solution. The extract was dried with anhydrous magnesium sulfate and then concentrated under reduced pressure. The residue was purified by silica gel column chromatography (elution solvent: n-hexane/ethyl acetate=95/5 to 70/30) to obtain (3-bromo-2-nitrophenyl)(2-chloro-5-fluorophenyl)methanol (0.997 g).

Ammonium chloride (2.94 g) and iron powder (0.615 g) were added to a mixture of the obtained Compound (0.997 g), ethanol (5.7 mL) and water (1.9 mL), and the mixture was stirred at 80°C for 30 minutes. The reaction mixture was cooled to room temperature and then filtered through Celite. Water was added to the filtrate, and the mixture was then extracted with ethyl acetate. The extract was washed with a saturated saline solution, dried with anhydrous magnesium sulfate, and then concentrated under reduced pressure. The residue was suspended in DCM. The supernatant of the suspension was purified by silica gel column chromatography (elution solvent: n-hexane/ethyl acetate=95/5 to 70/30) to obtain (2-amino-3-bromophenyl)(2-chloro-5-fluorophenyl)methanol (0.106 g). The remaining insoluble substances in the suspension were washed with water and n-hexane, and then dried under reduced pressure to obtain (2-amino-3-bromophenyl)(2-chloro-5-fluorophenyl)methanol (0.635 g).

TFA (2.55 g) and triethylsilane (0.521 g) were added to a mixture of (2-amino-3-bromophenyl)(2-chloro-5-fluorophenyl)methanol (0.740 g) and DCM (5.4 mL), and the mixture was stirred at room temperature for 24 hours. A 5 mol/L sodium hydroxide aqueous solution (4.5 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(2-chloro-5-fluorobenzyl)aniline (0.584 g).

Methyllithium (1.04 mol/L in diethyl ether) (1.96 mL) was added to a mixture of the obtained Compound (0.584 g) and THF (5.7 mL) at -78°C, and the mixture was stirred at the same temperature for 1 hour. Methyl iodide (0.316 g) was added to the reaction mixture at - 78°C, and the mixture was then stirred at the same temperature for 10 minutes and under ice cooling for 30 minutes. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=98/2 to 85/15) to obtain a title compound (0.517 g).

### Reference Example D-4

### (3R)-3-amino-8-((2-chloro-5-fluorophenyl)methyl)-1-methyl-1,2,3,4-tetrahydroquinolin-2-one

Under an argon atmosphere, a mixture of methyl (S)-2-((tert-butoxycarbonyl)amino)-3-iodopropanate (0.777 g) and THF (1.0 mL) was added to a mixture of zinc (0.226 g) and DMF (2.0 mL), and the mixture was stirred at room temperature for 1 hour. A mixture of Reference Example D-3 (0.517 g) and THF (2.0 mL), palladium(II) acetate (0.018 g) and Xphos (0.075 g) were added to the reaction mixture, and the mixture was stirred at room temperature for 21 hours. A saturated ammonium chloride aqueous solution, water, ethyl acetate and n-hexane were added to the reaction mixture under ice cooling. The mixture was filtered through Celite, and the filtrate was extracted with ethyl acetate/n-hexane (1/1). The extract was washed with water and a saturated saline solution and dried with anhydrous magnesium sulfate and then concentrated under reduced pressure.

Methanesulfonic acid (0.454 g) was added to a mixture of the residue, toluene (2.5 mL) and ethanol (1.6 mL), and the mixture was stirred at 70°C for 2 hours. A 5 mol/L sodium hydroxide aqueous solution (1.0 mL) was added to the reaction mixture under ice cooling. The mixture was extracted with DCM, and the extract was concentrated under reduced pressure. The residue was purified by Method A (elution solvent: n-hexane/ethyl acetate/methanol=98/2/0 to 0/100/0 to 0/80/20) to obtain a title compound (0.232 g).

### Reference Example D-5

Reference Example D-5 was synthesized in the same method as in Reference Example D-1 except that 1-iodo-2-(trifluoromethyl)benzene was used in place of 1-chloro-2-iodobenzene.

### Reference Example D-6

Reference Example D-6 was synthesized in the same method as in Reference Example D-4 except that Reference Example D-5 was used in place of Reference Example D-3.

### Reference Example D-7

Reference Example D-7 was synthesized in the same method as in Reference Example D-1 except that 1-iodo-2-methylbenzene was used in place of 1-chloro-2-iodobenzene.

### Reference Example D-8

Reference Example D-8 was synthesized in the same method as in Reference Example D-4 except that Reference Example D-7 was used in place of Reference Example D-3.

### Reference Example D-9

Reference Example D-9 was synthesized in the same method as in Reference Example D-1 except that 1-(difluoromethyl)-2-iodobenzene was used in place of 1-chloro-2-iodobenzene.

### Reference Example D-10

### (3R)-3-amino-8-((2-(difluoromethyl)phenyl)methyl)-1-methyl-1,2,3,4-tetrahydroquinolin-2-one

Under an argon atmosphere, a mixture of methyl (S)-2-((tert-butoxycarbonyl)amino)-3-iodopropanate (0.221 g) and THF (0.3 mL) was added to a mixture of zinc (0.064 g) and DMF (0.6 mL), and the mixture was stirred at room temperature for 1 hour. A mixture of Reference Example D-9 (0.146 g) and THF (0.6 mL), palladium(II) acetate (0.005 g) and Xphos (0.021 g) were added to the reaction mixture, and the mixture was stirred at room temperature for 12 hours and at 40°C for 3 hours. The reaction mixture was cooled to room temperature, and a saturated ammonium chloride aqueous solution, water, ethyl acetate and n-hexane were then added to the reaction mixture. The mixture was filtered through Celite, and the filtrate was extracted with ethyl acetate/n-hexane (1/1). The extract was washed with water and then concentrated under reduced pressure.

Methanesulfonic acid (0.129 g) was added to a mixture of the residue, toluene (0.75 mL) and ethanol (0.45 mL), and the mixture was stirred at 70°C for 3 hours. The reaction mixture was cooled to room temperature and then dispensed with water and ethyl acetate. The aqueous layer was separated, ethyl acetate was added, and under stirring, a saturated sodium bicarbonate aqueous solution was then added. The organic layer was separated and washed with a saturated saline solution. The organic layer was dried with anhydrous magnesium sulfate and then concentrated under reduced pressure to obtain a title compound (0.032 g).

### Reference Example D-11

### 6-bromo-2-((2-chloro-5-fluorophenyl)methyl)-3-fluoro-N-methylaniline

Isopropylmagnesium chloride (2 mol/L in THF) (1.21 mL) was added to a mixture of 1-chloro-4-fluoro-2-iodobenzene (0.620 g) and THF (3 mL) at -40°C, and the mixture was stirred under ice cooling for 30 minutes. A mixture of 3-bromo-6-fluoro-2-nitrobenzaldehyde (0.300 g) and THF (3 mL) was added to the reaction mixture at -40°C, and the mixture was stirred at the same temperature for 10 minutes and under ice cooling for 1 hour. A saturated ammonium chloride aqueous solution and water were added to the reaction mixture under ice cooling. The mixture was extracted with ethyl acetate, and the extract was washed with a saturated saline solution. The extract was dried with anhydrous magnesium sulfate and then concentrated under reduced pressure. The residue was purified by silica gel column chromatography (elution solvent: n-hexane/ethyl acetate=100/0 to 75/25) to obtain (3-bromo-6-fluoro-2-nitrophenyl)(2-chloro-5-fluorophenyl)methanol (0.424 g).

Ammonium chloride (1.50 g) and iron powder (0.313 g) were added to a mixture of the obtained Compound (0.424 g), ethanol (3 mL) and water (1 mL), and the mixture was stirred at 80°C for 1.5 hours. The reaction mixture was cooled to room temperature and then filtered through Celite. Water was added to the filtrate, the mixture was then extracted with ethyl acetate, and the extract was washed with water and a saturated saline solution. The extract was dried with anhydrous magnesium sulfate and then concentrated under reduced pressure to obtain (2-amino-3-bromo-6-fluorophenyl)(2-chloro-5-fluorophenyl)methanol (0.328 g).

Boron trifluoride diethyl ether complex (0.098 g) and triethylsilane (0.081 g) were added to a mixture of the obtained Compound (0.161 g) and DCM (1.6 mL), and the mixture was stirred at room temperature for 15 hours. TFA (0.527 g) was added to the reaction mixture, and the mixture was refluxed for 8 hours. A 5 mol/L sodium hydroxide aqueous solution (1 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=100/0 to 85/15) to obtain 6-bromo-2-(2-chloro-5-fluorobenzyl)-3-fluoroaniline (0.073 g).

Methyllithium (3.1 mol/L in diethoxymethane) (0.078 mL) was added to a mixture of the obtained Compound (0.073 g) and THF (2 mL) at -78°C, and the mixture was stirred at the same temperature for 30 minutes. Methyl iodide (0.037 g) was added to the reaction mixture at -78°C, and the mixture was then stirred at the same temperature for 10 minutes and under ice cooling for 1 hour. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.051 g).

### Reference Example D-12

Reference Example D-12 was synthesized in the same method as in Reference Example D-10 except that Reference Example D-11 was used in place of Reference Example D-9.

### Reference Example D-13

### 6-bromo-3-chloro-2-((2-chloro-5-fluorophenyl)methyl)-N-methylaniline

Isopropylmagnesium chloride (2 mol/L in THF) (1.5 mL) was added to a mixture of 1-chloro-4-fluoro-2-iodobenzene (0.769 g) and THF (3.7 mL) at -40°C, and the mixture was stirred under ice cooling for 30 minutes. A mixture of Reference Example E-1 (0.397 g) and THF (3.7 mL) was added to the reaction mixture at -40°C, and the mixture was stirred at the same temperature for 10 minutes and under ice cooling for 30 minutes. A saturated ammonium chloride aqueous solution and water were added to the reaction mixture under ice cooling. The mixture was extracted with DCM, and the extract was concentrated under reduced pressure.

Ammonium chloride (2.01 g) and iron powder (0.419 g) were added to a mixture of the residue, ethanol (8.2 mL) and water (2.7 mL), the mixture was stirred at 80°C for 30 minutes. The reaction mixture was cooled to room temperature, and water and DCM were then added. The mixture was extracted with DCM, and the extract was concentrated under reduced pressure. DCM and n-hexane were added to the residue, and insoluble substances were collected by filtration. The obtained solid was washed with n-hexane and then dried under reduced pressure to obtain (2-amino-3-bromo-6-chlorophenyl)(2-chloro-5-fluorophenyl)methanol (0.455 g).

TFA (1.42 g) and triethylsilane (0.290 g) were added to a mixture of the obtained Compound (0.455 g) and 1,2-dichloroethane (3 mL), and the mixture was stirred at 50°C for 30 minutes and at 70°C for 2.5 hours. A 5 mol/L sodium hydroxide aqueous solution (2.5 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 6-bromo-3-chloro-2-(2-chloro-5-fluorobenzyl)aniline as a mixture with impurities. n-hexane was added to this product, and the mixture was stirred under ice cooling. Insoluble substances were removed by filtration, and washed with n-hexane, and the filtrate and the washing solution were then combined and concentrated under reduced pressure. n-hexane was added to the residue, the supernatant was separated and concentrated under reduced pressure to obtain 6-bromo-3-chloro-2-(2-chloro-5-fluorobenzyl)aniline (0.263 g).

Methyllithium (1.04 mol/L in diethyl ether) (0.718 mL) was added to a mixture of the obtained Compound (0.262 g) and THF (2.1 mL) at -78°C, and the mixture was stirred at the same temperature for 45 minutes. Methyl iodide (0.116 g) was added to the reaction mixture at -78°C, and the mixture was then stirred at the same temperature for 10 minutes and under ice cooling for 45 minutes. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=98/2 to 85/15) to obtain a title compound (0.228 g).

### Reference Example D-14

Reference Example D-14 was synthesized in the same method as in Reference Example D-4 except that Reference Example D-13 was used in place of Reference Example D-3.

### Reference Example D-15

### 6-bromo-2-((2-chlorophenyl)methyl)-N,3-dimethylaniline

Isopropylmagnesium chloride (2 mol/L in THF) (1.64 mL) was added to a mixture of 1-chloro-2-iodobenzene (0.782 g) and THF (4 mL) at -40°C and the mixture was stirred under ice cooling for 30 minutes. A mixture of Reference Example E-2 (0.400 g) and THF (4 mL) was added to the reaction mixture at -40°C, and the mixture was stirred at the same temperature for 10 minutes and under ice cooling for 1.5 hours. A saturated ammonium chloride aqueous solution and water were added to the reaction mixture under ice cooling. The mixture was extracted with DCM, and the extract was concentrated under reduced pressure.

Ammonium chloride (2.19 g) and iron powder (0.458 g) were added to a mixture of the residue, ethanol (9 mL) and water (3 mL), and the mixture was stirred at 80°C for 1.5 hours. The reaction mixture was cooled to room temperature, and water and DCM were then added. The mixture was extracted with DCM, and the extract was concentrated under reduced pressure. The residue was purified by silica gel column chromatography (elution solvent: n-hexane/ethyl acetate=95/5 to 70/30) to obtain (2-amino-3-bromo-6-methylphenyl)(2-chlorophenyl)methanol (0.390 g).

TFA (1.26 g) and triethylsilane (0.256 g) were added to a mixture of the obtained Compound (0.360 g) and 1,2-dichloroethane (5 mL), and the mixture was stirred at 70°C for 4 hours. A 5 mol/L sodium hydroxide aqueous solution (2.2 mL) was added to the reaction mixture under ice cooling, and the mixture was stirred at room temperature for 10 minutes. The mixture was extracted with DCM, and the extract was concentrated under reduced pressure. The residue was purified by Method A (elution solvent: n-hexane/ethyl acetate=98/2 to 80/20) to obtain 6-bromo-2-(2-chlorobenzyl)-3-methylaniline (0.350 g).

Methyllithium (3.1 mol/L in diethoxymethane) (0.4 mL) was added to a mixture of the obtained Compound (0.350 g) and THF (3 mL) at -78°C, and the mixture was stirred at the same temperature for 1 hour. Methyl iodide (0.192 g) was added to the reaction mixture at -78°C and the mixture was then stirred at the same temperature for 10 minutes and under ice cooling for 1 hour. A saturated ammonium chloride aqueous solution was added to the reaction mixture under ice cooling. The mixture was extracted with DCM, and the extract was concentrated under reduced pressure. The residue was purified by Method A (elution solvent: n-hexane/ethyl acetate=98/2 to 85/15) to obtain a title compound (0.330 g).

### Reference Example D-16

### (3R)-3-amino-8-((2-chlorophenyl)methyl)-1,7-dimethyl-1,2,3,4-tetrahydroquinolin-2-one

Under an argon atmosphere, zinc (0.146 g) was added to a mixture of methyl (S)-2-((tert-butoxycarbonyl)amino)-3-iodopropanate (0.502 g) and DMF (2 mL), and the mixture was stirred at room temperature for 1 hour. A mixture of Reference Example D-15 (0.330 g) and DMF (2 mL), palladium(II) acetate (0.011 g) and Xphos (0.048 g) were added to the reaction mixture, and the mixture was stirred at room temperature for 14 hours. A saturated ammonium chloride aqueous solution and ethyl acetate were added to the reaction mixture. The mixture was filtered through Celite, and the filtrate was extracted with ethyl acetate. The extract was washed with water and a saturated saline solution, dried with anhydrous sodium sulfate, and then concentrated under reduced pressure.

TFA (2.32 g) was added to a mixture of the residue and 1,2-dichloroethane (5 mL), and the mixture was stirred at 70°C for 2 hours. A 5 mol/L sodium hydroxide aqueous solution (4.1 mL) was added to the reaction mixture under ice cooling. The mixture was extracted with DCM, and the extract was concentrated under reduced pressure. The residue was purified by Method A (elution solvent: ethyl acetate/methanol=98/2 to 80/20) to obtain a title compound (0.009 g).

### Reference Example D-17

### 6-bromo-2-((2-chloro-5-fluorophenyl)methyl)-N,3-dimethylaniline

Isopropylmagnesium chloride (2 mol/L in THF) (2.05 mL) was added to a mixture of 1-chloro-4-fluoro-2-iodobenzene (1.05 g) and THF (5 mL) at -40°C, and the mixture was stirred under ice cooling for 30 minutes. A mixture of Reference Example E-2 (0.500 g) and THF (5 mL) was added to the reaction mixture at -40°C, and the mixture was stirred at the same temperature for 10 minutes and under ice cooling for 1.5 hours. A saturated ammonium chloride aqueous solution and water were added to the reaction mixture under ice cooling. The mixture was extracted with DCM, and the extract was concentrated under reduced pressure.

Ammonium chloride (2.74 g) and iron powder (0.572 g) were added to a mixture of the residue, ethanol (9 mL) and water (3 mL), and the mixture was stirred at 80°C for 1.5 hours. The reaction mixture was cooled to room temperature, and water and DCM were then added. The mixture was extracted with DCM, and the extract was concentrated under reduced pressure. The residue was suspended in n-hexane, and the solid was collected by filtration. The obtained solid was dried under reduced pressure to obtain (2-amino-3-bromo-6-methylphenyl)(2-chloro-5-fluorophenyl)methanol (0.540 g).

TFA (1.79 g) and triethylsilane (0.364 g) were added to a mixture of the obtained Compound (0.540 g) and 1,2-dichloroethane (5 mL), and the mixture was stirred at 70°C for 10 hours. A 5 mol/L sodium hydroxide aqueous solution (4.0 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 6-bromo-2-(2-chloro-5-fluorobenzyl)-3-methylaniline (0.580 g).

Methyllithium (3.1 mol/L in diethoxymethane) (0.559 mL) was added to a mixture of the obtained Compound (0.580 g) and THF (5 mL) at -78°C, and the mixture was stirred at the same temperature for 1 hour. Methyl iodide (0.268 g) was added to the reaction mixture at -78°C, and the mixture was stirred at the same temperature for 10 minutes and under ice cooling for 1 hour. A saturated ammonium chloride aqueous solution and water were added to the reaction mixture under ice cooling. The mixture was extracted with DCM, and the extract was concentrated under reduced pressure. The residue was purified by Method A (elution solvent: n-hexane/ethyl acetate=98/2 to 85/15) to obtain a title compound (0.410 g).

### Reference Example D-18

Reference Example D-18 was synthesized in the same method as in Reference Example D-16 except that Reference Example D-17 was used in place of Reference Example D-15.

### Reference Example D-19

### 6-bromo-2-((2-chlorophenyl)methyl)-3-fluoro-N-methylaniline

Isopropylmagnesium chloride (2 mol/L in THF) (1.21 mL) was added to a mixture of 1-chloro-2-iodobenzene (0.577 g) and THF (3 mL) at -40°C, and the mixture was stirred under ice cooling for 30 minutes. A mixture of 3-bromo-6-fluoro-2-nitrobenzaldehyde (0.300 g) and THF (3 mL) was added to the reaction mixture at -40°C, and the mixture was stirred at the same temperature for 10 minutes and under ice cooling for 1 hour. A saturated ammonium chloride aqueous solution and water were added to the reaction mixture under ice cooling. The mixture was extracted with ethyl acetate, and the extract was washed with a saturated saline solution. The extract was dried with anhydrous magnesium sulfate and then concentrated under reduced pressure.

Ammonium chloride (1.62 g) and iron powder (0.338 g) were added to a mixture of the residue, ethanol (3 mL) and water (1 mL), and the mixture was stirred at 80°C for 1.5 hours. The reaction mixture was cooled to room temperature and then filtered through Celite. Water was added to the filtrate, the mixture was then extracted with ethyl acetate, and the extract was washed with water and a saturated saline solution. The extract was dried with anhydrous magnesium sulfate and then concentrated under reduced pressure.

TFA (1.38 g) and triethylsilane (0.281 g) were added to a mixture of the residue and DCM (4 mL) under ice cooling, and the mixture was stirred at room temperature for 15 minutes. The reaction mixture was refluxed overnight. A 5 mol/L sodium hydroxide aqueous solution (2.42 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=100/0 to 92/8) to obtain 6-bromo-2-(2-chlorobenzyl)-3-fluoroaniline (0.192 g).

Methyllithium (3.1 mol/L in diethoxymethane) (0.217 mL) was added to a mixture of the obtained Compound (0.192 g) and THF (2 mL) at -78°C, and the mixture was stirred at the same temperature for 30 minutes. Methyl iodide (0.104 g) was added to the reaction mixture at -78°C and the mixture was stirred at the same temperature for 10 minutes and under ice cooling for 1 hour. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=100/0 to 92/8) to obtain a title compound (0.178 g).

### Reference Example D-20

Reference Example D-20 was synthesized in the same method as in Reference Example D-10 except that Reference Example D-19 was used in place of Reference Example D-9.

### Reference Example D-21

Reference Example D-21 was synthesized in the same method as in Reference Example D-19 except that 1-iodo-2-(trifluoromethyl)benzene was used in place of 1-chloro-2-iodobenzene.

### Reference Example D-22

Reference Example D-22 was synthesized in the same method as in Reference Example D-10 except that Reference Example D-21 was used in place of Reference Example D-9.

### Reference Example D-23

Reference Example D-23 was synthesized in the same method as in Reference Example D-19 except that Reference Example E-1 was used in place of 3-bromo-6-fluoro-2-nitrobenzaldehyde.

### Reference Example D-24

Reference Example D-24 was synthesized in the same method as in Reference Example D-10 except that Reference Example D-23 was used in place of Reference Example D-9.

### Reference Example D-25

### 6-bromo-3-fluoro-N-methyl-2-((2-methylphenyl)methyl)aniline

Isopropylmagnesium chloride (2 mol/L in THF) (1.21 mL) was added to a mixture of 1-iodo-2-methylbenzene (0.527 g) and THF (3 mL) at -40°C, and the mixture was stirred under ice cooling for 30 minutes. A mixture of 3-bromo-6-fluoro-2-nitrobenzaldehyde (0.300 g) and THF (3 mL) was added to the reaction mixture at -40°C, and the mixture was stirred at the same temperature for 10 minutes and under ice cooling for 1 hour. A saturated ammonium chloride aqueous solution and water were added to the reaction mixture under ice cooling. The mixture was extracted with ethyl acetate, and the extract was washed with a saturated saline solution. The extract was dried with anhydrous magnesium sulfate and then concentrated under reduced pressure.

Ammonium chloride (1.62 g) and iron powder (0.337 g) were added to a mixture of the residue, ethanol (3 mL) and water (1 mL), and the mixture was stirred at 80°C for 1.5 hours. The reaction mixture was cooled to room temperature and then filtered through Celite. Water was added to the filtrate, the mixture was then extracted with ethyl acetate, and the extract was washed with water and a saturated saline solution. The extract was dried with anhydrous magnesium sulfate and then concentrated under reduced pressure.

TFA (0.669 g) and triethylsilane (0.136 g) were added to a mixture of the residue and DCM (2 mL) under ice cooling, and the mixture was stirred at room temperature for 3 hours. A 5 mol/L sodium hydroxide aqueous solution (1.18 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=100/0 to 90/10) to obtain 6-bromo-3-fluoro-2-(2-methylbenzyl)aniline (0.149 g).

Methyllithium (3.1 mol/L in diethoxymethane) (0.180 mL) was added to a mixture of the obtained Compound (0.149 g) and THF (2 mL) at -78°C, and the mixture was stirred at the same temperature for 30 minutes. Methyl iodide (0.086 g) was added to the reaction mixture at -78°C and the mixture was then stirred at the same temperature for 10 minutes and under ice cooling for 1 hour. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=100/0 to 90/10) to obtain a title compound (0.138 g).

### Reference Example D-26

Reference Example D-26 was synthesized in the same method as in Reference Example D-10 except that Reference Example D-25 was used in place of Reference Example D-9.

### Reference Example D-27

### 6-bromo-3-chloro-2-((2-fluorophenyl)methyl)-N-methylaniline

Isopropylmagnesium chloride (2 mol/L in THF) (1.50 mL) was added to a mixture of 1-fluoro-2-iodobenzene (0.666 g) and THF (3.7 mL) at -40°C, and the mixture was stirred under ice cooling for 50 minutes. A mixture of Reference Example E-1 (0.397 g) and THF (3.7 mL) was added to the reaction mixture at -40°C, and the mixture was stirred at the same temperature for 10 minutes and under ice cooling for 30 minutes. A saturated ammonium chloride aqueous solution and water were added to the reaction mixture under ice cooling. The mixture was extracted with DCM, and the extract was concentrated under reduced pressure.

Ammonium chloride (2.01 g) and iron powder (0.419 g) were added to a mixture of the residue, ethanol (8.2 mL) and water (2.7 mL), and the mixture was stirred at 80°C for 30 minutes. The reaction mixture was cooled to room temperature, and water and DCM were then added. The mixture was extracted with DCM, and the extract was concentrated under reduced pressure. The residue was suspended in n-hexane, and the solid was collected by filtration. The obtained solid was dried under reduced pressure to obtain (2-amino-3-bromo-6-chlorophenyl)(2-fluorophenyl)methanol (0.471 g).

TFA (1.62 g) and triethylsilane (0.331 g) were added to a mixture of the obtained Compound (0.470 g) and DCM (1.4 mL), and the mixture was refluxed for 1.5 hours. A 5 mol/L sodium hydroxide aqueous solution (2.9 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 6-bromo-3-chloro-2-(2-fluorobenzyl)aniline (0.298 g).

Methyllithium (1.04 mol/L in diethyl ether) (0.999 mL) was added to a mixture of the obtained Compound (0.297 g) and THF (2.9 mL) at -78°C, and the mixture was stirred at the same temperature for 45 minutes. Methyl iodide (0.161 g) was added to the reaction mixture at -78°C, and the mixture was then stirred at the same temperature for 10 minutes and under ice cooling for 45 minutes. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=98/2 to 85/15) to obtain a title compound (0.297 g).

### Reference Example D-28

Reference Example D-28 was synthesized in the same method as in Reference Example D-4 except that Reference Example D-27 was used in place of Reference Example D-3.

### Reference Example D-29

### 6-bromo-3-chloro-2-(3-fluorobenzyl)-N-methylaniline

Isopropylmagnesium chloride (2 mol/L in THF) (1.50 mL) was added to a mixture of 1-fluoro-3-iodobenzene (0.666 g) and THF (3.7 mL) at -40°C, and the mixture was stirred under ice cooling for 50 minutes. A mixture of Reference Example E-1 (0.397 g) and THF (3.7 mL) was added to the reaction mixture at -40°C, and the mixture was stirred at the same temperature for 10 minutes and under ice cooling for 30 minutes. A saturated ammonium chloride aqueous solution and water were added to the reaction mixture under ice cooling. The mixture was extracted with DCM, and the extract was concentrated under reduced pressure.

Ammonium chloride (2.01 g) and iron powder (0.419 g) were added to a mixture of the residue, ethanol (8.2 mL) and water (2.7 mL), and the mixture was stirred at 80°C for 30 minutes. The reaction mixture was cooled to room temperature, and water and DCM were then added. The mixture was extracted with DCM, and the extract was concentrated under reduced pressure. The residue was suspended in n-hexane, and the solid was collected by filtration. The obtained solid was dried under reduced pressure to obtain (2-amino-3-bromo-6-chlorophenyl)(3-fluorophenyl)methanol (0.471 g).

TFA (1.62 g) and triethylsilane (0.331 g) were added to a mixture of the obtained Compound (0.470 g) and DCM (1.4 mL), and the mixture was refluxed for 2 hours. A 5 mol/L sodium hydroxide aqueous solution (2.9 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 6-bromo-3-chloro-2-(3-fluorobenzyl)aniline as a mixture with impurities. n-hexane was added to this product, and the mixture was left under ice cooling. Insoluble substances were removed by filtration, and the filtrate was concentrated under reduced pressure to obtain 6-bromo-3-chloro-2-(3-fluorobenzyl)aniline (0.369 g).

Methyllithium (1.04 mol/L in diethyl ether) (1.24 mL) was added to a mixture of the obtained Compound (0.369 g) and THF (3.6 mL) at -78°C, and the mixture was stirred at the same temperature for 45 minutes. Methyl iodide (0.200 g) was added to the reaction mixture at -78°C and the mixture was then stirred at the same temperature for 10 minutes and under ice cooling for 45 minutes. A saturated ammonium chloride aqueous solution and water were added to the reaction mixture under ice cooling. The mixture was extracted with DCM, and the extract was concentrated under reduced pressure. The residue was purified by silica gel column chromatography (elution solvent: n-hexane/ethyl acetate=98/2 to 85/15) to obtain a title compound (0.272 g).

### Reference Example D-30

Reference Example D-30 was synthesized in the same method as in Reference Example D-4 except that Reference Example D-29 was used in place of Reference Example D-3.

### Reference Example D-31

Reference Example D-31 was synthesized in the same method as in Reference Example D-27 except that Reference Example E-2 was used in place of Reference Example E-1.

### Reference Example D-32

Reference Example D-32 was synthesized in the same method as in Reference Example D-4 except that Reference Example D-31 was used in place of Reference Example D-3.

### Reference Example D-33

Reference Example D-33 was synthesized in the same method as in Reference Example D-27 except that 1-fluoro-3-iodobenzene was used in place of 1-fluoro-2-iodobenzene, and Reference Example E-2 was used in place of Reference Example E-1.

### Reference Example D-34

Reference Example D-34 was synthesized in the same method as in Reference Example D-4 except that Reference Example D-33 was used in place of Reference Example D-3.

### Reference Example D-35

### 2-bromo-6-(5-fluoro-2-methylbenzyl)-N-methylaniline

Isopropylmagnesium chloride (2 mol/L in THF) (1.44 mL) was added to a mixture of 4-fluoro-2-iodo-1-methylbenzene (0.677 g) and THF (3 mL) at -40°C, and the mixture was stirred under ice cooling for 30 minutes. A mixture of 3-bromo-2-nitrobenzaldehyde (0.330 g) and THF (3 mL) was added to the reaction mixture at -40°C, and the mixture was stirred at the same temperature for 10 minutes and under ice cooling for 1.5 hours. A saturated ammonium chloride aqueous solution and water were added to the reaction mixture under ice cooling. The mixture was extracted with DCM, and the extract was concentrated under reduced pressure.

Ammonium chloride (1.92 g) and iron powder (0.401 g) were added to a mixture of the residue, ethanol (6 mL) and water (2 mL), and the mixture was stirred at 80°C for 1.5 hours. The reaction mixture was cooled to room temperature, and water and DCM were then added. The mixture was extracted with DCM, and the extract was concentrated under reduced pressure. The residue was purified by silica gel column chromatography (elution solvent: n-hexane/ethyl acetate=98/2 to 80/20) to obtain (2-amino-3-bromophenyl)(5-fluoro-2-methylphenyl)methanol (0.550 g).

TFA (1.64 g) and triethylsilane (0.334 g) were added to a mixture of the obtained Compound (0.550 g) and DCM (5 mL), and the mixture was stirred at room temperature for 13 hours. A 5 mol/L sodium hydroxide aqueous solution (3.0 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(5-fluoro-2-methylbenzyl)aniline (0.480 g).

Methyllithium (3.1 mol/L in diethoxymethane) (0.510 mL) was added to a mixture of the obtained Compound (0.480 g) and THF (5 mL) at -78°C, and the mixture was stirred at the same temperature for 30 minutes. Methyl iodide (0.245 g) was added to the reaction mixture at -78°C, and the mixture was stirred at the same temperature for 10 minutes and under ice cooling for 1 hour. A saturated ammonium chloride aqueous solution was added to the reaction mixture under ice cooling. The mixture was extracted with DCM, and the extract was concentrated under reduced pressure. The residue was purified by Method A (elution solvent: n-hexane/ethyl acetate=98/2 to 85/15) to obtain a title compound (0.520 g).

### Reference Example D-36

Reference Example D-36 was synthesized in the same method as in Reference Example D-16 except that Reference Example D-35 was used in place of Reference Example D-15.

### Reference Example E-1

### 3-bromo-6-chloro-2-nitrobenzaldehyde

A mixture of 70% nitric acid (4.92 g) and concentrated sulfuric acid (51.1 g) was slowly added to a mixture of 5-bromo-2-chlorobenzaldehyde (6.00 g) and concentrated sulfuric acid (41.6 g) under ice cooling. The reaction mixture was stirred at room temperature for 15 hours. The reaction mixture was slowly added to ice water. The mixture was extracted with ethyl acetate, and the extract was washed with a saturated saline solution. The extract was dried with anhydrous sodium sulfate and then concentrated under reduced pressure. The residue was dissolved in ethyl acetate (15 mL), n-hexane (30 mL) was then added, and the mixture was stirred at room temperature for 30 minutes and under ice cooling for 30 minutes. Insoluble substances were collected by filtration, and the obtained solid was dried under reduced pressure to obtain a title compound (3.10 g).

### Reference Example E-2

Reference Example E-2 was synthesized in the same method as in Reference Example E-1 except that 5-bromo-2-methylbenzaldehyde was used in place of 5-bromo-2-chlorobenzaldehyde.

### Reference Example F-1

Reference Example F-1 was synthesized in the same method as in Reference Example B-73 except that 1-bromo-4-chloro-3-fluoro-2-nitrobenzene was used in place of 1-bromo-3-fluoro-2-nitrobenzene, and 2,5-difluorophenol was used in place of 2-methylphenol.

### Reference Example F-2

### (3R)-3-amino-7-chloro-8-(2,5-difluorophenoxy)-1-methyl-1,2,3,4-tetrahydroquinolin-2-one

Under an argon atmosphere, a mixture of methyl (S)-2-((tert-butoxycarbonyl)amino)-3-iodopropanate (0.306 g) and DMF (0.4 mL) was added to a mixture of zinc (0.089 g) and DMF (0.8 mL), and the mixture was stirred at room temperature for 1 hour. A mixture of Reference Example F-1 (0.216 g) and DMF (0.8 mL), palladium(II) acetate (0.007 g) and Xphos (0.030 g) were added to the reaction mixture, and the mixture was stirred at 40°C for 3 hours. A saturated ammonium chloride aqueous solution, water, ethyl acetate and n-hexane were added to the reaction mixture under ice cooling. The mixture was filtered through Celite, and the filtrate was extracted with ethyl acetate/n-hexane (1/1). The extract was washed with water and a saturated saline solution and dried with anhydrous magnesium sulfate and then concentrated under reduced pressure.

Methanesulfonic acid (0.179 g) was added to a mixture of the residue, toluene (1.0 mL) and ethanol (0.7 mL), and the mixture was stirred at 80°C for 0.5 hours. A 5 mol/L sodium hydroxide aqueous solution (0.375 mL) was added to the reaction mixture under ice cooling. The mixture was extracted with DCM, and the extract was concentrated under reduced pressure. The residue was purified by Method A (elution solvent: ethyl acetate/methanol=98/2 to 80/20) to obtain a title compound (0.084 g).

### Reference Example F-3

Reference Example F-3 was obtained in the same method as in Reference Example B-17 except that 1-bromo-3-fluoro-4-methyl-2-nitrobenzene was used in place of 1-bromo-3-fluoro-2-nitrobenzene and 4-fluoro-2-hydroxybenzaldehyde was used in place of 2-hydroxybenzaldehyde.

### Reference Example F-4

Reference Example F-4 was obtained in the same method as in Reference Example F-2 except that Reference Example F-3 was used in place of Reference Example F-1.

### Reference Example F-5

### (4-bromo-2-(2-chloro-5-fluorophenoxy)-3-(methylamino)phenyl)methanol

Sodium hydride (about 60%) (0.761 g) was added to a mixture of 4-bromo-2-fluorobenzyl alcohol (3.00 g) and DMF (30 mL) under ice cooling, and the mixture was stirred at room temperature for 10 minutes. 4-methoxybenzylchloride (2.75 g) was added to the reaction mixture under ice cooling, and the mixture was stirred at room temperature for 1 hour. A saturated ammonium chloride aqueous solution, water, ethyl acetate and n-hexane were added to the reaction mixture under ice cooling, and the mixture was stirred at room temperature for 10 minutes. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain 4-bromo-2-fluoro-1-(((4-methoxyphenyl)methoxy)methyl)benzene (4.96 g).

LDA (1.07 mol/L in n-hexane/THF) (22 mL) was added to a mixture of the obtained Compound (4.76 g) and THF (50 mL) at -78°C, and the mixture was stirred at the same temperature for 1 hour. DMF (3.21 g) was added to the reaction mixture at -78°C, and the mixture was stirred at the same temperature for 0.5 hours. 2 mol/L hydrochloric acid (40 mL) was added to the reaction mixture at -20°C, and the mixture was then stirred at room temperature for 10 minutes. The mixture was extracted with n-hexane, and the extract was washed with water and a saturated saline solution. The extract was dried with anhydrous sodium sulfate and then concentrated under reduced pressure. Potassium carbonate (4.05 g) was added to a mixture of the residue, NMP (40 mL) and 2-chloro-5-fluorophenol (2.25 g), and the mixture was stirred at 110°C for 3 hours. The reaction mixture was cooled to room temperature, water, ethyl acetate and n-hexane were then added, and the mixture was stirred at room temperature for 10 minutes. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=95/5 to 80/20) to obtain 6-bromo-2-(2-chloro-5-fluorophenoxy)-3-(((4-methoxyphenyl)methoxy)methyl)benzaldehyde (5.00 g).

Concentrated sulfuric acid (0.613 g) was added to a mixture of the obtained Compound (5.00 g), MeCN (22.5 mL) and DMSO (0.977 g) under a salt ice bath. A mixture of sodium chlorite (80%) (1.41 g) and water (7.5 mL) was added to the mixture under a salt ice bath, and the mixture was stirred at the same temperature for 0.5 hours. A saturated sodium sulfite aqueous solution (1.5 mL) was added to the reaction mixture under a salt ice bath, and the mixture was stirred at room temperature for 10 minutes. Water was added to the mixture, and the mixture was extracted with n-hexane/ethyl acetate (1/1). The extract was washed with water and a saturated saline solution. The extract was dried with anhydrous sodium sulfate and then concentrated under reduced pressure. TEA (2.11 g) and DPPA (4.31 g) were added to a mixture of the residue and DMF (25 mL), and the mixture was stirred at room temperature for 2 hours. A 5 mol/L sodium hydroxide aqueous solution (8.3 mL) was added to the mixture under water cooling, and the mixture was stirred at room temperature for 0.5 hours. The mixture was extracted with n-hexane/ethyl acetate (1/1). The extract was washed with a saturated ammonium chloride aqueous solution, water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain 6-bromo-2-(2-chloro-5-fluorophenoxy)-3-(((4-methoxyphenyl)methoxy)methyl)aniline (2.80 g).

Pyridine (0.617 g) and trifluoroacetic anhydride (1.64 g) were added to a mixture of the obtained Compound (2.80 g) and ethyl acetate (28 mL) under a salt ice bath, and the mixture was stirred at the same temperature for 0.5 hours. Methanol (0.192 g) and water were added to the reaction mixture, and the mixture was stirred at room temperature for 10 minutes. The mixture was extracted with ethyl acetate, and the extract was washed with water and a saturated saline solution. The extract was dried with anhydrous sodium sulfate and then concentrated under reduced pressure. Methyl iodide (1.28 g) and potassium carbonate (1.66 g) were added to a mixture of the residue and DMF (14 mL), and the mixture was stirred at room temperature for 3 hours. Water, ethyl acetate and n-hexane were added to the reaction mixture. The mixture was extracted with ethyl acetate, and the extract was washed with water and then concentrated under reduced pressure. A 5 mol/L sodium hydroxide aqueous solution (2.4 mL) was added to a mixture of the residue, methanol (14 mL) and THF (14 mL), and the mixture was stirred at 60°C for 2 hours. The reaction mixture was cooled to room temperature, and water and ethyl acetate were then added to the reaction mixture. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=98/2 to 85/15) to obtain 6-bromo-2-(2-chloro-5-fluorophenoxy)-3-(((4-methoxyphenyl)methoxy)methyl)-N-methylaniline (2.50 g).

2,3-dichloro-5,6-dicyano-p-benzoquinone (0.779 g) was added to a mixture of the obtained Compound (1.50 g), DCM (15 mL) and water (0.15 mL) under ice cooling, and the mixture was stirred at the same temperature for 2 hours. A saturated sodium bicarbonate aqueous solution and water were added to the reaction mixture under ice cooling, and the mixture was stirred at room temperature for 10 minutes. The mixture was extracted with DCM, and the extract was concentrated under reduced pressure. The residue was purified by Method A (elution solvent: n-hexane/ethyl acetate=90/10 to 60/40) to obtain a title compound (0.920 g).

### Reference Example F-6

Reference Example F-6 was obtained in the same method as in Reference Example F-2 except that Reference Example F-5 was used in place of Reference Example F-1.

### Reference Example F-7

### 6-bromo-2-(2-chloro-5-fluorophenoxy)-3-(difluoromethyl)-N-methylaniline

LDA (1.07 mol/L in n-hexane/THF) (5.5 mL) was added to a mixture of 4-bromo-1-(difluoromethyl)-2-fluorobenzene (0.800 g) and THF (8 mL) at -78°C, and the mixture was stirred at the same temperature for 1 hour. DMF (0.780 g) was added to the reaction mixture at -78°C, and the mixture was stirred at the same temperature for 0.5 hours. A saturated ammonium chloride aqueous solution was added to the reaction mixture at -78°C, and the mixture was stirred at room temperature for 10 minutes. The mixture was extracted with n-hexane, and the extract was washed with water and a saturated saline solution. The extract was dried with anhydrous sodium sulfate and then concentrated under reduced pressure. Potassium carbonate (0.721 g) was added to a mixture of the residue, NMP (5 mL) and 2-chloro-5-fluorophenol (0.535 g), and the mixture was stirred at 110°C for 2 hours. The reaction mixture was cooled to room temperature, water, ethyl acetate and n-hexane were then added, and the mixture was stirred at room temperature for 10 minutes. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain 6-bromo-2-(2-chloro-5-fluorophenoxy)-3-(difluoromethyl)benzaldehyde (0.790 g).

Concentrated sulfuric acid (0.122 g) was added to a mixture of the obtained Compound (0.790 g), MeCN (3.6 mL) and DMSO (0.195 g) under a salt ice bath. A mixture of sodium chlorite (80%) (0.282 g) and water (1.2 mL) was added to the mixture under a salt ice bath, and the mixture was stirred at the same temperature for 0.5 hours. A saturated sodium sulfite aqueous solution (0.24 mL) was added to the reaction mixture under a salt ice bath, and the mixture was stirred at room temperature for 10 minutes. Water was added to the mixture, and the mixture was extracted with n-hexane/ethyl acetate (1/1). The extract was washed with water and a saturated saline solution. The extract was dried with anhydrous sodium sulfate and then concentrated under reduced pressure. TEA (0.420 g) and DPPA (0.856 g) were added to a mixture of the residue and DMF (4 mL), and the mixture was stirred at room temperature for 2 hours. A 5 mol/L sodium hydroxide aqueous solution (1.7 mL) was added to the mixture under water cooling, and the mixture was stirred at room temperature for 1 hour. The mixture was extracted with n-hexane/ethyl acetate (1/1), and the extract was washed with a saturated ammonium chloride aqueous solution, water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain 6-bromo-2-(2-chloro-5-fluorophenoxy)-3-(difluoromethyl)aniline (0.330 g).

Pyridine (0.093 g) and trifluoroacetic anhydride (0.246 g) were added to a mixture of the obtained Compound (0.330 g) and ethyl acetate (3 mL) under ice cooling, and the mixture was stirred at the same temperature for 1 hour. Methanol (0.029 g) and water were added to the reaction mixture, and the mixture was stirred at room temperature for 10 minutes. The mixture was extracted with ethyl acetate, and the extract was washed with water and a saturated saline solution. The extract was dried with anhydrous sodium sulfate and then concentrated under reduced pressure. Methyl iodide (0.192 g) and potassium carbonate (0.249 g) were added to a mixture of the residue and DMF (1.5 mL), and the mixture was stirred at room temperature for 1 hour. Water, ethyl acetate and n-hexane were added to the reaction mixture. The mixture was extracted with ethyl acetate, and the extract was washed with water and then concentrated under reduced pressure. A 5 mol/L sodium hydroxide aqueous solution (0.36 mL) was added to a mixture of the residue, methanol (1.5 mL) and THF (1.5 mL), and the mixture was stirred at 60°C for 2 hours. The reaction mixture was cooled to room temperature, and water and ethyl acetate were then added to the reaction mixture. The mixture was extracted with ethyl acetate, and the extract was washed with water and a saturated saline solution. The extract was dried with anhydrous sodium sulfate and then concentrated under reduced pressure. The residue was purified by silica gel column chromatography (elution solvent: n-hexane/ethyl acetate=98/2 to 80/20) to obtain a title compound (0.300 g).

### Reference Example F-8

### (3R)-3-amino-8-(2-chloro-5-fluorophenoxy)-7-(difluoromethyl)-1-methyl-1,2,3,4-tetrahydroquinolin-2-one

Under an argon atmosphere, methyl (S)-2-((tert-butoxycarbonyl)amino)-3-iodopropanate (0.389 g) was added to a mixture of zinc (0.113 g) and DMF (1 mL), and the mixture was stirred at room temperature for 1 hour. A mixture of Reference Example F-7 (0.300 g) and DMF (1 mL), palladium(II) acetate (0.009 g) and Xphos (0.038 g) were added to the reaction mixture, and the mixture was stirred at 40°C for 2 hours. The reaction mixture was cooled to room temperature, and a saturated ammonium chloride aqueous solution, water, ethyl acetate and n-hexane were then added to the reaction mixture. The mixture was filtered through Celite, and the filtrate was extracted with ethyl acetate. The extract was washed with water and a saturated saline solution. The extract was dried with anhydrous sodium sulfate and then concentrated under reduced pressure.

Methanesulfonic acid (0.227 g) was added to a mixture of the residue, toluene (1 mL) and ethanol (0.5 mL), and the mixture was stirred at 70°C for 2 hours. The reaction mixture was cooled to room temperature and then concentrated under reduced pressure. Water, toluene and ethyl acetate were added to the residue and dispensed. The aqueous layer was separated, ethyl acetate was added, and under stirring, a saturated sodium bicarbonate aqueous solution was then added. The organic layer was separated and washed with a saturated saline solution. The organic layer was dried with anhydrous sodium sulfate and then concentrated under reduced pressure to obtain a title compound (0.050 g).

### Reference Example F-9

Reference Example F-9 was obtained in the same method as in Reference Example F-7 except that 4-bromo-2-fluoro-1-(fluoromethyl)benzene was used in place of 4-bromo-1-(difluoromethyl)-2-fluorobenzene.

### Reference Example F-10

Reference Example F-10 was obtained in the same method as in Reference Example F-2 except that Reference Example F-9 was used in place of Reference Example F-1.

### Reference Example F-11

Reference Example F-11 was obtained in the same method as in Reference Example F-7 except that 4-bromo-2-fluoro-1-(trifluoromethyl)benzene was used in place of 4-bromo-1-(difluoromethyl)-2-fluorobenzene.

### Reference Example F-12

### (3R)-3-amino-8-(2-chloro-5-fluorophenoxy)-1-methyl-7-(trifluoromethyl)-1,2,3,4-tetrahydroquinolin-2-one

Under an argon atmosphere, methyl (S)-2-((tert-butoxycarbonyl)amino)-3-iodopropanate (0.997 g) was added to a mixture of zinc (0.256 g) and DMF (3 mL), and the mixture was stirred at room temperature for 1 hour. A mixture of Reference Example F-11 (0.710 g) and DMF (1.5 mL), palladium(II) acetate (0.020 g) and Xphos (0.085 g) were added to the reaction mixture, and the mixture was stirred at 40°C for 2 hours. The reaction mixture was cooled to room temperature, and a saturated ammonium chloride aqueous solution, water, ethyl acetate and n-hexane were then added. The mixture was filtered through Celite, and the filtrate was extracted with ethyl acetate. The extract was washed with water and a saturated saline solution, dried with anhydrous sodium sulfate, and then concentrated under reduced pressure.

Methanesulfonic acid (0.514 g) was added to a mixture of the residue, toluene (3.5 mL) and ethanol (2 mL), and the mixture was stirred at 70°C for 2 hours. The reaction mixture was cooled to room temperature, and a saturated sodium bicarbonate aqueous solution was then added. The mixture was extracted with ethyl acetate, and the extract was washed with a saturated saline solution. The extract was dried with anhydrous sodium sulfate and then concentrated under reduced pressure. The residue was purified by silica gel column chromatography (elution solvent: n-hexane/ethyl acetate/methanol=49/49/2 to 0/98/2 to 0/86/14) to obtain a title compound (0.400 g).

### Reference Example F-13

Reference Example F-13 was obtained in the same method as in Reference Example F-7 except that 4-bromo-1-(1,1-difluoroethyl)-2-fluorobenzene was used in place of 4-bromo-1-(difluoromethyl)-2-fluorobenzene.

### Reference Example F-14

Reference Example F-14 was obtained in the same method as in Reference Example F-12 except that Reference Example F-13 was used in place of Reference Example F-11.

### Reference Example F-15

Reference Example F-15 was obtained in the same method as in Reference Example F-7 except that 4-bromo-1-ethyl-2-fluorobenzene was used in place of 4-bromo-1-(difluoromethyl)-2-fluorobenzene.

### Reference Example F-16

Reference Example F-16 was obtained in the same method as in Reference Example F-12 except that Reference Example F-15 was used in place of Reference Example F-11.

### Reference Example G-1

### 2-bromo-6-(1-(2-chlorophenyl)ethenyl)-N-methylaniline

Isopropylmagnesium chloride (2.0 mol/L in THF) (1.5 mL) was added to a mixture of 1-chloro-2-iodobenzene (0.715 g) and THF (3.7 mL) at -40°C, and the mixture was stirred at 0°C for 45 minutes. A mixture of 3-bromo-2-nitrobenzaldehyde (0.345 g) and THF (3.7 mL) was added to the reaction mixture at -40°C and the mixture was stirred at the same temperature for 10 minutes and at 0°C for 30 minutes. A saturated ammonium chloride aqueous solution and water were added to the reaction mixture at 0°C and the mixture was stirred at room temperature for 10 minutes. The mixture was extracted with DCM, and the extract was concentrated under reduced pressure. Iron powder (0.419 g) and ammonium chloride (2.01 g) were added to a mixture of the residue, ethanol (8.2 mL) and water (2.7 mL), the mixture was stirred at 80°C for 2 hours and then left at room temperature for 13 hours. The reaction mixture was stirred at 80°C for 1 hour. The reaction mixture was cooled to room temperature and water and DCM were then added. The mixture was extracted with DCM, and the extract was concentrated under reduced pressure. The residue was suspended in n-hexane, and the solid was collected by filtration. The obtained solid was dried under reduced pressure to obtain (2-amino-3-bromophenyl)(2-chlorophenyl)methanol (0.351 g).

Manganese dioxide (0.348 g) was added to a mixture of the obtained Compound (0.250 g) and DCM (7.3 mL), and the mixture was stirred at room temperature for 2.5 hours and at 35°C for 17 hours. The reaction mixture was cooled to room temperature and then filtered through Celite. The filtrate was concentrated under reduced pressure to obtain 2-bromo-6-(2-chlorobenzoyl)aniline (0.247 g).

Under an argon atmosphere, methylmagnesium bromide (3.0 mol/L in diethyl ether) (0.52 mL) was added to a mixture of the obtained Compound (0.231 g) and diethyl ether (2.5 mL) under ice cooling and the mixture was stirred at the same temperature for 15 minutes and at room temperature for 1 hour. A saturated ammonium chloride aqueous solution and water were added to the reaction mixture under ice cooling and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 1-(2-amino-3-bromophenyl)-1-(2-chlorophenyl)ethan-1-ol (0.247 g).

Boron trifluoride diethyl ether complex (0.127 g) and triethylsilane (0.174 g) were added to a mixture of the obtained Compound (0.195 g) and 1,2-dichloroethane (3 mL) and the mixture was stirred at 70°C for 2 hours and at 50°C for 15 hours. A 5 mol/L sodium hydroxide aqueous solution (0.18 mL) was added to the reaction mixture under ice cooling and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(1-(2-chlorophenyl)ethenyl)aniline (0.171 g).

Methyllithium (1.04 mol/L in diethyl ether) (0.59 mL) was added to a mixture of the obtained Compound (0.171 g) and THF (1.7 mL) at -78°C and the mixture was stirred at the same temperature for 30 minutes. Methyl iodide (0.094 g) was added to the reaction mixture at -78°C and the mixture was stirred at the same temperature for 10 minutes and at 0°C for 1.5 hours. A saturated ammonium chloride aqueous solution and water were added to the reaction mixture at 0°C and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain a title compound (0.168 g).

### Reference Example G-2

Reference Example G-2 was obtained in the same method as in Reference Example F-2 except that Reference Example G-1 was used in place of Reference Example F-1.

### Reference Example G-3

### 2-bromo-N-methyl-6-(1-(2-(trifluoromethyl)phenyl)ethenyl)aniline

Isopropylmagnesium chloride (2.0 mol/L in THF) (1.3 mL) was added to a mixture of 1-iodo-2-(trifluoromethyl)benzene (0.710 g) and THF (3 mL) at -40°C and the mixture was stirred at 0°C for 30 minutes. A mixture of 3-bromo-2-nitrobenzaldehyde (0.300 g) and THF (3 mL) was added to the reaction mixture at -40°C and the mixture was stirred at the same temperature for 10 minutes and at 0°C for 1 hour. A saturated ammonium chloride aqueous solution and water were added to the reaction mixture at 0°C and the mixture was stirred at room temperature for 10 minutes. The mixture was extracted with DCM, and the extract was concentrated under reduced pressure. Iron powder (0.365 g) and ammonium chloride (1.75 g) were added to a mixture of the residue, ethanol (3 mL) and water (1 mL), and the mixture was stirred at 80°C for 1.5 hours. The reaction mixture was cooled to room temperature and then filtered through Celite. Water was added to the filtrate, the mixture was then extracted with ethyl acetate, and the extract was washed with water and a saturated saline solution. The extract was dried with anhydrous magnesium sulfate and then concentrated under reduced pressure. The residue was purified by silica gel column chromatography (elution solvent: n-hexane/ethyl acetate=100/0 to 75/25) to obtain (2-amino-3-bromophenyl)(2-(trifluoromethyl)phenyl)methanol (0.286 g).

Manganese dioxide (0.359 g) was added to a mixture of the obtained Compound (0.286 g) and 1,2-dichloroethane (6 mL), and the mixture was stirred at 50°C for 3 hours. The reaction mixture was cooled to room temperature and then filtered through Celite. The filtrate was concentrated under reduced pressure. The residue was purified by silica gel column chromatography (elution solvent: n-hexane/ethyl acetate=100/0 to 80/20) to obtain 2-bromo-6-(2-(trifluoromethyl)benzoyl)aniline (0.224 g).

Methylmagnesium bromide (3.0 mol/L in THF) (0.65 mL) was added to a mixture of the obtained Compound (0.224 g) and THF (3 mL) under ice cooling, and the mixture was stirred at room temperature for 0.5 hours. Methylmagnesium bromide (3.0 mol/L in THF) (0.65 mL) was added to the reaction mixture at room temperature, and the mixture was stirred at room temperature for 3 hours. Methylmagnesium bromide (3.0 mol/L in THF) (0.65 mL) was added to the reaction mixture at room temperature, and the mixture was stirred at room temperature for 10 hours. A saturated ammonium chloride aqueous solution and water were added to the reaction mixture, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=100/0 to 75/25) to obtain 1-(2-amino-3-bromophenyl)-1-(2-(trifluoromethyl)phenyl)ethan-1-ol (0.187 g).

TFA (0.592 g) and triethylsilane (0.121 g) were added to a mixture of the obtained Compound (0.187 g) and 1,2-dichloroethane (3 mL), and the mixture was stirred at 60°C for 1 hour. A 5 mol/L sodium hydroxide aqueous solution (1.0 mL) was added to the reaction mixture under ice cooling, and the mixture was stirred at room temperature for 10 minutes. The mixture was extracted with DCM, and the extract was concentrated under reduced pressure. The residue was purified by silica gel column chromatography (elution solvent: n-hexane/ethyl acetate=98/2 to 85/15) to obtain 2-bromo-6-(1-(2-(trifluoromethyl)phenyl)ethenyl)aniline (0.138 g).

Methyllithium (3.1 mol/L in diethoxymethane) (0.14 mL) was added to a mixture of the obtained Compound (0.138 g) and THF (3 mL) at -78°C, and the mixture was stirred at the same temperature for 30 minutes. Methyl iodide (0.069 g) was added to the reaction mixture at -78°C, and the mixture was stirred at the same temperature for 10 minutes and at 0°C for 1 hour. A saturated ammonium chloride aqueous solution and water were added to the reaction mixture at 0°C and the mixture was stirred at room temperature for 10 minutes. The mixture was extracted with DCM, and the extract was concentrated under reduced pressure. The residue was purified by silica gel column chromatography (elution solvent: n-hexane/ethyl acetate=100/0 to 90/10) to obtain a title compound (0.102 g).

### Reference Example G-4

Reference Example G-4 was obtained in the same method as in Reference Example D-10 except that Reference Example G-3 was used in place of Reference Example D-9.

### Reference Example G-5

### 2-bromo-6-(1-(2-chloro-5-fluorophenyl)ethenyl)-N-methylaniline

Isopropylmagnesium chloride (2.0 mol/L in THF) (1.4 mL) was added to a mixture of 1-chloro-4-fluoro-2-iodobenzene (0.719 g) and THF (3 mL) at -40°C, and the mixture was stirred at 0°C for 0.5 hours. A mixture of 1-(2-amino-3-bromophenyl)ethan-1-one (0.300 g) and THF (3 mL) was added to the reaction mixture at -40°C, and the mixture was stirred at 0°C for 1 hour and at room temperature for 12 hours. A saturated ammonium chloride aqueous solution and water were added to the reaction mixture, and the mixture was stirred at room temperature for 10 minutes. The mixture was extracted with DCM, and the extract was concentrated under reduced pressure. The residue was purified by APS column chromatography (elution solvent: n-hexane/ethyl acetate=95/5 to 70/30) to obtain 1-(2-amino-3-bromophenyl)-1-(2-chloro-5-fluorophenyl)ethan-1-ol (0.360 g).

TFA (1.19 g) and triethylsilane (0.243 g) were added to a mixture of the obtained Compound (0.360 g) and 1,2-dichloroethane (4 mL), and the mixture was stirred at room temperature for 1 hour and at 70°C for 3 hours. A 5 mol/L sodium hydroxide aqueous solution (2.2 mL) was added to the reaction mixture under ice cooling, and the mixture was then stirred at room temperature for 10 minutes. The mixture was extracted with DCM, and the extract was concentrated under reduced pressure. The residue was purified by Method A (elution solvent: n-hexane/ethyl acetate=98/2 to 85/15) to obtain 2-bromo-6-(1-(2-chloro-5-fluorophenyl)ethenyl)aniline (0.320 g).

Methyllithium (3.1 mol/L in diethoxymethane) (0.35 mL) was added to a mixture of the obtained Compound (0.320 g) and THF (5 mL) at -78°C, and the mixture was stirred at the same temperature for 0.5 hours. Methyl iodide (0.167 g) was added to the mixture at - 78°C, and the mixture was stirred at the same temperature for 10 minutes and at 0°C for 1 hour. A saturated ammonium chloride aqueous solution and water were added to the reaction mixture at 0°C, and the mixture was then stirred at room temperature for 10 minutes. The mixture was extracted with DCM, and the extract was concentrated under reduced pressure. The residue was purified by APS column chromatography (elution solvent: n-hexane/ethyl acetate=98/2 to 85/15) to obtain a title compound (0.244 g).

### Reference Example G-6

Reference Example G-6 was obtained in the same method as in Reference Example D-16 except that Reference Example G-5 was used in place of Reference Example D-15.

The following tables show the structural formulae of reference examples.

**[Table 1]**

| Ref. No. | Str. | Ref. No. | Str. |
|---|---|---|---|
| A-1 | 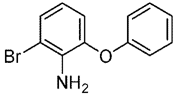 | A-2 | 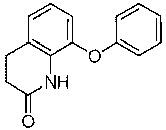 |
| A-3 | 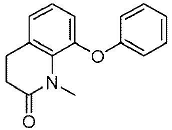 | A-4 | 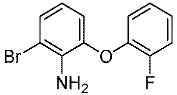 |
| A-5 | 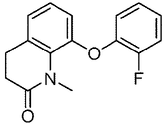 | A-6 | 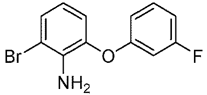 |
| A-7 | 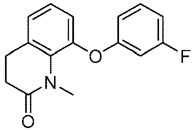 | A-8 | 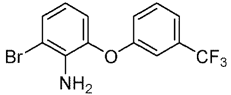 |
| A-9 | 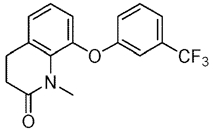 | A-10 | 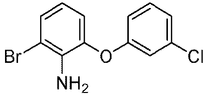 |
| A-11 | 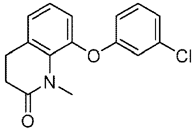 | A-12 | 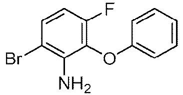 |
| A-13 | 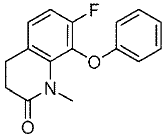 | A-14 | 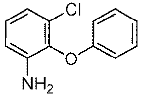 |

**[Table 2]**

| Ref. No. | Str. | Ref. No. | Str. |
|---|---|---|---|
| A-15 | 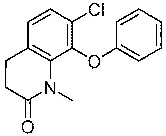 | A-16 | 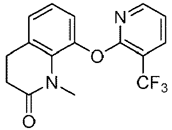 |
| B-1 | 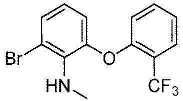 | B-2 | 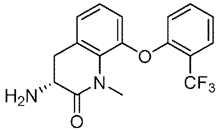 |
| B-3 | 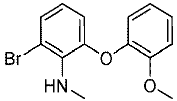 | B-4 | 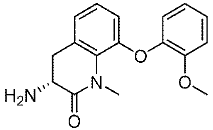 |
| B-5 | 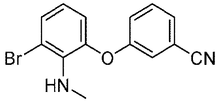 | B-6 | 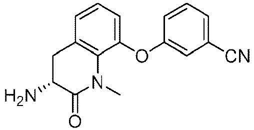 |
| B-7 | 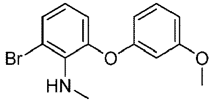 | B-8 | 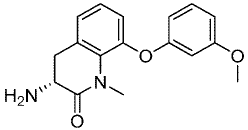 |
| B-9 | 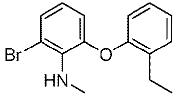 | B-10 | 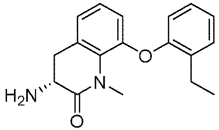 |
| B-11 | 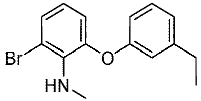 | B-12 | 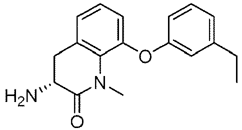 |
| B-13 | 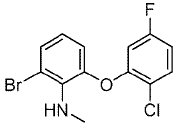 | B-14 | 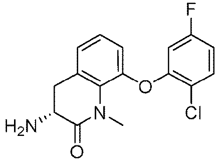 |

**[Table 3]**

| Ref. No. | Str. | Ref. No. | Str. |
|---|---|---|---|
| B-15 | 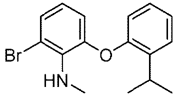 | B-16 | 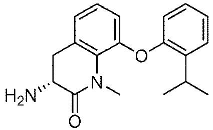 |
| B-17 | 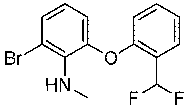 | B-18 | 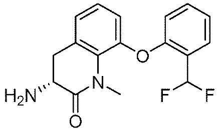 |
| B-19 | 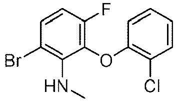 | B-20 | 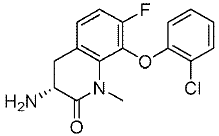 |
| B-21 | 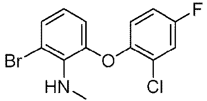 | B-22 | 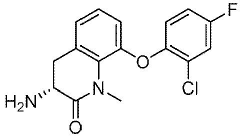 |
| B-23 | 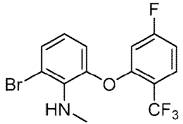 | B-24 | 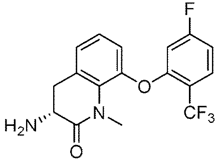 |
| B-25 | 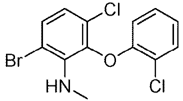 | B-26 | 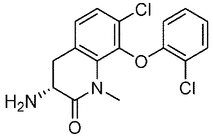 |
| B-27 | 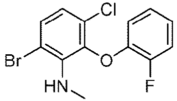 | B-28 | 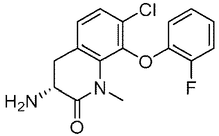 |

**[Table 4]**

| Ref. No. | Str. | Ref. No. | Str. |
|---|---|---|---|
| B-29 | 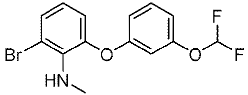 | B-30 | 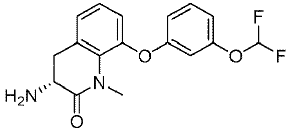 |
| B-31 | 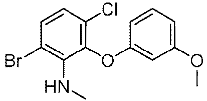 | B-32 | 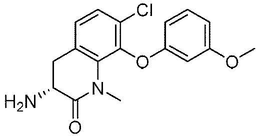 |
| B-33 | 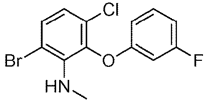 | B-34 | 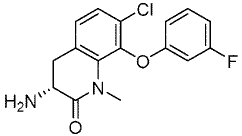 |
| B-35 | 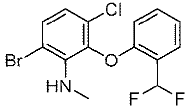 | B-36 | 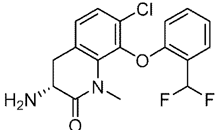 |
| B-37 | 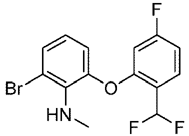 | B-38 | 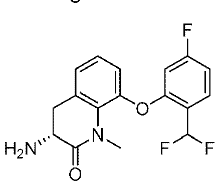 |
| B-39 | 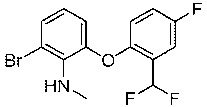 | B-40 | 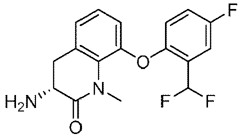 |
| B-41 | 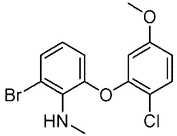 | B-42 | 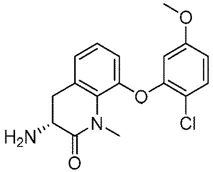 |

**[Table 5]**

| Ref. No. | Str. | Ref. No. | Str. |
|---|---|---|---|
| B-43 | 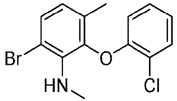 | B-44 | 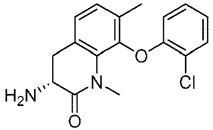 |
| B-45 | 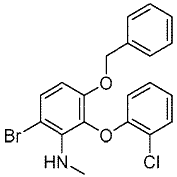 | B-46 | 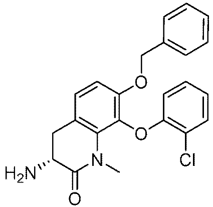 |
| B-47 | 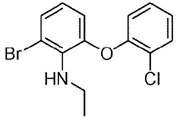 | B-48 | 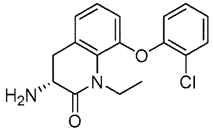 |
| B-49 | 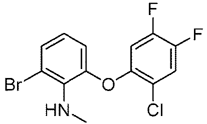 | B-50 | 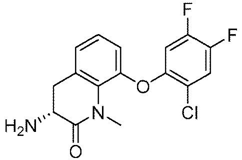 |
| B-51 | 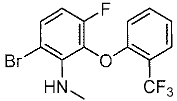 | B-52 | 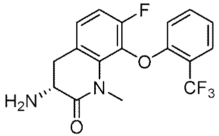 |
| B-53 | 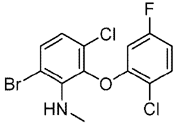 | B-54 | 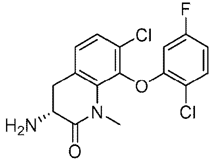 |

**[Table 6]**

| Ref. No. | Str. | Ref. No. | Str. |
|---|---|---|---|
| B-55 | 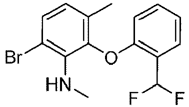 | B-56 | 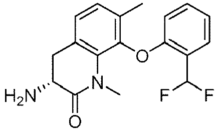 |
| B-57 | 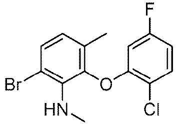 | B-58 | 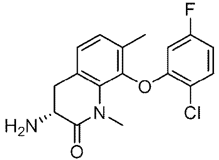 |
| B-59 | 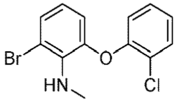 | B-60 | 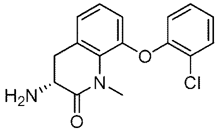 |
| B-61 | 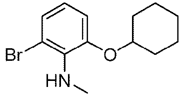 | B-62 | 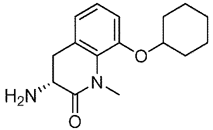 |
| B-63 | 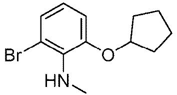 | B-64 | 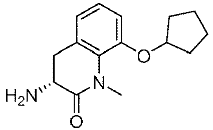 |
| B-65 | 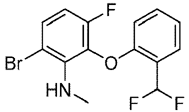 | B-66 | 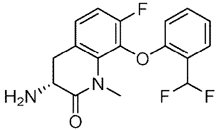 |
| B-67 | 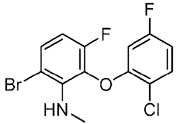 | B-68 | 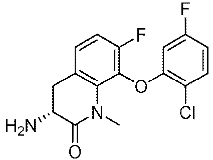 |

**[Table 7]**

| Ref. No. | Str. | Ref. No. | Str. |
|---|---|---|---|
| B-69 | 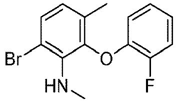 | B-70 | 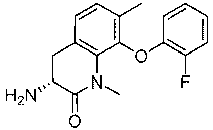 |
| B-71 | 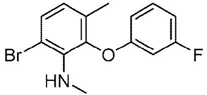 | B-72 | 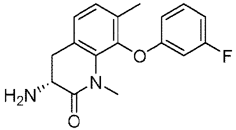 |
| B-73 | 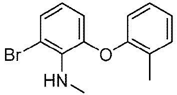 | B-74 | 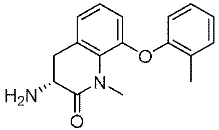 |
| B-75 | 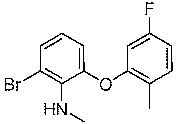 | B-76 | 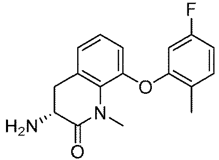 |
| B-77 | 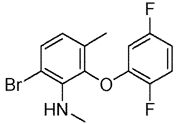 | B-78 | 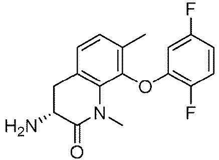 |
| C-1 | 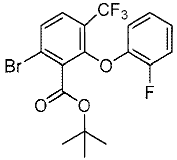 | C-2 | 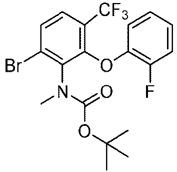 |
| C-3 | 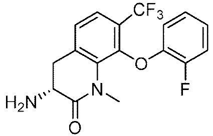 | D-1 | 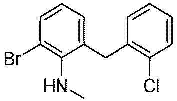 |

**[Table 8]**

| Ref. No. | Str. | Ref. No. | Str. |
|---|---|---|---|
| D-2 | 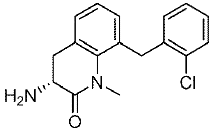 | D-3 | 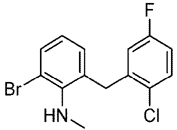 |
| D-4 | 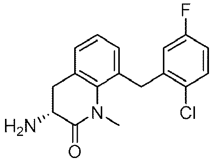 | D-5 | 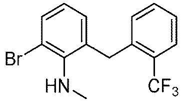 |
| D-6 | 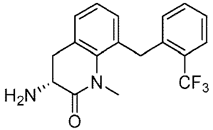 | D-7 | 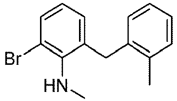 |
| D-8 | 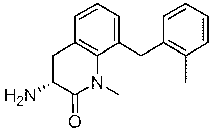 | D-9 | 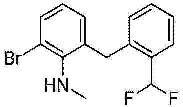 |
| D-10 | 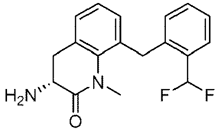 | D-11 | 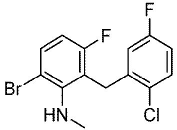 |
| D-12 | 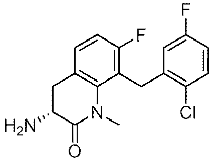 | D-13 | 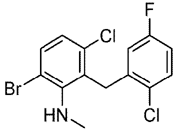 |
| D-14 | 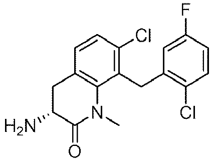 | D-15 | 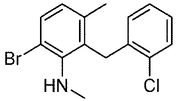 |

**[Table 9]**

| Ref. No. | Str. | Ref. No. | Str. |
|---|---|---|---|
| D-16 | 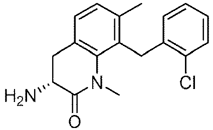 | D-17 | 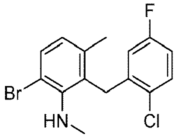 |
| D-18 | 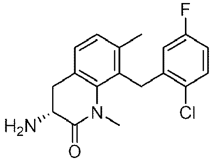 | D-19 | 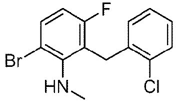 |
| D-20 | 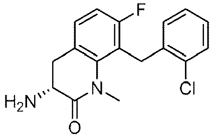 | D-21 | 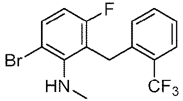 |
| D-22 | 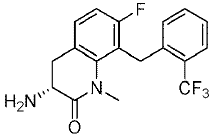 | D-23 | 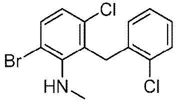 |
| D-24 | 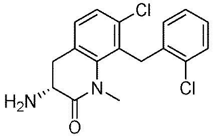 | D-25 | 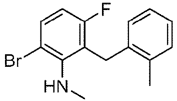 |
| D-26 | 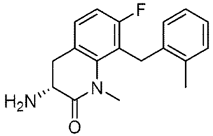 | D-27 | 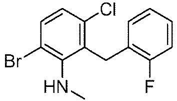 |
| D-28 | 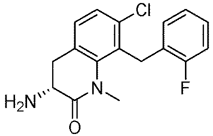 | D-29 | 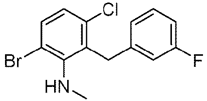 |

**[Table 10]**

| Ref. No. | Str. | Ref. No. | Str. |
|---|---|---|---|
| D-30 | 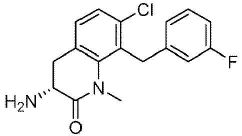 | D-31 | 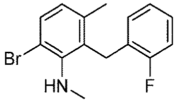 |
| D-32 | 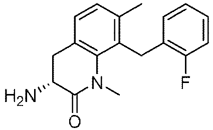 | D-33 | 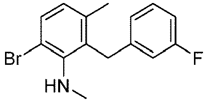 |
| D-34 | 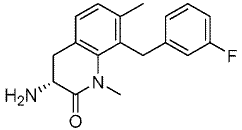 | D-35 | 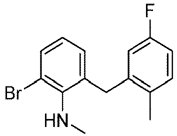 |
| D-36 | 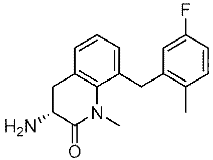 | E-1 | 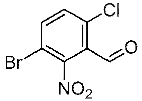 |
| E-2 | 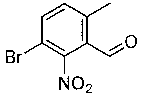 | | |

**[Table 11]**

| Ref. No. | Str. | Ref. No. | Str. |
|---|---|---|---|
| F-1 | 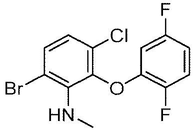 | F-2 | 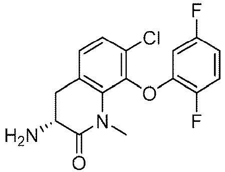 |
| F-3 | 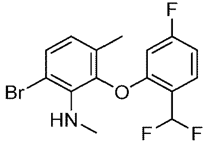 | F-4 | 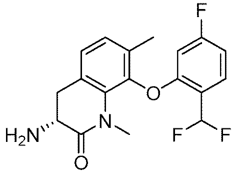 |
| F-5 | 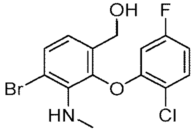 | F-6 | 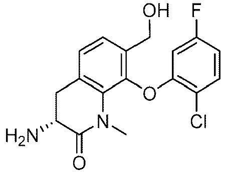 |
| F-7 | 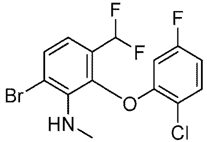 | F-8 | 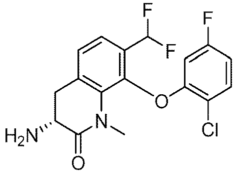 |
| F-9 | 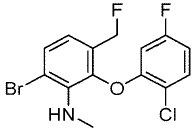 | F-10 | 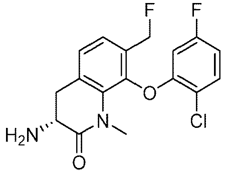 |
| F-11 | 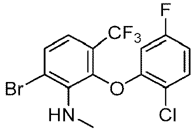 | F-12 | 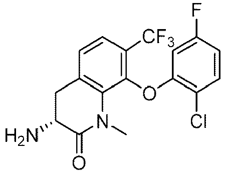 |
| F-13 | 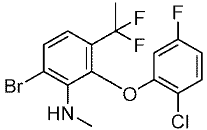 | F-14 | 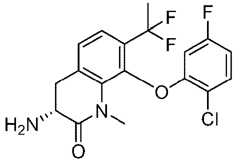 |

**[Table 12]**

| Ref. No. | Str. | Ref. No. | Str. |
|---|---|---|---|
| F-15 | 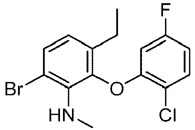 | F-16 | 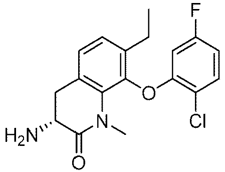 |
| G-1 | 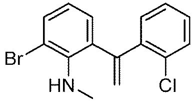 | G-2 | 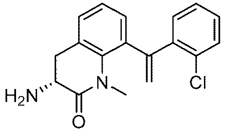 |
| G-3 | 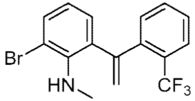 | G-4 | 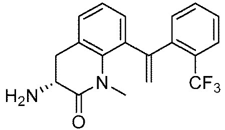 |
| G-5 | 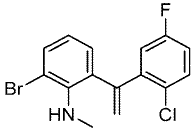 | G-6 | 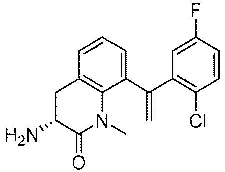 |

### Example 1

### 2-(1-methyl-2-oxo-8-phenoxy-1,2,3,4-tetrahydroquinolin-3-yl)acetamide

LDA (1.09 mol/L in THF/n-hexane) (0.188 mL) was added to a mixture of Reference Example A-3 (0.040 g) and THF (2 mL) at -78°C, and the mixture was stirred at the same temperature for 30 minutes. tert-butyl bromoacetate (0.062 g) was added to the reaction mixture at -78°C, and the mixture was stirred at the same temperature for 30 minutes and under ice cooling for 30 minutes. A saturated ammonium chloride aqueous solution was added to the reaction mixture under ice cooling. The mixture was extracted with ethyl acetate, and the extract was concentrated under reduced pressure. The residue was purified by silica gel column chromatography (elution solvent: n-hexane/ethyl acetate=100/0 to 2/98) to obtain tert-butyl 2-(1-methyl-2-oxo-8-phenoxy-1,2,3,4-tetrahydroquinolin-3-yl)acetate (0.032 g).

A mixture of the obtained Compound (0.032 g) and hydrogen chloride (4 mol/L in 1,4-dioxane) (2 mL) was stirred at room temperature for 1 hour. The reaction mixture was concentrated under reduced pressure. TEA (0.045 g), HATU (0.044 g) and ammonia (2 mol/L in methanol) (0.090 mL) were added to a mixture of the residue and DCM (2 mL), and the mixture was stirred at room temperature for 1 hour. A saturated sodium bicarbonate aqueous solution was added to the reaction mixture. The mixture was extracted with DCM, and the extract was concentrated under reduced pressure. The residue was purified by silica gel column chromatography (elution solvent: n-hexane/ethyl acetate/methanol=90/10/0 to 0/100/0 to 0/49/51) to obtain a title compound (0.016 g).

### Example 2

### (1-methyl-2-oxo-8-phenoxy-1,2,3,4-tetrahydroquinolin-3-yl)urea

LDA (1.09 mol/L in THF/n-hexane) (0.905 mL) was added to a mixture of Reference Example A-3 (0.100 g) and THF (2 mL) at -78°C, and the mixture was stirred at the same temperature for 30 minutes. DPPA (0.272 g) was added to the reaction mixture at - 78°C, and the mixture was stirred at the same temperature for 30 minutes. A mixture of Boc₂O (0.172 g) and THF (0.5 mL) was added to the reaction mixture at -78°C, and the mixture was stirred at the same temperature for 30 minutes and under ice cooling for 30 minutes. The reaction mixture was stirred at room temperature for 15 minutes and at 50°C for 30 minutes. The reaction mixture was cooled to room temperature and a saturated ammonium chloride aqueous solution was then added. The mixture was extracted with DCM, and the extract was concentrated under reduced pressure. The residue was purified by silica gel column chromatography (elution solvent: n-hexane/ethyl acetate=100/0 to 66/34) to obtain tert-butyl (1-methyl-2-oxo-8-phenoxy-1,2,3,4-tetrahydroquinolin-3-yl)carbamate (0.125 g).

A mixture of the obtained Compound (0.125 g) and hydrogen chloride (4 mol/L in 1,4-dioxane) (2 mL) was stirred at room temperature for 1 hour. The reaction mixture was concentrated under reduced pressure to obtain 3-amino-1-methyl-8-phenoxy-3,4-dihydroquinolin-2(1H)-one hydrochloride (0.114 g).

TEA (0.017 g) and trimethylsilyl isocyanate (0.057 g) were added to a mixture of the obtained Compound (0.025 g) and THF (1 mL), and the mixture was stirred at room temperature for 1 hour. Water and DCM were added to the reaction mixture. The mixture was extracted with DCM, and the extract was concentrated under reduced pressure. The residue was purified by ODS column chromatography (elution solvent: water/MeCN=70/30 to 10/90) to obtain a title compound (0.010 g).

### Example 3

### (8-(2-fluorophenoxy)-1-methyl-2-oxo-1,2,3,4-tetrahydroquinolin-3-yl)urea

LDA (1.09 mol/L in THF/n-hexane) (0.220 mL) was added to a mixture of Reference Example A-5 (0.050 g) and THF (2 mL) at -78°C, and the mixture was stirred at the same temperature for 30 minutes. DPPA (0.127 g) was added to the reaction mixture at - 78°C, and the mixture was stirred at the same temperature for 30 minutes. A mixture of Boc₂O (0.080 g) and THF (0.5 mL) was added to the reaction mixture at -78°C, and the mixture was stirred at the same temperature for 30 minutes and under ice cooling for 30 minutes. The reaction mixture was stirred at room temperature for 15 minutes and at 50°C for 30 minutes. The reaction mixture was cooled to room temperature, and a saturated ammonium chloride aqueous solution was then added. The mixture was extracted with DCM, and the extract was concentrated under reduced pressure. The residue was purified by silica gel column chromatography (elution solvent: n-hexane/ethyl acetate=76/24 to 41/59) to obtain tert-butyl (8-(2-fluorophenoxy)-1-methyl-2-oxo-1,2,3,4-tetrahydroquinolin-3-yl)carbamate (0.065 g).

A mixture of the obtained Compound (0.065 g) and hydrogen chloride (4 mol/L in 1,4-dioxane) (1 mL) was stirred at room temperature for 30 minutes. The reaction mixture was concentrated under reduced pressure. The mixture of the residue and methanol was passed through APS, and the filtrate was concentrated under reduced pressure. A mixture of potassium cyanate (0.018 g) and water (0.1 mL), and methanesulfonic acid (0.018 g) were added to a mixture of the residue and THF (1 mL), and the mixture was stirred at room temperature for 1 hour. A saturated sodium bicarbonate aqueous solution and DCM were added to the reaction mixture. The mixture was extracted with DCM, and the extract was concentrated under reduced pressure. The residue was purified by silica gel column chromatography (elution solvent: n-hexane/ethyl acetate/methanol=90/10/0 to 0/100/0 to 0/62/38) to obtain a title compound (0.028 g).

### Example 4

### (8-(3-fluorophenoxy)-1-methyl-2-oxo-1,2,3,4-tetrahydroquinolin-3-yl)urea

LDA (1.09 mol/L in THF/n-hexane) (0.330 mL) was added to a mixture of Reference Example A-7 (0.075 g) and THF (2 mL) at -78°C, and the mixture was stirred at the same temperature for 30 minutes. DPPA (0.190 g) was added to the reaction mixture at - 78°C, and the mixture was stirred at the same temperature for 30 minutes. A mixture of Boc₂O (0.121 g) and THF (0.5 mL) was added to the reaction mixture at -78°C, and the mixture was stirred at the same temperature for 30 minutes and under ice cooling for 30 minutes. The reaction mixture was stirred at room temperature for 15 minutes and at 50°C for 30 minutes. The reaction mixture was cooled to room temperature and a saturated ammonium chloride aqueous solution was then added. The mixture was extracted with DCM, and the extract was concentrated under reduced pressure. The residue was purified by silica gel column chromatography (elution solvent: n-hexane/ethyl acetate=90/10 to 41/59) to obtain tert-butyl (8-(3-fluorophenoxy)-1-methyl-2-oxo-1,2,3,4-tetrahydroquinolin-3-yl)carbamate (0.039 g).

A mixture of the obtained Compound (0.039 g) and hydrogen chloride (4 mol/L in 1,4-dioxane) (1 mL) was stirred at room temperature for 30 minutes. The reaction mixture was concentrated under reduced pressure. The mixture of the residue and methanol was passed through APS, and the effluent was concentrated under reduced pressure. A mixture of potassium cyanate (0.010 g) and water (0.1 mL), and methanesulfonic acid (0.010 g) were added to a mixture of the residue and THF (1 mL), and the mixture was stirred at room temperature for 3 hours. A saturated sodium bicarbonate aqueous solution and ethyl acetate were added to the reaction mixture. The mixture was extracted with ethyl acetate, and the extract was concentrated under reduced pressure. The mixture of the residue and methanol was passed through APS, and the effluent was concentrated under reduced pressure. The residue was purified by ODS column chromatography (elution solvent: water/MeCN=70/30 to 10/90) to obtain a title compound (0.016 g).

### Example 5

### (1-methyl-2-oxo-8-(3-(trifluoromethyl)phenoxy)-1,2,3,4-tetrahydroquinolin-3-yl)urea

LDA (1.09 mol/L in THF/n-hexane) (2.25 mL) was added to a mixture of Reference Example A-9 (0.607 g) and THF (2 mL) at -78°C, and the mixture was stirred at the same temperature for 30 minutes. DPPA (1.04 g) was added to the reaction mixture at -78°C, and the mixture was stirred at the same temperature for 30 minutes. A mixture of Boc₂O (0.825 g) and THF (0.5 mL) was added to the reaction mixture at -78°C, and the mixture was stirred at the same temperature for 30 minutes, and under ice cooling for 30 minutes. The reaction mixture was stirred at room temperature for 15 minutes and at 50°C for 30 minutes. The reaction mixture was cooled to room temperature and a saturated ammonium chloride aqueous solution was then added. The mixture was extracted with DCM, and the extract was concentrated under reduced pressure. The residue was purified by silica gel column chromatography (elution solvent: n-hexane/ethyl acetate=95/5 to 67/33) to obtain tert-butyl (1-methyl-2-oxo-8-(3-(trifluoromethyl)phenoxy)-1,2,3,4-tetrahydroquinolin-3-yl)carbamate (0.830 g).

A mixture of the obtained Compound (0.050 g) and hydrogen chloride (4 mol/L in 1,4-dioxane) (1 mL) was stirred at room temperature for 30 minutes. The reaction mixture was concentrated under reduced pressure. The mixture of the residue and methanol was passed through APS, and the effluent was concentrated under reduced pressure. A mixture of potassium cyanate (0.012 g) and water (0.1 mL), and methanesulfonic acid (0.011 g) were added to the mixture of the residue and THF (1 mL), and the mixture was stirred at room temperature for 1 hour. A saturated sodium bicarbonate aqueous solution and ethyl acetate were added to the reaction mixture. The mixture was extracted with ethyl acetate, and the extract was concentrated under reduced pressure. The mixture of the residue and methanol was passed through APS, and the effluent was concentrated under reduced pressure. The residue was purified by silica gel column chromatography (elution solvent: ethyl acetate/methanol=100/0 to 80/20) to obtain a title compound (0.030 g).

### Example 6

### (8-(3-chlorophenoxy)-1-methyl-2-oxo-1,2,3,4-tetrahydroquinolin-3-yl)urea

LDA (1.09 mol/L in THF/n-hexane) (1.32 mL) was added to a mixture of Reference Example A-11 (0.345 g) and THF (5 mL) at -78°C, and the mixture was stirred at the same temperature for 30 minutes. DPPA (0.660 g) was added to the reaction mixture at -78°C, and the mixture was stirred at the same temperature for 30 minutes. A mixture of Boc₂O (0.523 g) and THF (0.5 mL) was added to the mixture at -78°C, and the mixture was stirred at the same temperature for 30 minutes and under ice cooling for 30 minutes. The reaction mixture was stirred at room temperature for 15 minutes and at 50°C for 30 minutes. The reaction mixture was cooled to room temperature and a saturated ammonium chloride aqueous solution was then added. The mixture was extracted with DCM, and the extract was concentrated under reduced pressure. The residue was purified by silica gel column chromatography (elution solvent: n-hexane/ethyl acetate=100/0 to 73/27) to obtain tert-butyl N-(8-(3-chlorophenoxy)-1-methyl-2-oxo-1,2,3,4-tetrahydroquinolin-3-yl)carbamate (0.378 g).

A mixture of the obtained Compound (0.065 g) and hydrogen chloride (4 mol/L in 1,4-dioxane) (1 mL) was stirred at room temperature for 30 minutes. The reaction mixture was concentrated under reduced pressure. The mixture of the residue and methanol was passed through APS, and the effluent was concentrated under reduced pressure. A mixture of potassium cyanate (0.017 g) and water (0.1 mL), and methanesulfonic acid (0.017 g) were added to a mixture of the residue and THF (1 mL), and the mixture was stirred at room temperature for 14 hours. A saturated sodium bicarbonate aqueous solution, and DCM were added to the reaction mixture. The mixture was extracted with DCM, and the extract was concentrated under reduced pressure. The residue was suspended in diethyl ether, and insoluble substances were collected by filtration. The obtained solid was dried under reduced pressure to obtain a title compound (0.028 g).

### Example 7

Example 7 was synthesized in the same method as in Example 3 except that Reference Example A-13 was used in place of Reference Example A-5.

### Example 8

Example 8 was synthesized in the same method as in Example 6 except that Reference Example A-15 was used in place of Reference Example A-11.

### Example 9

### (1-methyl-2-oxo-8-((3-(trifluoromethyl)pyridine-2-yl)oxy)-1,2,3,4-tetrahydroquinolin-3-yl)urea

LDA (1.09 mol/L in THF/n-hexane) (1.7 mL) was added to a mixture of Reference Example A-16 (0.240 g) and THF (2 mL) at -78°C, and the mixture was stirred at the same temperature for 30 minutes. DPPA (0.512 g) was added to the reaction mixture at -78°C, and the mixture was stirred at the same temperature for 1 hour. A mixture of Boc₂O (0.325 g) and THF (1 mL) was added to the reaction mixture at -78°C, and the mixture was stirred at the same temperature for 10 minutes and under ice cooling for 1 hour. Water (3 mL) was added to the reaction mixture under ice cooling, and the mixture was then stirred at 50°C for 1 hour. The reaction mixture was cooled to room temperature and a saturated ammonium chloride aqueous solution and ethyl acetate were then added. The mixture was extracted with ethyl acetate, and the extract was washed with a saturated saline solution. The extract was dried with anhydrous sodium sulfate and then concentrated under reduced pressure. The residue was purified by Method A (elution solvent: n-hexane/ethyl acetate=90/10 to 60/40) to obtain tert-butyl (1-methyl-2-oxo-8-((3-(trifluoromethyl)pyridine-2-yl)oxy)-1,2,3,4-tetrahydroquinolin-3-yl)carbamate (0.091 g).

TFA (0.469 g) was added to a mixture of the obtained Compound (0.090 g) and DCM (1 mL) under ice cooling, and the mixture was stirred at room temperature for 1.5 hours. A 1 mol/L sodium hydroxide aqueous solution (5 mL) was added to the reaction mixture under ice cooling. The mixture was extracted with DCM, and the extract was concentrated under reduced pressure.

Potassium cyanate (0.022 g) and methanesulfonic acid (0.022 g) were added to a mixture of the residue, water (0.037 g) and THF (1 mL), and the mixture was stirred at room temperature for 5 hours. A saturated sodium bicarbonate aqueous solution and water were added to the reaction mixture, and the mixture was stirred at room temperature for 10 minutes. Insoluble substances were collected by filtration, and the obtained solid was washed with water, MTBE and then dried under reduced pressure to obtain a title compound (0.045 g).

### Example 10

### ((3R)-1-methyl-2-oxo-8-(2-(trifluoromethyl)phenoxy)-1,2,3,4-tetrahydroquinolin-3-yl)urea

Potassium cyanate (0.029 g), water (0.043 g) and methanesulfonic acid (0.025 g) were added to a mixture of Reference Example B-2 (0.080 g) and THF (1 mL), and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=49/49/2 to 0/98/2 to 0/86/14), and then APS column chromatography (elution solvent: n-hexane/ethyl acetate/methanol=49/49/2 to 0/98/2 to 0/86/14) to obtain a title compound (0.040 g).

### Example 11

### ((3R)-8-(2-methoxyphenoxy)-1-methyl-2-oxo-1,2,3,4-tetrahydroquinolin-3-yl)urea

A mixture of potassium cyanate (0.061 g) and water (0.1 mL), and methanesulfonic acid (0.061 g) were added to a mixture of Reference Example B-4 (0.173 g) and THF (1 mL), and the mixture was stirred at room temperature for 14 hours. A saturated sodium bicarbonate aqueous solution and diethyl ether were added to the reaction mixture. Insoluble substances were collected by filtration, and the obtained solid was dried under reduced pressure to obtain a title compound (0.109 g).

### Example 12

### ((3R)-8-(3-cyanophenoxy)-1-methyl-2-oxo-1,2,3,4-tetrahydroquinolin-3-yl)urea

Potassium cyanate (0.184 g) and acetic acid (0.108 g) were added to a mixture of Reference Example B-6 (0.504 g), THF (5 mL) and water (0.5 mL), and the mixture was stirred at room temperature for 3 hours. Water and ethyl acetate were added to the reaction mixture. The mixture was extracted with ethyl acetate, and the extract was concentrated under reduced pressure. The residue was suspended in DCM, and insoluble substances were collected by filtration. The obtained solid was dried under reduced pressure to obtain a title compound (0.068 g).

### Example 13 to Example 15

Example 13 to Example 15 were synthesized in the same method as in Example 11 except that corresponding raw materials were used in place of Reference Example B-4.

### Example 16

### ((3R)-8-(2-chloro-5-fluorophenoxy)-1-methyl-2-oxo-1,2,3,4-tetrahydroquinolin-3-yl)urea

A mixture of potassium cyanate (0.016 g) and water (0.1 mL), and methanesulfonic acid (0.018 g) were added to a mixture of Reference Example B-14 (0.050 g) and THF (2 mL), and the mixture was stirred at room temperature for 14 hours. A saturated sodium bicarbonate aqueous solution and diethyl ether were added to the reaction mixture. Insoluble substances were collected by filtration, and the obtained solid was dried under reduced pressure to obtain a title compound (0.031 g).

### Example 17

### ((3R)-1-methyl-2-oxo-8-(2-(propan-2-yl)phenoxy)-1,2,3,4-tetrahydroquinolin-3-yl)urea

Potassium cyanate (0.095 g) and acetic acid (0.055 g) were added to a mixture of Reference Example B-16 (0.273 g), THF (3 mL) and water (0.3 mL), and the mixture was stirred at room temperature for 1.5 hours. Water and diethyl ether were added to the reaction mixture. The mixture was extracted with diethyl ether, and the extract was concentrated under reduced pressure. The residue was suspended in diethyl ether, and insoluble substances were collected by filtration. The obtained solid was dried under reduced pressure to obtain a title compound (0.233 g).

### Example 18

### ((3R)-8-(2-(difluoromethyl)phenoxy)-1-methyl-2-oxo-1,2,3,4-tetrahydroquinolin-3-yl)urea

Potassium cyanate (0.037 g) and acetic acid (0.021 g) were added to a mixture of Reference Example B-18 (0.108 g), THF (1 mL) and water (0.1 mL), and the mixture was stirred at room temperature for 1.5 hours. Water was added to the reaction mixture. Insoluble substances were collected by filtration, and the obtained solid was dried under reduced pressure to obtain a title compound (0.091g).

### Example 19

### ((3R)-8-(2-chlorophenoxy)-7-fluoro-1-methyl-2-oxo-1,2,3,4-tetrahydroquinolin-3-yl)urea

A mixture of potassium cyanate (0.098 g) and water (0.1 mL), and acetic acid (0.062 g) were added to a mixture of Reference Example B-20 (0.278 g) and THF (2 mL), and the mixture was stirred at room temperature for 14 hours. A saturated sodium bicarbonate aqueous solution and DCM were added to the reaction mixture. The mixture was extracted with DCM, and the extract was concentrated under reduced pressure. Diethyl ether was added to the residue. Insoluble substances were collected by filtration, and the obtained solid was dried under reduced pressure to obtain a title compound (0.209 g).

### Example 20

### ((3R)-8-(2-chloro-4-fluorophenoxy)-1-methyl-2-oxo-1,2,3,4-tetrahydroquinolin-3-yl)urea

A mixture of potassium cyanate (0.104 g) and water (0.1 mL), and acetic acid (0.066 g) were added to a mixture of Reference Example B-22 (0.295 g) and THF (2 mL), and the mixture was stirred at room temperature for 14 hours. A saturated sodium bicarbonate aqueous solution and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.220 g).

### Example 21

### ((3R)-8-(5-fluoro-2-(trifluoromethyl)phenoxy)-1-methyl-2-oxo-1,2,3,4-tetrahydroquinolin-3-yl)urea

Potassium cyanate (0.011 g), water (0.019 g) and methanesulfonic acid (0.011 g) were added to a mixture of Reference Example B-24 (0.038 g) and THF (0.5 mL), and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=49/49/2 to 0/98/2 to 0/86/14), and then APS column chromatography (elution solvent: ethyl acetate/methanol=98/2 to 88/12) to obtain a title compound (0.013 g).

### Example 22

### ((3R)-7-chloro-8-(2-chlorophenoxy)-1-methyl-2-oxo-1,2,3,4-tetrahydroquinolin-3-yl)urea

Potassium cyanate (0.012 g), water (0.020 g) and methanesulfonic acid (0.011 g) were added to a mixture of Reference Example B-26 (0.038 g) and THF (0.5 mL), and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was suspended in ethyl acetate, and insoluble substances were collected by filtration. The obtained solid was dried under reduced pressure to obtain a title compound (0.018 g).

### Example 23

### ((3R)-7-chloro-8-(2-fluorophenoxy)-1-methyl-2-oxo-1,2,3,4-tetrahydroquinolin-3-yl)urea

Potassium cyanate (0.014 g), water (0.025 g) and methanesulfonic acid (0.014 g) were added to a mixture of Reference Example B-28 (0.044 g) and THF (0.5 mL), and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=49/49/2 to 0/98/2 to 0/86/14) to obtain a title compound (0.042 g).

### Example 24

### ((3R)-8-(3-(difluoromethoxy)phenoxy)-1-methyl-2-oxo-1,2,3,4-tetrahydroquinolin-3-yl)urea

Potassium cyanate (0.060 g) and acetic acid (0.036 g) were added to a mixture of Reference Example B-30 (0.188 g), THF (2 mL) and water (0.2 mL), and the mixture was stirred at room temperature for 1.5 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.175 g).

### Example 25

Example 25 was synthesized in the same method as in Example 23 except that B-32 was used in place of Reference Example B-28.

### Example 26

### ((3R)-7-chloro-8-(3-fluorophenoxy)-1-methyl-2-oxo-1,2,3,4-tetrahydroquinolin-3-yl)urea

Potassium cyanate (0.014 g), water (0.024 g) and methanesulfonic acid (0.014 g) were added to a mixture of Reference Example B-34 (0.043 g) and THF (0.5 mL), and the mixture was stirred at room temperature for 14 hours. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=49/49/2 to 0/98/2 to 0/86/14) to obtain a title compound (0.042 g).

### Example 27

### ((3R)-7-chloro-8-(2-(difluoromethyl)phenoxy)-1-methyl-2-oxo-1,2,3,4-tetrahydroquinolin-3-yl)urea

Potassium cyanate (0.008 g), water (0.014 g) and methanesulfonic acid (0.008 g) were added to a mixture of Reference Example B-36 (0.027 g) and THF (0.5 mL), and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was purified by APS column chromatography (elution solvent: n-hexane/ethyl acetate/methanol=49/49/2 to 0/98/2 to 0/86/14), and then silica gel column chromatography (elution solvent: n-hexane/ethyl acetate/methanol=49/49/2 to 0/98/2 to 0/86/14) to obtain a title compound (0.015 g).

### Example 28

### ((3R)-8-(2-(difluoromethyl)-5-fluorophenoxy)-1-methyl-2-oxo-1,2,3,4-tetrahydroquinolin-3-yl)urea

Potassium cyanate (0.008 g) and acetic acid (0.005 g) were added to a mixture of Reference Example B-38 (0.025 g), THF (1 mL) and water (0.1 mL), and the mixture was stirred at room temperature for 15 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.011 g).

### Example 29

Example 29 was synthesized in the same method as in Example 24 except that Reference Example B-40 was used in place of Reference Example B-30.

### Example 30

### ((3R)-8-(2-chloro-5-methoxyphenoxy)-1-methyl-2-oxo-1,2,3,4-tetrahydroquinolin-3-yl)urea

A mixture of potassium cyanate (0.045 g) and water (0.1 mL), and acetic acid (0.029 g) were added to a mixture of Reference Example B-42 (0.133 g) and THF (2 mL), and the mixture was stirred at room temperature for 14 hours. Water and DCM were added to the reaction mixture. The mixture was extracted with DCM, and the extract was concentrated under reduced pressure. The residue was purified by ODS column chromatography (elution solvent: water/MeCN=70/30 to 10/90) to obtain a title compound (0.056 g).

### Example 31

### ((3R)-8-(2-chlorophenoxy)-1,7-dimethyl-2-oxo-1,2,3,4-tetrahydroquinolin-3-yl)urea

A mixture of potassium cyanate (0.027 g) and water (0.1 mL), and acetic acid (0.017 g) were added to a mixture of Reference Example B-44 (0.074 g) and THF (2 mL), and the mixture was stirred at room temperature for 14 hours. Water and MTBE were added to the reaction mixture. Insoluble substances were collected by filtration, and the obtained solid was dried under reduced pressure to obtain a title compound (0.031 g).

### Example 32

### ((3R)-7-(benzyloxy)-8-(2-chlorophenoxy)-1-methyl-2-oxo-1,2,3,4-tetrahydroquinolin-3-yl)urea

A mixture of potassium cyanate (0.028 g) and water (0.1 mL), and acetic acid (0.018 g) were added to a mixture of Reference Example B-46 (0.100 g) and THF (2 mL) and the mixture was stirred at room temperature for 14 hours. Water and MTBE were added to the reaction mixture. Insoluble substances were collected by filtration, and the obtained solid was purified by ODS column chromatography (elution solvent: water/MeCN=70/30 to 10/90) to obtain a title compound (0.042 g).

### Example 33

### ((3R)-8-(2-chlorophenoxy)-7-hydroxy-1-methyl-2-oxo-1,2,3,4-tetrahydroquinolin-3-yl)urea

10% Pd/C (0.008 g) was added to a mixture of Example 32 (0.034 g), THF (0.5 mL) and methanol (0.5 mL) and the mixture was stirred at room temperature under a hydrogen atmosphere for 1 hour. The reaction mixture was filtered through Celite, and the filtrate was concentrated under reduced pressure. The residue was purified by ODS column chromatography (elution solvent: water/MeCN=70/30 to 10/90) to obtain a title compound (0.016 g).

### Example 34

### ((3R)-8-(2-chlorophenoxy)-1-ethyl-2-oxo-1,2,3,4-tetrahydroquinolin-3-yl)urea

A mixture of potassium cyanate (0.053 g) and water (0.1 mL), and acetic acid (0.037 g) were added to a mixture of Reference Example B-48 (0.138 g) and THF (2 mL) and the mixture was stirred at room temperature for 14 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.036 g).

### Example 35

### ((3R)-8-(2-chloro-4,5-difluorophenoxy)-1-methyl-2-oxo-1,2,3,4-tetrahydroquinolin-3-yl)urea

Potassium cyanate (0.021 g), water (0.036 g) and methanesulfonic acid (0.020 g) were added to a mixture of Reference Example B-50 (0.068 g) and THF (1 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was purified sequentially by silica gel column chromatography (elution solvent: n-hexane/ethyl acetate/methanol=49/49/2 to 0/98/2 to 0/86/14), APS column chromatography (elution solvent: n-hexane/ethyl acetate/methanol=49/49/2 to 0/98/2 to 0/86/14) and ODS column chromatography (elution solvent: water/MeCN=70/30 to 10/90) to obtain a title compound (0.018 g).

### Example 36

Example 36 was synthesized in the same method as in Example 10 except that Reference Example B-52 was used in place of Reference Example B-2.

### Example 37

### ((3R)-7-chloro-8-(2-chloro-5-fluorophenoxy)-1-methyl-2-oxo-1,2,3,4-tetrahydroquinolin-3-yl)urea

Potassium cyanate (0.001 g), water (0.002 g) and methanesulfonic acid (0.001 g) were added to a mixture of Reference Example B-54 (0.003 g) and THF (0.2 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=49/49/2 to 0/98/2 to 0/86/14) to obtain a title compound (0.003 g).

### Example 38

### ((3R)-8-(2-(difluoromethyl)phenoxy)-1,7-dimethyl-2-oxo-1,2,3,4-tetrahydroquinolin-3-yl)urea

Potassium cyanate (0.027 g) and acetic acid (0.017 g) were added to a mixture of Reference Example B-56 (0.080 g), THF (1 mL) and water (0.1 mL) and the mixture was stirred at room temperature for 3.5 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.070 g).

### Example 39

### ((3R)-8-(2-chloro-5-fluorophenoxy)-1,7-dimethyl-2-oxo-1,2,3,4-tetrahydroquinolin-3-yl)urea

Potassium cyanate (0.039 g) and acetic acid (0.025 g) were added to a mixture of Reference Example B-58 (0.118 g), THF (1 mL) and water (0.1 mL) and the mixture was stirred at room temperature for 3.5 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.062 g).

### Example 40

### ((3R)-8-(2-chlorophenoxy)-1-methyl-2-oxo-1,2,3,4-tetrahydroquinolin-3-yl)urea

Potassium cyanate (0.129 g), water (0.220 g) and methanesulfonic acid (0.123 g) were added to a mixture of Reference Example B-60 (0.370 g) and THF (2.5 mL) and the mixture was stirred at room temperature for 2 hours. A saturated sodium bicarbonate aqueous solution, water and ethyl acetate were added to the reaction mixture. The mixture was extracted with ethyl acetate, and the extract was washed with a saturated saline solution. The extract was dried with anhydrous sodium sulfate and then concentrated under reduced pressure. The residue was purified by silica gel column chromatography (elution solvent: n-hexane/ethyl acetate/methanol=49/49/2 to 0/98/2 to 0/86/14) to obtain a title compound (0.390 g).

### Example 41

Example 41 was synthesized in the same method as in Example 23 except that Reference Example B-62 was used in place of Reference Example B-28.

### Example 42

### ((3R)-8-(cyclopentyloxy)-1-methyl-2-oxo-1,2,3,4-tetrahydroquinolin-3-yl)urea

Potassium cyanate (0.041 g), water (0.069 g) and methanesulfonic acid (0.039 g) were added to a mixture of Reference Example B-64 (0.100 g) and THF (1 mL) and the mixture was stirred at room temperature for 2 hours. A saturated sodium bicarbonate aqueous solution, water, DCM and ethyl acetate were added to the reaction mixture. The mixture was extracted with DCM, and the extract was concentrated under reduced pressure. Ethyl acetate and water were added to the residue. Insoluble substances were collected by filtration, and the obtained solid was dried under reduced pressure to obtain a title compound (0.076 g).

### Example 43

### ((3R)-8-(2-(difluoromethyl)phenoxy)-7-fluoro-1-methyl-2-oxo-1,2,3,4-tetrahydroquinolin-3-yl)urea

A mixture of potassium cyanate (0.013 g) and water (0.1 mL), and acetic acid (0.009 g) were added to a mixture of Reference Example B-66 (0.035 g) and THF (2 mL) and the mixture was stirred at room temperature for 14 hours. Water and MTBE were added to the reaction mixture. Insoluble substances were collected by filtration, and the obtained solid was dried under reduced pressure to obtain a title compound (0.030 g).

### Example 44

### ((3R)-8-(2-chloro-5-fluorophenoxy)-7-fluoro-1-methyl-2-oxo-1,2,3,4-tetrahydroquinolin-3-yl)urea

A mixture of potassium cyanate (0.025 g) and water (0.1 mL), and acetic acid (0.017 g) were added to a mixture of Reference Example B-68 (0.070 g) and THF (2 mL) and the mixture was stirred at room temperature for 14 hours. Water and MTBE were added to the reaction mixture. Insoluble substances were collected by filtration, and the obtained solid was dried under reduced pressure to obtain a title compound (0.052 g).

### Example 45

### ((3R)-8-(2-fluorophenoxy)-1,7-dimethyl-2-oxo-1,2,3,4-tetrahydroquinolin-3-yl)urea

A mixture of potassium cyanate (0.013 g) and water (0.1 mL), and acetic acid (0.009 g) were added to a mixture of Reference Example B-70 (0.031 g) and THF (2 mL) and the mixture was stirred at room temperature for 14 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.030 g).

### Example 46

### ((3R)-8-(3-fluorophenoxy)-1,7-dimethyl-2-oxo-1,2,3,4-tetrahydroquinolin-3-yl)urea

A mixture of potassium cyanate (0.026 g) and water (0.1 mL), and acetic acid (0.018 g) were added to a mixture of Reference Example B-72 (0.065 g) and THF (2 mL), and the mixture was stirred at room temperature for 14 hours. Water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.027 g).

### Example 47

### ((3R)-1-methyl-8-(2-methylphenoxy)-2-oxo-1,2,3,4-tetrahydroquinolin-3-yl)urea

Potassium cyanate (0.108 g), water (0.185 g) and methanesulfonic acid (0.104 g) were added to a mixture of Reference Example B-74 (0.290 g) and THF (3 mL), and the mixture was stirred at room temperature for 1 hour. A saturated sodium bicarbonate aqueous solution, water and ethyl acetate were added to the reaction mixture. The mixture was extracted with ethyl acetate, and the extract was washed with a saturated saline solution. The extract was dried with anhydrous sodium sulfate and then concentrated under reduced pressure. The residue was suspended in MTBE, and insoluble substances were collected by filtration. The obtained solid was dried under reduced pressure to obtain a title compound (0.210 g).

### Example 48

### ((3R)-8-(5-fluoro-2-methylphenoxy)-1-methyl-2-oxo-1,2,3,4-tetrahydroquinolin-3-yl)urea

Potassium cyanate (0.077 g), water (0.132 g) and methanesulfonic acid (0.074 g) were added to a mixture of Reference Example B-76 (0.220 g) and THF (2 mL), and the mixture was stirred at room temperature for 2 hours. A saturated sodium bicarbonate aqueous solution, water and ethyl acetate were added to the reaction mixture. The mixture was extracted with ethyl acetate, and the extract was washed with a saturated saline solution. The extract was dried with anhydrous sodium sulfate and then concentrated under reduced pressure. The residue was suspended in MTBE, and insoluble substances were collected by filtration. The obtained solid was dried under reduced pressure to obtain a title compound (0.190 g).

### Example 49

### ((3R)-8-(2-fluorophenoxy)-1-methyl-2-oxo-7-(trifluoromethyl)-1,2,3,4-tetrahydroquinolin-3-yl)urea

Potassium cyanate (0.008 g), water (0.013 g) and methanesulfonic acid (0.007 g) were added to a mixture of Reference Example C-3 (0.026 g) and THF (1 mL), and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: n-hexane/ethyl acetate/methanol=50/50/0 to 0/100/0 to 0/90/10) to obtain a title compound (0.025 g).

### Example 50

### ((3R)-8-(2,5-difluorophenoxy)-1,7-dimethyl-2-oxo-1,2,3,4-tetrahydroquinolin-3-yl)urea

Potassium cyanate (0.094 g), water (0.278 g) and acetic acid (0.065 g) were added to a mixture of Reference Example B-78 (0.274 g), THF (1.5 mL) and methanol (1.0 mL), and the mixture was stirred at room temperature for 1 hour. Water and DCM were added to the reaction mixture. The mixture was extracted with DCM, and the extract was concentrated under reduced pressure. The residue was suspended in MTBE, and the solid was collected by filtration. The obtained solid was dried under reduced pressure to obtain a title compound (0.242 g).

### Example 51

### ((3R)-8-((2-chlorophenyl)methyl)-1-methyl-2-oxo-1,2,3,4-tetrahydroquinolin-3-yl)urea

Potassium cyanate (0.042 g), water (0.072 g) and methanesulfonic acid (0.040 g) were added to a mixture of Reference Example D-2 (0.120 g) and THF (1 mL), and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate, and insoluble substances were collected by filtration. The obtained solid was dried under reduced pressure to obtain a title compound (0.096 g).

### Example 52

### ((3R)-8-((2-chloro-5-fluorophenyl)methyl)-1-methyl-2-oxo-1,2,3,4-tetrahydroquinolin-3-yl)urea

Potassium cyanate (0.033 g) and methanesulfonic acid (0.032 g) were added to a mixture of Reference Example D-4 (0.100 g), THF (0.8 mL) and water (0.057 mL), and the mixture was stirred at room temperature for 2.5 hours. Methanesulfonic acid (0.003 g) was added to the reaction mixture, and the mixture was stirred at room temperature for 30 minutes. A saturated sodium bicarbonate aqueous solution, water and DCM were added to the reaction mixture. The mixture was extracted with DCM, and the extract was concentrated under reduced pressure. The residue was suspended in ethyl acetate, and insoluble substances were collected by filtration. The obtained solid was dried under reduced pressure to obtain a title compound (0.055 g).

### Example 53

### ((3R)-1-methyl-2-oxo-8-((2-(trifluoromethyl)phenyl)methyl)-1,2,3,4-tetrahydroquinolin-3-yl)urea

Potassium cyanate (0.032 g) and methanesulfonic acid (0.030 g) were added to a mixture of Reference Example D-6 (0.100 g), THF (0.8 mL) and water (0.054 mL) and the mixture was stirred at room temperature for 2.5 hours. Methanesulfonic acid (0.003 g) was added to the reaction mixture and the mixture was stirred at room temperature for 30 minutes. A saturated sodium bicarbonate aqueous solution, water and DCM were added to the reaction mixture. The mixture was extracted with DCM, and the extract was concentrated under reduced pressure. The residue was purified by silica gel column chromatography (elution solvent: DCM/methanol=100/0 to 90/10). 2 mol/L hydrochloric acid was added to the obtained product, and the mixture was extracted with DCM. The extract was concentrated under reduced pressure. Ethyl acetate and n-hexane were added to the residue. The obtained solid was collected by filtration, and the obtained solid was dried under reduced pressure to obtain a title compound (0.026 g).

### Example 54

### ((3R)-1-methyl-8-((2-methylphenyl)methyl)-2-oxo-1,2,3,4-tetrahydroquinolin-3-yl)urea

Potassium cyanate (0.017 g) and methanesulfonic acid (0.017 g) were added to a mixture of Reference Example D-8 (0.046 g), THF (0.5 mL) and water (0.030 mL) and the mixture was stirred at room temperature for 2.5 hours. A saturated sodium bicarbonate aqueous solution, water and DCM were added to the reaction mixture. The mixture was extracted with DCM, and the extract was concentrated under reduced pressure. The residue was suspended in ethyl acetate, and insoluble substances were collected by filtration. The obtained solid was dried under reduced pressure to obtain a title compound (0.012 g).

### Example 55

### ((3R)-8-((2-(difluoromethyl)phenyl)methyl)-1-methyl-2-oxo-1,2,3,4-tetrahydroquinolin-3-yl)urea

Potassium cyanate (0.011 g) and methanesulfonic acid (0.010 g) were added to a mixture of Reference Example D-10 (0.032 g), THF (1 mL) and water (0.018 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate/n-hexane (1/1), and insoluble substances were collected by filtration. The obtained solid was dried under reduced pressure to obtain a title compound (0.018 g).

### Example 56

### ((3R)-8-((2-chloro-5-fluorophenyl)methyl)-7-fluoro-1-methyl-2-oxo-1,2,3,4-tetrahydroquinolin-3-yl)urea

Potassium cyanate (0.002 g) and methanesulfonic acid (0.002 g) were added to a mixture of Reference Example D-12 (0.006 g), THF (1 mL) and water (0.003 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was purified by APS column chromatography (elution solvent: DCM/methanol=100/0 to 90/10) to obtain a title compound (0.004 g).

### Example 57

### ((3R)-7-chloro-8-((2-chloro-5-fluorophenyl)methyl)-1-methyl-2-oxo-1,2,3,4-tetrahydroquinolin-3-yl)urea

Potassium cyanate (0.012 g), water (0.035 mL) and acetic acid (0.008 g) were added to a mixture of Reference Example D-14 (0.036 g), THF (0.3 mL) and methanol (0.2 mL) and the mixture was stirred at room temperature for 1.5 hours. Water and DCM were added to the reaction mixture. The mixture was extracted with DCM, and the extract was concentrated under reduced pressure. Ethyl acetate and n-hexane were added to the residue. The obtained solid was collected by filtration, and the obtained solid was dried under reduced pressure to obtain a title compound (0.004 g).

### Example 58

### ((3R)-8-((2-chlorophenyl)methyl)-1,7-dimethyl-2-oxo-1,2,3,4-tetrahydroquinolin-3-yl)urea

Potassium cyanate (0.003 g) and methanesulfonic acid (0.003 g) were added to a mixture of Reference Example D-16 (0.009 g), THF (0.3 mL) and water (0.005 mL) and the mixture was stirred at room temperature for 20 hours. A saturated sodium bicarbonate aqueous solution, water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.004 g).

### Example 59

### ((3R)-8-((2-chloro-5-fluorophenyl)methyl)-1,7-dimethyl-2-oxo-1,2,3,4-tetrahydroquinolin-3-yl)urea

Potassium cyanate (0.003 g) and methanesulfonic acid (0.002 g) were added to a mixture of Reference Example D-18 (0.008 g), THF (0.3 mL) and water (0.004 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.002 g).

### Example 60

### ((3R)-8-((2-chlorophenyl)methyl)-7-fluoro-1-methyl-2-oxo-1,2,3,4-tetrahydroquinolin-3-yl)urea

Potassium cyanate (0.016 g) and methanesulfonic acid (0.016 g) were added to a mixture of Reference Example D-20 (0.049 g), THF (1 mL) and water (0.028 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate/n-hexane (1/1), and insoluble substances were collected by filtration. The obtained solid was dried under reduced pressure to obtain a title compound (0.018 g).

### Example 61

### ((3R)-7-fluoro-1-methyl-2-oxo-8-((2-(trifluoromethyl)phenyl)methyl)-1,2,3,4-tetrahydroquinolin-3-yl)urea

Potassium cyanate (0.008 g) and methanesulfonic acid (0.008 g) were added to a mixture of Reference Example D-22 (0.028 g), THF (1 mL) and water (0.014 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate/n-hexane (1/1), and insoluble substances were collected by filtration. The obtained solid was dried under reduced pressure to obtain a title compound (0.014 g).

### Example 62

### ((3R)-7-chloro-8-((2-chlorophenyl)methyl)-1-methyl-2-oxo-1,2,3,4-tetrahydroquinolin-3-yl)urea

Potassium cyanate (0.008 g) and methanesulfonic acid (0.008 g) were added to a mixture of Reference Example D-24 (0.026 g), THF (1 mL) and water (0.014 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate/n-hexane (1/1), and insoluble substances were collected by filtration. The obtained solid was dried under reduced pressure to obtain a title compound (0.013 g).

### Example 63

### ((3R)-7-fluoro-1-methyl-8-((2-methylphenyl)methyl)-2-oxo-1,2,3,4-tetrahydroquinolin-3-yl)urea

Potassium cyanate (0.014 g) and methanesulfonic acid (0.014 g) were added to a mixture of Reference Example D-26 (0.040 g), THF (1 mL) and water (0.024 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in ethyl acetate/n-hexane (1/1), and insoluble substances were collected by filtration. The obtained solid was dried under reduced pressure to obtain a title compound (0.026 g).

### Example 64

### ((3R)-7-chloro-8-((2-fluorophenyl)methyl)-1-methyl-2-oxo-1,2,3,4-tetrahydroquinolin-3-yl)urea

Potassium cyanate (0.037 g), water (0.110 mL) and acetic acid (0.026 g) were added to a mixture of Reference Example D-28 (0.097 g), THF (0.9 mL) and methanol (0.6 mL) and the mixture was stirred at room temperature for 17.5 hours. Water was added to the reaction mixture, and insoluble substances were collected by filtration. The obtained solid was suspended in MTBE, and the solid was collected by filtration. The obtained solid was dried under reduced pressure to obtain a title compound (0.073 g).

### Example 65 to Example 67

Example 65 to Example 67 were synthesized in the same method as in Example 64 except that corresponding raw materials were used in place of Reference Example D-28.

### Example 68

### ((3R)-8-((5-fluoro-2-methylphenyl)methyl)-1-methyl-2-oxo-1,2,3,4-tetrahydroquinolin-3-yl)urea

Potassium cyanate (0.011 g) and methanesulfonic acid (0.010 g) were added to a mixture of Reference Example D-36 (0.030 g), THF (0.3 mL) and water (0.018 mL) and the mixture was stirred at room temperature for 1 hour. A saturated sodium bicarbonate aqueous solution, water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.026 g).

### Example 69

### ((3R)-7-chloro-8-(2,5-difluorophenoxy)-1-methyl-2-oxo-1,2,3,4-tetrahydroquinolin-3-yl)urea

Potassium cyanate (0.030 g), water (0.089 g) and acetic acid (0.021 g) were added to a mixture of Reference Example F-2 (0.084 g), THF (0.48 mL) and methanol (0.32 mL) and the mixture was stirred at room temperature for 2 hours. Water and DCM were added to the reaction mixture. The mixture was extracted with DCM, and the extract was concentrated under reduced pressure. The residue was purified by APS column chromatography (elution solvent: DCM/methanol=100/0 to 95/5) to obtain a title compound (0.013 g).

### Example 70

### ((3R)-8-(2-(difluoromethyl)-5-fluorophenoxy)-1,7-dimethyl-2-oxo-1,2,3,4-tetrahydroquinolin-3-yl)urea

Potassium cyanate (0.002 g), water (0.005 g) and acetic acid (0.001 g) were added to a mixture of Reference Example F-4 (0.005 g), THF (0.3 mL) and methanol (0.2 mL) and the mixture was stirred at room temperature for 2 hours. Water and DCM were added to the reaction mixture. The mixture was extracted with DCM, and the extract was concentrated under reduced pressure. The residue was purified by APS column chromatography (elution solvent: DCM/methanol=100/0 to 90/10) to obtain a title compound (0.002 g).

### Example 71

### ((3R)-8-(2-chloro-5-fluorophenoxy)-7-(hydroxymethyl)-1-methyl-2-oxo-1,2,3,4-tetrahydroquinolin-3-yl)urea

Potassium cyanate (0.081 g), water (0.139 g) and acetic acid (0.049 g) were added to a mixture of Reference Example F-6 (0.270 g) and THF (3.0 mL) and the mixture was stirred at room temperature for 21 hours. A saturated sodium bicarbonate aqueous solution, water and DCM were added to the reaction mixture. The mixture was extracted with DCM, and the extract was concentrated under reduced pressure. The residue was purified by APS column chromatography (elution solvent: n-hexane/ethyl acetate/methanol=49/49/2 to 0/98/2 to 0/78/22) to obtain a title compound (0.210 g).

### Example 72

### ((3R)-8-(2-chloro-5-fluorophenoxy)-7-(difluoromethyl)-1-methyl-2-oxo-1,2,3,4-tetrahydroquinolin-3-yl)urea

Potassium cyanate (0.014 g), water (0.024 g) and acetic acid (0.009 g) were added to a mixture of Reference Example F-8 (0.050 g) and THF (1 mL) and the mixture was stirred at room temperature for 2 hours. A saturated sodium bicarbonate aqueous solution, water and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.035 g).

### Example 73

### ((3R)-8-(2-chloro-5-fluorophenoxy)-7-(fluoromethyl)-1-methyl-2-oxo-1,2,3,4-tetrahydroquinolin-3-yl)urea

Potassium cyanate (0.030 g), water (0.051 g) and acetic acid (0.018 g) were added to a mixture of Reference Example F-10 (0.100 g) and THF (1 mL) and the mixture was stirred at room temperature for 12 hours. A saturated sodium bicarbonate aqueous solution, water, methanol and DCM were added to the reaction mixture. The mixture was extracted with DCM, and the extract was concentrated under reduced pressure. The residue was suspended in MTBE, and insoluble substances were collected by filtration. The obtained solid was dried under reduced pressure to obtain a title compound (0.090 g).

### Example 74

### ((3R)-8-(2-chloro-5-fluorophenoxy)-1-methyl-2-oxo-7-(trifluoromethyl)-1,2,3,4-tetrahydroquinolin-3-yl)urea

Potassium cyanate (0.109 g), water (0.371 g) and acetic acid (0.068 g) were added to a mixture of Reference Example F-12 (0.400 g), methanol (1.2 mL) and THF (1.8 mL) and the mixture was stirred at room temperature for 3 hours. Water and DCM were added to the reaction mixture. The mixture was extracted with DCM, and the extract was concentrated under reduced pressure. The residue was purified by APS column chromatography (elution solvent: n-hexane/ethyl acetate/methanol=49/49/2 to 0/98/2 to 0/86/14) to obtain a title compound (0.390 g).

### Example 75

### ((3R)-8-(2-chloro-5-fluorophenoxy)-7-(1,1-difluoroethyl)-1-methyl-2-oxo-1,2,3,4-tetrahydroquinolin-3-yl)urea

Potassium cyanate (0.069 g), water (0.234 g) and acetic acid (0.043 g) were added to a mixture of Reference Example F-14 (0.250 g), methanol (0.75 mL) and THF (1.1 mL) and the mixture was stirred at room temperature for 2 hours. Water and DCM were added to the reaction mixture. The mixture was extracted with DCM, and the extract was concentrated under reduced pressure. The residue was purified by APS column chromatography (elution solvent: n-hexane/ethyl acetate/methanol=49/49/2 to 0/98/2 to 0/86/14) to obtain a title compound (0.200 g).

### Example 76

### ((3R)-8-(2-chloro-5-fluorophenoxy)-7-ethyl-1-methyl-2-oxo-1,2,3,4-tetrahydroquinolin-3-yl)urea

Potassium cyanate (0.145 g), water (0.496 g) and acetic acid (0.091 g) were added to a mixture of Reference Example F-16 (0.480 g), methanol (1.4 mL) and THF (2.2 mL) and the mixture was stirred at room temperature for 2 hours. Water was added to the reaction mixture, and insoluble substances were collected by filtration. The obtained solid was washed with water and MTBE and then dried under reduced pressure to obtain a title compound (0.473 g).

### Example 77

### ((3R)-8-(1-(2-chlorophenyl)ethyl)-1-methyl-2-oxo-1,2,3,4-tetrahydroquinolin-3-yl)urea

Potassium cyanate (0.009 g), water (0.027 g) and acetic acid (0.006 g) were added to a mixture of Reference Example G-2 (0.024 g), THF (0.3 mL) and methanol (0.2 mL) and the mixture was stirred at room temperature for 45 minutes. Water and DCM were added to the reaction mixture. The mixture was extracted with DCM, and the extract was concentrated under reduced pressure. The residue was purified by ODS column chromatography (elution solvent: water/MeCN=70/30 to 10/90) to obtain ((3R)-8-(1-(2-chlorophenyl)ethenyl)-1-methyl-2-oxo-1,2,3,4-tetrahydroquinolin-3-yl)urea (0.015 g).

Platinum(IV) oxide (0.003 g) was added to a mixture of the obtained Compound (0.014 g) and THF (1 mL) and the mixture was stirred at room temperature under a hydrogen atmosphere for 1 hour. Platinum(IV) oxide (0.009 g) was added to the reaction mixture and the mixture was stirred at room temperature under a hydrogen atmosphere for 1 hour. The reaction mixture was filtered through Celite, and the filtrate was concentrated under reduced pressure. The residue was purified by ODS column chromatography (elution solvent: water/MeCN=70/30 to 10/90) to obtain a title compound (0.005 g).

### Example 78

### ((3R)-1-Methyl-2-oxo-8-(1-(2-(trifluoromethyl)phenyl)ethyl)-1,2,3,4-tetrahydroquinolin-3-yl)urea

Potassium cyanate (0.004 g) and methanesulfonic acid (0.004 g) were added to a mixture of Reference Example G-4 (0.013 g), THF (1 mL) and water (0.007 mL) and the mixture was stirred at room temperature for 10 hours. A saturated sodium bicarbonate aqueous solution, water and DCM were added to the reaction mixture. The mixture was extracted with DCM, and the extract was concentrated under reduced pressure. The residue was purified by APS column chromatography (elution solvent: DCM/methanol=100/0 to 90/10) to obtain ((3R)-1-Methyl-2-oxo-8-(1-(2-(trifluoromethyl)phenyl)ethenyl)-1,2,3,4-tetrahydroquinolin-3-yl)urea (0.012 g).

10% Pd/C (0.001 g) was added to a mixture of the obtained Compound (0.012 g) and methanol (1 mL) and the mixture was stirred at room temperature under a hydrogen atmosphere for 12 hours. The reaction mixture was filtered through Celite, and the filtrate was concentrated under reduced pressure. The residue was purified by APS column chromatography (elution solvent: DCM/methanol=100/0 to 95/5) and then ODS column chromatography (elution solvent: water/MeCN=70/30 to 10/90) to obtain a title compound (0.005 g).

### Example 79

### ((3R)-8-(1-(2-chloro-5-fluorophenyl)ethyl)-1-methyl-2-oxo-1,2,3,4-tetrahydroquinolin-3-yl)urea

Potassium cyanate (0.012 g) and methanesulfonic acid (0.012 g) were added to a mixture of Reference Example G-6 (0.038 g), THF (0.3 mL) and water (0.021 g) and the mixture was stirred at room temperature for 1 hour. A saturated sodium bicarbonate aqueous solution, water and DCM were added to the reaction mixture and the mixture was stirred at room temperature for 10 minutes. The mixture was extracted with DCM, and the extract was concentrated under reduced pressure. 10% Pt/C (0.010 g) was added to a mixture of the residue, methanol (0.5 mL) and THF (0.5 mL) and the mixture was stirred at room temperature under a hydrogen atmosphere for 1 hour. The reaction mixture was filtered through Celite, and the filtrate was concentrated under reduced pressure. The residue was purified by ODS column chromatography (elution solvent: water/MeCN=70/30 to 10/90) to obtain a title compound (0.005 g).

### Example 80

### 1-((3R)-8-(2-chloro-5-fluorophenoxy)-1,7-dimethyl-2-oxo-1,2,3,4-tetrahydroquinolin-3-yl)-3-methylurea

DIPEA (0.069 g) was added to a mixture of Reference Example B-58 (0.036 g), carbonyldiimidazole (0.017 g) and DCM (1 mL) and the mixture was stirred at room temperature for 1 hour. Methylamine (2 mol/L in THF) (0.11 mL) was added to the reaction mixture and the mixture was stirred at room temperature for 3 hours. Water and DCM were added to the reaction mixture and the mixture was stirred at room temperature for 5 minutes. The mixture was extracted with DCM, and the extract was concentrated under reduced pressure. The residue was purified by silica gel column chromatography (elution solvent: n-hexane/ethyl acetate/methanol=30/70/0 to 0/100/0 to 0/70/30) to obtain a title compound (0.026 g).

### Example 81

### N-((3R)-8-(2-chloro-5-fluorophenoxy)-1,7-dimethyl-2-oxo-1,2,3,4-tetrahydroquinolin-3-yl)acetamide

Acetyl chloride (0.030 g) was added to a mixture of Reference Example B-58 (0.100 g), DIPEA (0.077 g) and DCM (1 mL) under water cooling and the mixture was stirred at the same temperature for 1 hour. Water was added to the reaction mixture and the mixture was stirred at room temperature for 5 minutes. The mixture was extracted with DCM, and the extract was concentrated under reduced pressure. The residue was purified by silica gel column chromatography (elution solvent: n-hexane/ethyl acetate/methanol=30/70/0 to 0/100/0 to 0/70/30) to obtain a title compound (0.079 g).

### Example 82

Example 82 was obtained in the same method as in Example 81 except that cyclopropanecarbonyl chloride was used in place of acetyl chloride.

### Example 83

Example 83 was obtained in the same method as in Example 81 except that dichloroacetyl chloride was used in place of acetyl chloride.

### Example 84

Example 84 was obtained in the same method as in Example 81 except that Reference Example B-14 was used in place of Reference Example B-58 and cyclopropanecarbonyl chloride was used in place of acetyl chloride.

### Example 85

### N-((3R)-8-(2-chloro-5-fluorophenoxy)-1,7-dimethyl-2-oxo-1,2,3,4-tetrahydroquinolin-3-yl)-3,3,3-trifluoropropanamide

DIPEA (0.046 g) was added to a mixture of Reference Example B-58 (0.100 g), 3,3,3-trifluoropropionic acid (0.042 g), HATU (0.125 g) and MeCN (1 mL), and the mixture was stirred at room temperature for 1 hour. Water and DCM were added to the reaction mixture and the mixture was stirred at room temperature for 5 minutes. The mixture was extracted with DCM, and the extract was concentrated under reduced pressure. The residue was purified by silica gel column chromatography (elution solvent: n-hexane/ethyl acetate/methanol=20/80/0 to 0/100/0 to 0/70/30), and then ODS column chromatography (elution solvent: water/MeCN=90/10 to 10/90) to obtain a title compound (0.074 g).

The following tables show the structural formulae, physical property values, and TSHR antagonist activity (refer to Test Example 1) of examples.

**[Table 13]**

| Ex. No. | Str. | Phys. data | IC₅₀ |
|---|---|---|---|
| 1 | 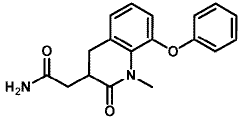 | MS m/z : 311 (M+H)⁺ | C |
| 2 | 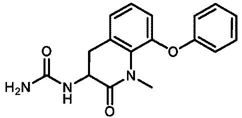 | MS m/z : 312 (M+H)⁺ | B |
| 3 | 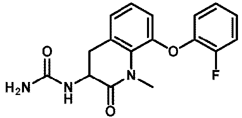 | MS m/z : 330 (M+H)⁺ | B |
| 4 | 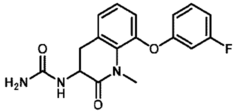 | MS m/z : 330 (M+H)⁺ | C |
| 5 | 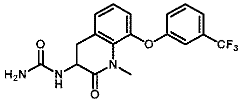 | MS m/z : 380 (M+H)⁺ | B |
| 6 | 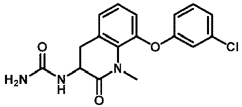 | MS m/z : 346 (M+H)⁺ | B |

**[Table 14]**

| Ex. No. | Str. | Phys. data | IC₅₀ |
|---|---|---|---|
| 7 | 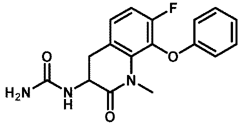 | MS m/z : 330 (M+H)⁺ | B |
| 8 | 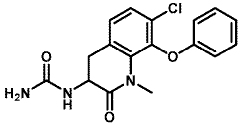 | MS m/z : 346 (M+H)⁺ | B |
| 9 | 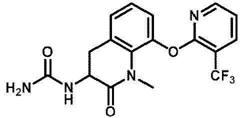 | MS m/z : 381 (M+H)⁺ | C |
| 10 | 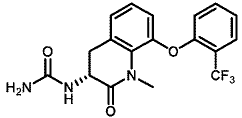 | ¹H-NMR (DMSO-d6) δ ppm : 2. 75-2. 91 (1H, m), 3. 15 (1H, dd, J=5. 1, 14.8Hz), 3.22 (3H, s), 4. 24-4. 34 (1H, m), 5.85 (2H, s), 6.39 (1H, d, J=6. 1Hz), 6.87-6.96(2H, m), 7.11-7.33 (3H, m), 7.57-7.66 (1H, m), 7.75-7.85 (1H, m) MS m/z : 380 (M+H)⁺ | A |
| 11 | 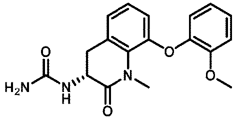 | MS m/z : 342 (M+H)⁺ | C |

**[Table 15]**

| Ex. No. | Str. | Phys. data | IC₅₀ |
|---|---|---|---|
| 12 | 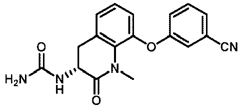 | MS m/z : 337 (M+H)⁺ | C |
| 13 | 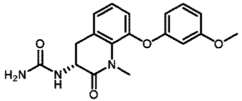 | MS m/z : 342 (M+H)⁺ | B |
| 14 | 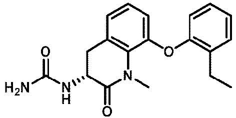 | MS m/z : 340 (M+H)⁺ | B |
| 15 | 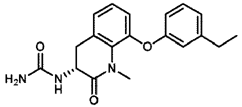 | MS m/z : 340 (M+H)⁺ | B |
| 16 | 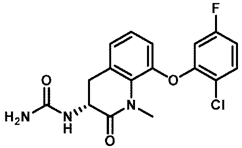 | ¹H-NMR (DMSO-d6) δ ppm : 2.76-2.88 (1H, m), 3.11 (1H, dd, J=5.3, 14. 8Hz), 3.27 (3H, s), 4. 19-4. 30 (1H, m), 5. 84 (2H, s), 6.41 (1H, d, J=6.5Hz), 6.77-6.86 (1H, m), 6.89-6.96 (1H, m), 7. 03-7. 09 (1H, m), 7. 12-7. 22 (2H, m), 7. 62-7. 70 (1H, m) | A |
| | | MS m/z : 364 (M+H)⁺ | |
| 17 | 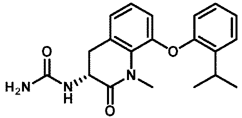 | MS m/z : 354 (M+H)⁺ | B |

**[Table 16]**

| Ex. No. | Str. | Phys. data | IC₅₀ |
|---|---|---|---|
| 18 | 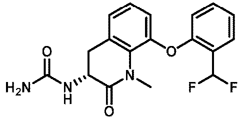 | MS m/z : 362 (M+H)⁺ | A |
| 19 | 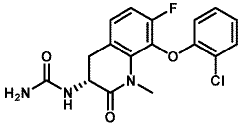 | ¹H-NMR (DMSO-d6) δ ppm : 2. 74-2. 87 (1H, m), 3. 12 (1H, dd, J=5. 3, 14. 9Hz), 3. 21 (3H, s), 4.23-4.34 (1H, m), 5.83 (2H, s), 6.37-6.45 (1H, m), 6. 67-6. 74 (1H, m), 7.08-7.33 (4H, m), 7.55-7.62 (1H, m) | A |
| | | MS m/z : 364 (M+H)⁺ | |
| 20 | 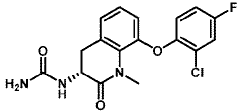 | MS m/z : 364 (M+H)⁺ | A |
| 21 | 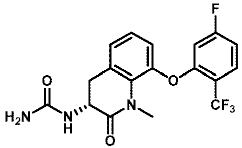 | ¹H-NMR (DMSO-d6) δ ppm : 2.75-2.89 (1H, m), 3.15 (1H, dd, J=5.3, 14.8Hz), 3.23 (3H, s), 4.29⁻4.41 (1H, m), 5.83 (2H, s), 6.38 (1H, d, J=6. 2Hz), 6.84-6.91 (1H, m), 6.95-7.01 (1H, m), 7.11-7.20 (2H, m), 7.24 (1H, d, J=7.2Hz), 7.84-7.92 (1H, m) | A |
| | | MS m/z : 398 (M+H)⁺ | |
| 22 | 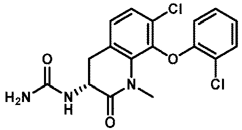 | ¹H-NMR (DMSO-d6) δ ppm : 2. 76-2. 88 (1H, m), 3.16 (1H, dd, J=5.3, 14.9Hz), 3.20 (3H, s), 4.22-4.34 (1H, m), 5.84 (2H, s), 6.36 (1H, d, J=6. 2Hz), 6.45-6.52 (1H, m), 7.04-7.11 (1H, m), 7.17-7.25 (1H, m), 7.33 (1H, d, J=8.1Hz), 7.40 (1H, d, J=8. 1Hz), 7.55-7.62 (1H, m) | A |
| | | MS m/z : 380 (M+H)⁺ | |
| 23 | 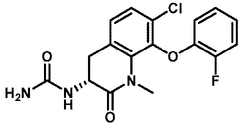 | ¹H-NMR (DMSO-d6) δ ppm : 2.74-2.89 (1H, m), 3.15 (1H, dd, J=5.2, 14.9Hz), 3.21 (3H, s), 4. 23-4.33 (1H, m), 5.84 (2H, s), 6.37 (1H, d, J=6. 3Hz), 6. 50-6.59 (1H, m), 7. 00-7. 11 (2H, m), 7. 29-7.45 (3H, m) | A |
| | | MS m/z : 364 (M+H)⁺ | |

**[Table 17]**

| Ex. No. | Str. | Phys. data | IC₅₀ |
|---|---|---|---|
| 24 | 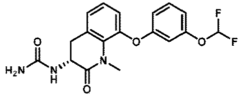 | MS m/z : 378 (M+H)⁺ | B |
| 25 | 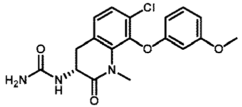 | MS m/z : 376 (M+H)⁺ | B |
| 26 | 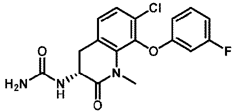 | ¹H-NMR (DMSO-d6) δ ppm : 2.75-2.88 (1H, m), 3.14 (1H, dd, J=5.3, 14.9Hz), 3.20 (3H, s), 4. 21-4. 34 (1H, m), 5.83 (2H, s), 6.36 (1H, d, J=6. 4Hz), 6.54-6.63 (1H, m), 6. 73-6. 81 (1H, m), 6.87-6.96 (1H, m), 7.28-7.43 (3H, m) | A |
| | | MS m/z : 364 (M+H)⁺ | |
| 27 | 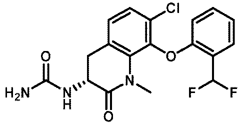 | ¹H-NMR (DMSO-d6) δ ppm : 2. 75-2. 90 (1H, m), 3. 12-3. 23 (4H, m), 4. 26-4. 38 (1H, m), 5.85 (2H, s), 6.37 (1H, d, J=6.2Hz), 6.40-6.60 (1H, br), 7. 16-7. 54 (5H, m), 7.64-7.72 (1H, m) | A |
| | | MS m/z : 396 (M+H)⁺ | |
| 28 | 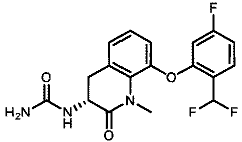 | ¹H-NMR (DMSO-d6) δ ppm : 2. 75-2. 89 (1H, m), 3.14 (1H, dd, J=5.3, 15.0Hz), 3.23 (3H, s), 4. 23-4. 35 (1H, m), 5.83 (2H, s), 6. 39 (1H, d, J=6. 3Hz), 6. 66-6.75 (1H, m), 6.94-7.03 (1H, m), 7.05-7.50 (4H, m), 7.66-7.80 (1H, m) | A |
| | | MS m/z : 380 (M+H)⁺ | |
| 29 | 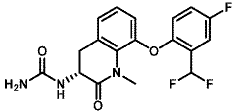 | MS m/z : 380 (M+H)⁺ | B |

**[Table 18]**

| Ex. No. | Str. | Phys. data | IC₅₀ |
|---|---|---|---|
| 30 | 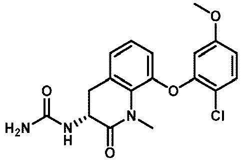 | ¹H-NMR (DMSO-d6) δ ppm : 2. 76-2. 89 (1H, m), 3. 10 (1H, dd, J=5. 1, 14. 7Hz), 3. 30 (3H, s), 3. 68 (3H, s), 4. 11-4. 25 (1H, m) , 5. 84 (2H, s), 6. 40 (1H, d, J=6.4Hz), 6. 44-6. 51 (1H, m), 6.74-6.88 (2H, m), 7. 08-7. 19 (2H, m) , 7.46-7.58 (1H, m) | A |
| | | MS m/z : 376 (M+H)⁺ | |
| 31 | 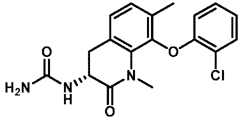 | ¹H-NMR (DMSO-d6) δ ppm : 2.05 (3H, s), 2.71-2.84 (1H, m), 3. 11 (1H, dd, J=5.2, 14. 9Hz), 3.18 (3H, s), 4. 14-4. 28 (1H, m), 5.84 (2H, s), 6.31-6.41 (2H, m), 7.01-7.23 (4H, m), 7.54-7.64 (1H, m) | A |
| | | MS m/z : 360 (M+H)⁺ | |
| 32 | 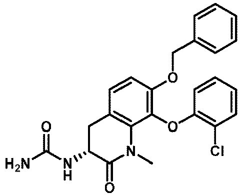 | MS m/z : 452 (M+H)⁺ | C |
| 33 | 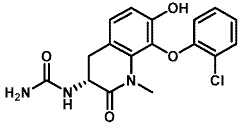 | MS m/z : 362 (M+H)⁺ | B |
| 34 | 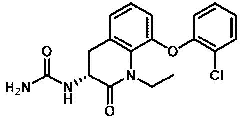 | ¹H-NMR (DMSO-d6) δ ppm : 1. 08-1. 14 (3H, m), 2. 72-2.83 (1H, m), 3.06-3. 14 (1H, m), 3. 81-3. 97 (2H, m), 4. 15-4. 25 (1H, m), 5.84 (2H, s), 6.41 (1H, d, J=6. 2Hz), 6.70-6.82 (1H, m), 6.87-7.00 (1H, m), 7.04-7.24 (3H, m), 7.27-7.37 (1H, m), 7.54-7.68 (1H, m) | A |
| | | MS m/z : 360 (M+H)⁺ | |
| 35 | 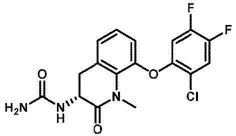 | ¹H-NMR (DMSO-d6) δ ppm : 2.74-2.89 (1H, m), 3.12 (1H, dd, J=5.2, 14.7Hz), 3.30 (3H, s), 4.21-4.33 (1H, m), 5.84 (2H, s), 6.39 (1H, d, J=6. 3Hz), 6.80-6.89 (1H, m), 7.07-7. 19 (2H, m), 7.24-7.36 (1H, m), 7.89-8.00 (1H, m) | A |
| | | MS m/z : 382 (M+H)⁺ | |

**[Table 19]**

| Ex. No. | Str. | Phys. data | IC₅₀ |
|---|---|---|---|
| 36 | 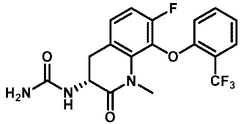 | MS m/z : 398 (M+H)⁺ | B |
| 37 | 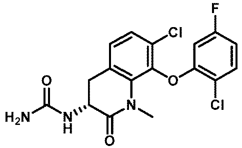 | ¹H-NMR (DMSO-d6) δ ppm : 2. 75-2. 88 (1H, m), 3.16 (1H, dd, J=5.2, 14.8Hz), 3.20 (3H, s), 4. 33-4. 46 (1H, m), 5.82 (2H, s), 6.36 (1H, d, J=6. 5Hz), 6.45-6.58 (1H, m), 6.93-7.05 (1H, m), 7.34 (1H, d, J=8.2Hz), 7.40 (1H, d, J=8.2Hz), 7. 61-7. 69 (1H, m) | A |
| | | MS m/z : 398 (M+H)⁺ | |
| 38 | 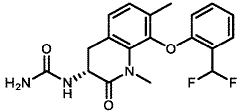 | ¹H-NMR (DMSO-d6) δ ppm : 2.04 (3H, s), 2.70-2.84 (1H, m), 3.08-3. 19 (4H, m), 4. 18-4. 29 (1H, m), 5.84 (2H, s), 6.22-6.49 (2H, m), 7.08-7.22 (3H, m), 7.25-7. 56 (2H, m), 7. 61-7. 70 (1H, m) | A |
| | | MS m/z : 376 (M+H)⁺ | |
| 39 | 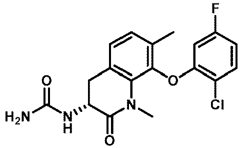 | ¹H-NMR (DMSO-d6) δ ppm : 2.05 (3H, s), 2.70-2.85 (1H, m), 3.06-3.22 (4H, m), 4. 21-4. 34 (1H, m), 5.82 (2H, s), 6.16-6.44 (2H, m), 6.87-7.04 (1H, m), 7.10-7.26 (2H, m), 7. 61-7. 71 (1H, m) | A |
| | | MS m/z : 378 (M+H)⁺ | |
| 40 | 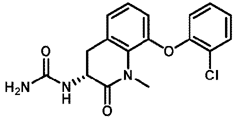 | ¹H-NMR (DMSO-d6) δ ppm : 2. 75-2. 88 (1H, m), 3.12 (1H, dd, J=5.2, 14.8Hz), 3.28 (3H, s), 4. 16-4. 29 (1H, m), 5.84 (2H, s), 6.39 (1H, d, J=6. 2Hz), 6.79-6.97 (2H, m), 7.08-7.22 (3H, m), 7.28-7.36 (1H, m), 7.57-7.65 (1H, m) | A |
| | | MS m/z : 346 (M+H)⁺ | |
| 41 | 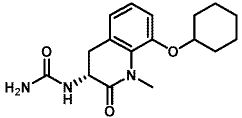 | MS m/z : 318 (M+H)⁺ | B |

**[Table 20]**

| Ex. No. | Str. | Phys. data | IC₅₀ |
|---|---|---|---|
| 42 | 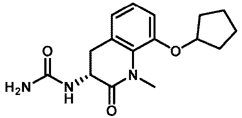 | MS m/z : 304 (M+H)⁺ | B |
| 43 | 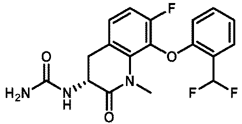 | MS m/z : 380 (M+H)⁺ | B |
| 44 | 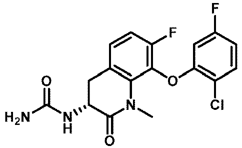 | ¹H-NMR (DMSO-d6) δ ppm : 2. 71-2. 86 (1H, m), 3. 10-3. 18 (1H, m), 3.22 (3H, s), 4. 32-4. 42 (1H, m), 5.82 (2H, s), 6. 36 (1H, d, J=6. 9Hz), 6. 71-6. 77 (1H, m), 6.96-7.06 (1H, m), 7.13⁻7.22 (1H, m), 7.28-7.35 (1H, m), 7.61-7.69 (1H, m) | A |
| | | MS m/z : 382 (M+H)⁺ | |
| 45 | 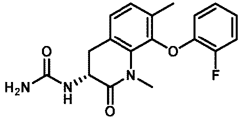 | ¹H-NMR (DMSO-d6) δ ppm : 2.08 (3H, s), 2.69-2.82 (1H, m), 3.08 (1H, dd, J=5. 3, 14. 7Hz), 3.19 (3H, s), 4.10-4.23 (1H, m), 5. 83 (2H, br), 6. 35 (1H, d, J=6. 3Hz), 6.49-6.57 (1H, m), 6. 63-6. 72 (1H, m), 6.83-6.92 (1H, m), 7.09-7.21 (2H, m)7.30-7.39 (1H, m) | A |
| | | MS m/z : 344 (M+H)⁺ | |
| 46 | 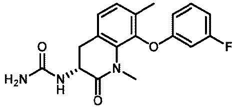 | ¹H-NMR (DMSO-d6) δ ppm : 2.09 (3H, s), 2.70-2.82 (1H, m), 3.06-3.13 (1H, m), 3.19 (3H, s), 4.12-4.25 (1H, m), 5.83 (2H, br), 6.31-6.45 (2H, m), 6.97-7.07 (2H, m), 7.09-7.21 (2H, m), 7.33-7.42 (1H, m) | A |
| | | MS m/z : 344 (M+H)⁺ | |
| 47 | 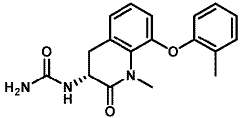 | ¹H-NMR (DMSO-d6) δ ppm : 2.27 (3H, s), 2.75-2.86 (1H, m), 3. 11 (1H, dd, J=5.4, 14.8 Hz), 3.29 (3H, s), 4.14-4.26 (1H, m), 5.84 (2H, s), 6.39 (1H, d, J=6. 3Hz), 6.66-6.80 (2H, m), 7.00-7.20 (4H, m), 7.29-7.35 (1H, m) | A |
| | | MS m/z : 326 (M+H)⁺ | |

**[Table 21]**

| Ex. No. | Str. | Phys. data | IC₅₀ |
|---|---|---|---|
| 48 | 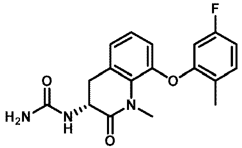 | ¹H-NMR (DMSO-d6) δ ppm : 2.25 (3H, s), 2. 75-2. 88 (1H, m), 3. 11 (1H, dd, J=5.2, 14. 8Hz), 3. 27 (3H, s), 4.16-4.27 (1H, m), 5.83 (2H, s), 6. 39 (1H, d, J=6. 3Hz), 6.50-6.58 (1H, m), 6.80-6.93 (2H, m), 7.08-7.18 (2H, m), 7.30-7.39 (1H, m) | A |
| | | MS m/z : 344 (M+H)⁺ | |
| 49 | 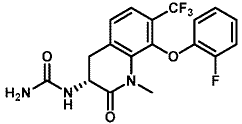 | ¹H-NMR (DMSO-d6) δ ppm : 2.85-2.99 (1H, m), 3.13 (3H, s), 3.25 (1H, dd, J=5. 3, 15. 0Hz), 4. 26-4. 36 (1H, m), 5. 86 (2H, s), 6.37 (1H, d, J=6.3Hz), 6.49-6. 56 (1H, m), 6. 99-7. 10 (2H, m), 7. 34-7.43 (1H, m), 7.51 (1H, d, J=7. 8Hz), 7.63 (1H, d, J=7.8Hz) | A |
| | | MS m/z : 398 (M+H)⁺ | |
| 50 | 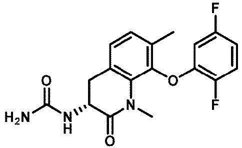 | ¹H-NMR (CDCl₃) δ ppm : 2. 17 (3H, s), 2.68-2.80 (1H, m), 3. 29-3. 49 (4H, m), 4. 24-4. 36 (1H, m), 4.54 (2H, s), 5. 78-5.94 (1H, m), 5. 95-6. 05 (1H, m), 6. 58-6.71 (1H, m), 6.98-7.18 (3H, m) | A |
| | | MS m/z : 362 (M+H)⁺ | |
| 51 | 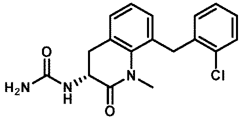 | ¹H-NMR (DMSO-d6) δ ppm : 2.68-2. 79 (1H, m), 3.01 (1H, dd, J=5.1, 14. 7Hz), 3.22 (3H, s), 4.05-4.16 (3H, m), 5. 85 (2H, s), 6.40 (1H, d, J=6. 3Hz), 6.82-6.88 (1H, m), 7.00-7.07 (1H, m), 7.13-7.21 (2H, m), 7.25-7.34 (2H, m), 7.42-7.49 (1H, m) | A |
| | | MS m/z : 344 (M+H)⁺ | |
| 52 | 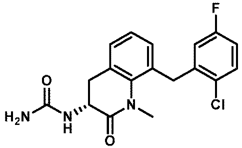 | ¹H-NMR (CDCl₃) δ ppm : 2.69-2.81 (1H, m), 3.28 (3H, s), 3.37 (1H, dd, J=5. 2, 14. 4Hz), 4. 00-4. 19 (2H, m), 4. 27-4. 38 (1H, m), 4.49 (2H, s), 5.77-5.96 (1H, m), 6. 70-6. 77 (1H, m), 6. 84-6. 99 (2H, m), 7.04-7.12 (1H, m), 7.14-7.20 (1H, m), 7.32-7.39 (1H, m) | A |
| | | MS m/z : 362 (M+H)⁺ | |
| 53 | 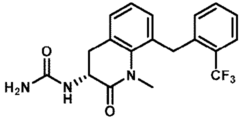 | ¹H-NMR (CDCl₃) δ ppm : 2. 70-2. 83 (1H, m), 3. 26 (3H, s), 3.35 (1H, dd, J=5.2, 14. 8Hz), 4.17-4.42 (3H, m), 4. 62 (2H, s), 5.91-6.11 (1H, m), 6.90-7.02 (2H, m), 7.03-7.10 (1H, m), 7.14-7.20 (1H, m), 7.30-7.38 (1H, m), 7.39-7.46 (1H, m), 7.66-7.72 (1H, m) | A |
| | | MS m/z : 378 (M+H)⁺ | |

**[Table 22]**

| Ex. No. | Str. | Phys. data | IC₅₀ |
|---|---|---|---|
| 54 | 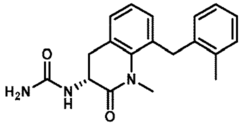 | ¹H-NMR (CDCl₃) δ ppm : 2.12 (3H, s), 2.70-2.82 (1H, m), 3. 25-3. 40 (4H, m), 3.87-4.03 (2H, m), 4. 30-4. 40 (1H, m), 4.56 (2H, s), 5.91-6.04 (1H, m), 6. 83-6.90 (1H, m), 6.97-7.05 (2H, m), 7.08-7. 23 (4H, m) | A |
| | | MS m/z : 324 (M+H) | |
| 55 | 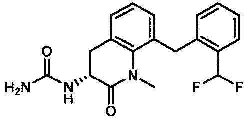 | MS m/z : 360 (M+H)⁺ | A |
| 56 | 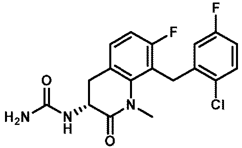 | ¹H-NMR (CDCl₃) δ ppm : 2.60-2.80 (1H, m), 3. 14 (3H, s), 3. 30-3. 40 (1H, m), 3.95-4. 20 (2H, m), 4.25-4.40 (1H, m), 4.64 (2H, s), 5.98 (1H, d, J=4. 6Hz), 6. 60-6. 70 (1H, m), 6.85-7.00 (2H, m), 7. 15-7. 25 (1H, m), 7.30-7.40 (1H, m) | A |
| | | MS m/z : 380 (M+H)⁺ | |
| 57 | 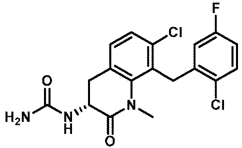 | ¹H-NMR (CDCl₃) δ ppm : 2.65-2.76 (1H, m), 3. 10 (3H, s), 3.37 (1H, dd, J=5.2, 14. 8Hz), 4. 05-4. 24 (2H, m), 4. 26-4. 35 (1H, m), 4. 50 (2H, s), 5. 73-5.92 (1H, m), 6. 53-6. 61 (1H, m), 6. 86-6. 96 (1H, m), 7. 20 (1H, d, J=8.0Hz), 7. 28 (1H, d, J=8.0Hz), 7. 35-7. 43 (1H, m) | A |
| | | MS m/z : 396 (M+H)⁺ | |
| 58 | 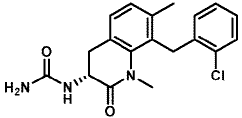 | ¹H-NMR (DMSO-d6) δ ppm : 2.08 (3H, s), 2.63-2.75 (1H, m), 2.95-3.06 (4H, m), 3.96-4.15 (3H, m), 5.83 (2H, s), 6.36 (1H, d, J=6. 1Hz), 6.73-6.81 (1H, m), 7.06 (1H, d, J=7. 5Hz), 7.18 (1H, d, J= 7.5Hz), 7.21-7.31 (2H, m), 7.48-7.55 (1H, m) | A |
| | | MS m/z : 358 (M+H)⁺ | |
| 59 | 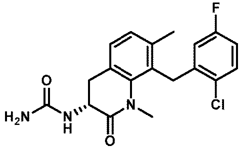 | ¹H-NMR (DMSO-d6) δ ppm : 2.09(3H, s), 2.64-2.76 (1H, m), 2.94-3.04 (4H, m), 3.96⁻4.15 (3H, m), 5.83 (2H, s), 6.37 (1H, d, J=6. 3Hz), 6.45-6.53 (1H, m), 7.08 (1H, d, J=7. 7Hz), 7.12-7.23 (2H, m), 7.54-7.62 (1H, m) | A |
| | | MS m/z : 376 (M+H)⁺ | |

**[Table 23]**

| Ex. No. | Str. | Phys. data | IC₅₀ |
|---|---|---|---|
| 60 | 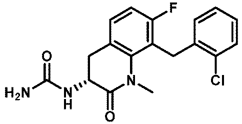 | ¹H-NMR (DMSO-d6) δ ppm : 2. 60-2. 80 (1H, m), 3. 00-3. 15 (4H, m), 4. 00-4. 20 (3H, m), 5.85 (2H, s), 6.38 (1H, d, J=6. 4Hz), 6.90-7.10 (2H, m), 7.20-7.40 (3H, m), 7.45-7.55 (1H, m) | A |
| | | MS m/z : 362 (M+H)⁺ | |
| 61 | 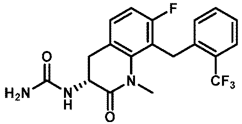 | ¹H-NMR (DMSO-d6) δ ppm : 2. 65-2. 80 (1H, m), 3. 00-3. 10 (4H, m), 4. 10-4. 30 (3H, m), 5.84 (2H, s), 6.38 (1H, d, J=6. 3Hz), 6.95-7.10 (2H, m), 7. 30-7. 40 (1H, m), 7.40-7.50 (1H, m), 7. 55-7. 65 (1H, m), 7.75-7.81 (1H, m) | A |
| | | MS m/z : 396 (M+H)⁺ | |
| 62 | 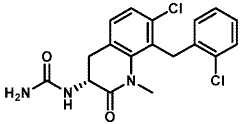 | ¹H-NMR (DMSO-d6) δ ppm : 2.65-2.80 (1H, m), 2.95-3.10 (4H, m), 4.05-4.25 (3H, m), 5.85 (2H, s), 6.38 (1H, d, J=6. 3Hz), 6.75-6.85 (1H, m), 7. 20-7. 38 (4H, m), 7.45-7.55 (1H, m) | A |
| | | MS m/z : 378 (M+H)⁺ | |
| 63 | 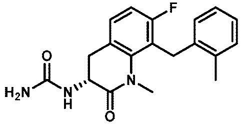 | ¹H-NMR (DMSO-d6) δ ppm : 2.32 (3H, s), 2. 65-2. 80 (1H, m), 2. 95-3. 10 (4H, m), 3. 85-4. 05 (2H, m), 4. 05-4. 20 (1H, m), 5.84 (2H, s), 6.38 (1H, d, J=6.4Hz), 6.69-6.77 (1H, m), 6.95-7.15 (3H, m), 7.15-7.25 (1H, m), 7.25-7.35 (1H, m) | A |
| | | MS m/z : 342 (M+H) | |
| 64 | 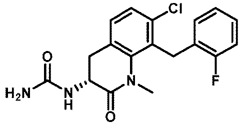 | MS m/z : 362 (M+H)⁺ | B |
| 65 | 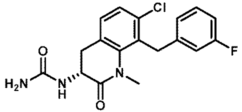 | MS m/z : 362 (M+H)⁺ | B |

**[Table 24]**

| Ex. No. | Str. | Phys. data | IC₅₀ |
|---|---|---|---|
| 66 | 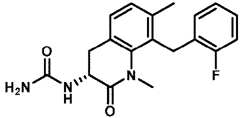 | MS m/z : 340 (M-H)⁻ | A |
| 67 | 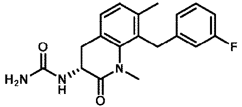 | MS m/z : 342 (M+H)⁺ | C |
| 68 | 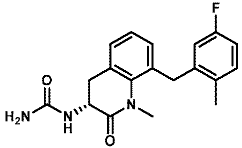 | ¹H-NMR (DMSO-d6) δ ppm : 2.05 (3H, s), 2.69-2.80 (1H, m), 3.01 (1H, dd, J=5.0, 14. 7Hz), 3. 22 (3H, s), 3.93-4. 15 (3H, m), 5. 86 (2H, s), 6. 41 (1H, d, J=6. 1Hz), 6.77-6.86 (2H, m), 6.93-7.08 (2H, m), 7.13-7.24 (2H, m) | A |
| | | MS m/z : 342 (M+H)⁺ | |

**[Table 25]**

| Ex. No. | Str. | Phys. data | IC₅₀ |
|---|---|---|---|
| 69 | 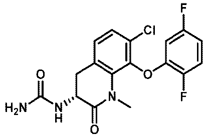 | ¹H-NMR (CDCl₃) δ ppm : 2.69-2.83 (1H, m), 3.37-3.50 (4H, m), 4.34 (1H, ddd, J=5.0, 5.0, 14. 1Hz), 4.68 (2H, br), 6.00 (1H, d, J=4.4Hz), 6.05-6.13 (1H, m), 6.64-6.73 (1H, m), 7.08-7.18 (2H, m), 7.23-7.30 (1H, m) | A |
| | | MS m/z : 382 (M+H) | |
| 70 | 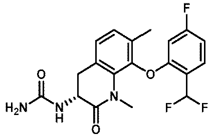 | ¹H-NMR (CDCl₃) δ ppm : 2.10 (3H, s), 2.68-2.81 (1H, m), 3.32 (3H, s), 3.44 (1H, dd, J=5.6, 14. 8Hz), 4.27-4.37 (1H, m), 4.49 (2H, br), 5.73-6.03 (2H, m), 6.74-6.84 (1H, m), 6.90-7.23 (3H, m), 7.55-7.68 (1H, m) | A |
| | | MS m/z : 394 (M+H)⁺ | |
| 71 | 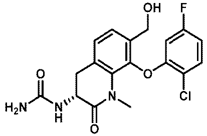 | ¹H-NMR (DMSO-d6) δ ppm : 2.73-2.87 (1H, m), 3.07-3.20 (4H, m), 4.17-4.33 (2H, m), 4. 36-4. 53 (1H, m), 5. 24 (1H, t, J=5.7Hz), 5.83 (2H, s), 6.18-6.29 (1H, m), 6. 36 (1H, d, J=6.6Hz), 6.90-6.99 (1H, m), 7.29 (1H, d, J=7.7Hz), 7.35 (1H, d, J=7. 7Hz), 7.64 (1H, dd, J=5.8, 8.9Hz) | A |
| | | MS m/z : 394 (M+H)⁺ | |
| 72 | 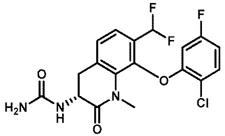 | ¹H-NMR (DMSO-d6) δ ppm : 2.83-2.95 (1H, m), 3. 13 (3H, s), 3. 20 (1H, dd, J=5.2, 15. 0Hz), 4.34-4.44 (1H, m), 5.83 (2H, s), 6.31-6.43 (2H, m), 6.83-7. 15 (2H, m), 7.43-7.52 (2H, m), 7. 64 (1H, dd, J=5.8, 8.9Hz) | A |
| | | MS m/z : 414 (M+H)⁺ | |
| 73 | 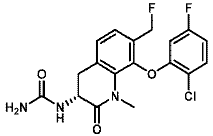 | ¹H-NMR (DMSO-d6) δ ppm : 2. 85 (1H, ddd, J=3.8, 14. 3, 14. 3Hz), 3.10-3.22 (4H, m), 4.29-4.41 (1H, m), 5.24 (1H, dd, J=10.7, 20. 8Hz), 5.36 (1H, dd, J=10. 7, 20. 8Hz), 5.84 (2H, s), 6. 31 (1H, dd, J=2.5, 9. 7Hz), 6.37 (1H, d, J=6. 4Hz), 6.92-7.00 (1H, m), 7.35-7.43 (2H, m), 7.64 (1H, dd, J=5.9, 8. 9Hz) | A |
| | | MS m/z : 396 (M+H) | |
| 74 | 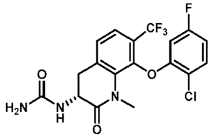 | ¹H-NMR (DMSO-d6) δ ppm : 2. 86-2. 99 (1H, m), 3.11 (3H, s), 3.24 (1H, dd, J=5. 2, 15. 2Hz), 4.40-4.51 (1H, m), 5.83 (2H, s), 6.30-6.43 (1H, m), 6.46-6.59 (1H, m), 6.93-7.02 (1H, m), 7.53 (1H, d, J=7. 9Hz), 7.59-7.69 (2H, m) | A |
| | | MS m/z : 432 (M+H) | |

**[Table 26]**

| Ex. No. | Str. | Phys. data | IC₅₀ |
|---|---|---|---|
| 75 | 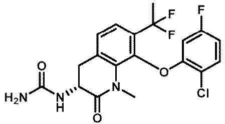 | ¹H-NMR (DMSO-d6) δ ppm : 2. 02 (3H, t, J=18.7Hz), 2. 80-2. 94 (1H, m), 3. 10 (3H, s), 3.14-3.25 (1H, m), 4.31-4.45 (1H, m), 5.85 (2H, s), 6. 26-6. 43 (2H, m), 6.90-7.00 (1H, m), 7.40-7.46 (2H, m), 7.63 (1H, dd, J=5.8, 8.9Hz) | A |
| | | MS m/z : 428 (M+H)⁺ | |
| 76 | 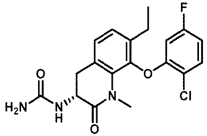 | ¹H-NMR (DMSO-d6) δ ppm : 1.09 (3H, t, J=7.4Hz), 2.42 (2H, q, J=7.4Hz), 2.72-2.84 (1H, m), 3.11 (1H, dd, J=5.3, 14.8Hz), 3.16 (3H, s), 4.23-4.34 (1H, m), 5.82 (2H, s), 6.15-6.30 (1H, m), 6. 35 (1H, d, J=6. 3Hz), 6.90-6.99 (1H, m), 7.17 (1H, d, J=7. 6Hz), 7.25 (1H, d, J=7. 6Hz), 7. 64 (1H, dd, J=5.9, 8.9Hz) | A |
| | | MS m/z : 392 (M+H)⁺ | |
| 77 | 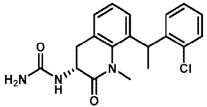 | ¹H-NMR (CDCl₃) δ ppm : 1. 73 (3H, d, J=7. 0Hz), 2.62-2.74 (1H, m), 3.30 (1H, dd, J=5. 2, 14. 8Hz), 3. 46 (3H, s), 4.05-4.15 (1H, m), 4.53 (2H, br), 4.74 (1H, q, J=7.0Hz), 5.93-6.03 (1H, m), 6.98-7.33 (7H, m) | A |
| | | MS m/z : 358 (M+H)⁺ | |
| 78 | 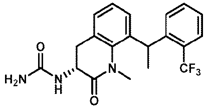 | ¹H-NMR (CDCl₃) δ ppm : 1.47 (3H, d, J=7.0Hz), 2.69-2.82 (1H, m), 3.28 (1H, dd, J=5.1, 14. 7Hz), 3.39 (3H, s), 4. 33-4. 44 (1H, m), 4.69 (2H, s), 4. 92 (1H, q, J=7.0Hz), 6.04-6.17 (1H, m), 6.62 (1H, d, J=7. 8Hz), 6.93-7.00 (1H, m), 7.06-7.13 (1H, m), 7.40-7.47 (1H, m), 7.52-7.66 (2H, m), 7.72-7.78 (1H, m) | A |
| | | MS m/z : 392 (M+H)⁺ | |
| 79 | 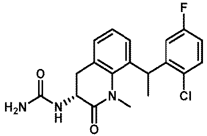 | ¹H-NMR (DMSO-d6) δ ppm : 1. 74 (3H, d, J=7.0Hz), 2. 63-2. 75 (1H, m), 2.93 (1H, dd, J=4.9, 14. 6Hz), 3.40 (3H, s), 3. 90-4. 00 (1H, m), 4. 56 (1H, q, J=7. 0Hz), 5. 85 (2H, s), 6. 40 (1H, d, J=6. 3Hz), 7. 00-7. 45 (6H, m) | A |
| | | MS m/z : 376 (M+H)⁺ | |
| 80 | 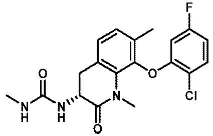 | ¹H-NMR (DMSO-d6) δ ppm : 2.05 (3H, s), 2. 56 (3H, d, J=4. 6Hz), 2.72-2.84 (1H, m), 3.09 (1H, dd, J=5.2, 14.8Hz), 3. 17 (3H, s), 4.24-4.36 (1H, m), 6. 17-6.35 (3H, m), 6.90-7.00 (1H, m), 7.14 (1H, d, J=7. 7Hz), 7.20 (1H, d, J=7.7Hz), 7.65 (1H, dd, J=5.9, 8.9Hz) | A |
| | | MS m/z : 392 (M+H)⁺ | |

**[Table 27]**

| Ex. No. | Str. | Phys. data | IC₅₀ |
|---|---|---|---|
| 81 | 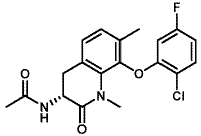 | ¹H-NMR (CDCl₃) δ ppm : 2.08 (3H, s), 2.14 (3H, s), 2.64-2.76 (1H, m), 3.37 (3H, s), 3.45 (1H, dd, J=5.4, 14.7Hz), 4.31-4.41 (1H, m), 5.87-6.00 (1H, m), 6.64-6.76 (2H, m), 7.06 (1H, d, J=7.9Hz), 7.10 (1H, d, J=7. 9Hz), 7.39 (1H, dd, J=5.7, 8.8Hz) | A |
| | | MS m/z : 377 (M+H) | |
| 82 | 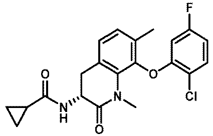 | ¹H-NMR (CDCl₃) δ ppm : 0.72-0.86 (2H, m), 0.92-1.07 (2H, m), 1. 45-1. 55 (1H, m), 2.13 (3H, s), 2.66-2.77 (1H, m), 3.38 (3H, s), 3.45 (1H, dd, J=5.4, 14. 7Hz), 4.32-4.42 (1H, m), 5.87-5.99 (1H, m), 6.64-6.73 (1H, m), 6.85-6.95 (1H, m), 7.05 (1H, d, J=8. 1Hz), 7.08 (1H, d, J=8.1Hz), 7.39 (1H, dd, J=5.7, 8.8Hz) | A |
| | | MS m/z : 403 (M+H)⁺ | |
| 83 | 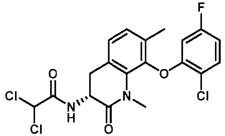 | ¹H-NMR (CDCl₃) δ ppm : 2. 15 (3H, s), 2. 74-2. 86 (1H, m), 3. 40 (3H, s), 3.45-3.53 (1H, m), 4.27-4.39 (1H, m), 5.88-6.01 (2H, m), 6. 64-6. 74 (1H, m), 7.05-7.15 (2H, m), 7.40 (1H, dd, J=5.7, 8.8 Hz), 7.77-7.87 (1H, m) | A |
| | | MS m/z : 443, 445 (M-2, M) | |
| 84 | 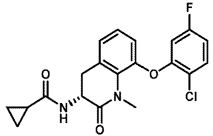 | ¹H-NMR (CDCl₃) δ ppm : 0.74-0.85 (2H, m), 0.90-1.08 (2H, m), 1. 48-1. 66 (1H, m), 2.70-2.81 (1H, m), 3.42-3.52 (4H, m), 4.41 (1H, ddd, J=5.0, 5.0, 14. 1Hz), 6.43 (1H, dd, J=2.8, 9.4Hz), 6.75-6.82 (1H, m), 6.84-6.97 (2H, m), 7.06-7.14 (2H, m), 7.42 (1H, dd, J=5.7, 8.9Hz) | A |
| | | MS m/z : 389 (M+H)⁺ | |
| 85 | 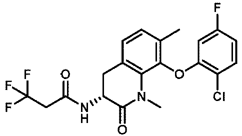 | ¹H-NMR (CDCl₃) δ ppm : 2. 14 (3H, s), 2.68-2.79 (1H, m), 3.16 (2H, q, J=10. 4Hz), 3.38 (3H, s), 3.44-3.54 (1H, m), 4. 32-4. 43 (1H, m), 5.88-6.00 (1H, m), 6.64-6.73 (1H, m), 7.02-7.14 (3H, m), 7.40 (1H, dd, J=5.7, 8.8Hz) | A |
| | | MS m/z : 443 (M-H) | |

### Test Example 1

Measurement of antagonist activity using TSH-induced cAMP production in human TSHR-stably expressing CHO cells as an indicator

The human TSHR gene sequence (reference number NM_000369.2) was inserted into the multicloning site of pcDNA3.1(+). The constructed plasmid vector was introduced into CHO cells using the lipofection method to establish human TSHR-stably expressing CHO cells. The obtained cells were seeded at 5 × 10⁴ cells/well in a 96-well poly-D-lysine-coated plate, and cultured in an F12 culture medium containing 10%FBS, 400 µg/mL of G418, 50 U/mL of penicillin and 50 µg/mL of streptomycin under conditions of 37°C and 5%CO₂ for 1 day. After the culture medium was removed, the cells were washed twice with 100 µL of an assay buffer solution (Hanks' Balanced Salt Solution containing 20 mM HEPES and 1 mM IBMX) per well. 30 µL of a test compound-containing assay buffer solution was added to the well and incubated at room temperature for 15 minutes. Then, 30 µL of an assay buffer solution containing human TSH (final concentration 50 ng/mL, R&D Systems, Inc.) was added, and incubated at 37°C for 1 hour. The supernatant was removed, a Lysis and Detection Buffer 2(Cisbio) was added, and the mixture was incubated at room temperature for 1 hour to prepare a cell lysate. According to the instruction manual of a cAMP Gs HiRange kit (Cisbio), the cell lysate, d2-labeled cAMP and anti-cAMP Europium Cryptate labeled antibodies (Cisbio) were reacted in a 384-well white microplate. Then, a fluorescence intensity ratio (measurement wavelength 665 nm/620 nm) was measured using a multi-plate reader (PHERAstar FSX, BMG LABTECH, Japan). The fluorescence intensity ratio of each sample was converted to a cAMP content using a standard curve. The cAMP content was converted as a percentage of the control value to calculate the cAMP production rate. The cAMP production rate was plotted against the test compound concentration using Prism (Graph Pad Software Inc.) to calculate the IC₅₀ value. The IC₅₀ of each test compound is shown in the above tables. In the tables, IC₅₀<0.5 µM: A, 0.5 µM≤IC₅₀<3.0 µM: B, 3.0 µM≤IC₅₀<30 µM: C are shown.

As shown in the above tables, it was found that the compound of the present invention had human TSHR antagonist activity.

### [Industrial Applicability]

The compound of the present invention or a pharmacologically acceptable salt thereof are useful as a therapeutic agent for thyroid-related diseases because it has TSHR antagonist activity.

## Claims

1. A compound represented by Formula (I): 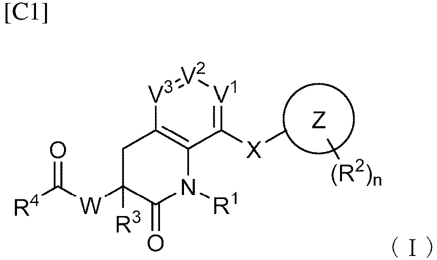 [wherein,
the ring Z is C₆₋₁₀ aryl, 5- or 6-membered heteroaryl, C₃₋₈ cycloalkyl, or 3- to 8-membered heterocycloalkyl;
X is -CHR^{X}- or -O-;
R^{X} is a hydrogen atom or C₁₋₆ alkyl;
V¹ is =CR^{V1}- or =N-;
V² is =CR^{V2}- or =N-;
V³ is =CR^{V3}- or =N-;
R^{V1}, R^{V2}, and R^{V3} are each independently a hydrogen atom, a halogen atom, a hydroxy group, C₁₋₆ alkyl, C₁₋₆ alkoxy, halo C₁₋₆ alkyl, hydroxy C₁₋₆ alkyl, C₆₋₁₀ aryl C₁₋₆ alkyl, or C₆₋₁₀ aryl C₁₋₆ alkoxy;
W is -CH₂- or -NH-;
R¹ is a hydrogen atom or C₁₋₆ alkyl;
R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
n is an integer of 0 to 3;
when n is 2 or 3, respective R²s may be the same as or different from each other;
R³ is a hydrogen atom, a halogen atom or C₁₋₆ alkyl;
R⁴ is C₁₋₆ alkyl, halo C₁₋₆ alkyl, -NR⁵R^{5'}, or C₃₋₈ cycloalkyl; and
R⁵ and R^{5'} are each independently a hydrogen atom or C₁₋₆ alkyl]
or a pharmacologically acceptable salt thereof.

2. The compound according to claim 1, which is represented by Formula (II): 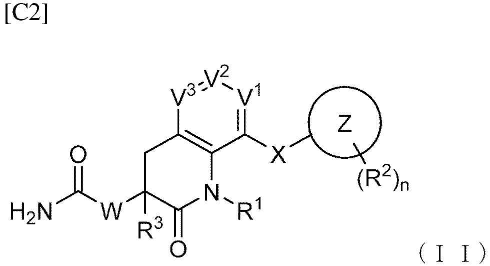 [wherein,
the ring Z is C₆₋₁₀ aryl, 5- or 6-membered heteroaryl, C₃₋₈ cycloalkyl, or 3- to 8-membered heterocycloalkyl;
X is -CHR^{X}- or -O-;
R^{X} is a hydrogen atom or C₁₋₆ alkyl;
V¹ is =CR^{V1}- or =N-;
V² is =CR^{V2}- or =N-;
V³ is =CR^{V3}- or =N-;
R^{V1}, R^{V2}, and R^{V3} are each independently a hydrogen atom, a halogen atom, a hydroxy group, C₁₋₆ alkyl, C₁₋₆ alkoxy, halo C₁₋₆ alkyl, C₆₋₁₀ aryl C₁₋₆ alkyl, or C₆₋₁₀ aryl C₁₋₆ alkoxy;
W is -CH₂- or -NH-;
R¹ is a hydrogen atom or C₁₋₆ alkyl;
R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
n is an integer of 0 to 3;
when n is 2 or 3, respective R²s may be the same as or different from each other; and
R³ is a hydrogen atom, a halogen atom, or C₁₋₆ alkyl]
or a pharmacologically acceptable salt thereof.

3. The compound according to claim 2:
wherein V¹ is =CR^{V1}-;
V² is =CR^{V2}-;
V³ is =CR^{V3}-; and
R^{V1}, R^{V2} and R^{V3} have the same meanings as in claim 2;
or a pharmacologically acceptable salt thereof.

4. The compound according to claim 3:
wherein the ring Z is C₆₋₁₀ aryl;
or a pharmacologically acceptable salt thereof.

5. The compound according to claim 2, which is represented by Formula (III): 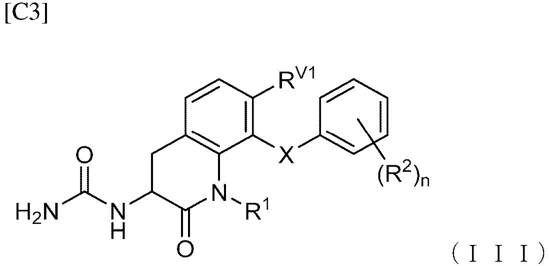 [wherein,
X is -CHR^{X}- or -O-;
R^{X} is a hydrogen atom or C₁₋₆ alkyl;
R^{V1} is a hydrogen atom, a halogen atom, a hydroxy group, a C₁₋₆ alkyl, C₁₋₆ alkoxy, halo C₁₋₆ alkyl, C₆₋₁₀ aryl C₁₋₆ alkyl, or C₆₋₁₀ aryl C₁₋₆ alkoxy;
R¹ is a hydrogen atom or C₁₋₆ alkyl;
R² is a halogen atom, a cyano group, a hydroxy group, an amino group, C₁₋₆ alkyl, halo C₁₋₆ alkyl, C₁₋₆ alkoxy, halo C₁₋₆ alkoxy, C₆₋₁₀ aryl C₁₋₆ alkyl or C₆₋₁₀ aryl C₁₋₆ alkoxy;
n is an integer of 0 to 3; and
when n is 2 or 3, respective R²s may be the same as or different from each other]
or a pharmacologically acceptable salt thereof.

6. The compound according to claim 5:
wherein R^{V1} is a hydrogen atom, a halogen atom, a hydroxy group, C₁₋₆ alkyl, C₁₋₆ alkoxy, or halo C₁₋₆ alkyl;
R¹ is C₁₋₆ alkyl; and
R² is a halogen atom, C₁₋₆ alkyl, halo C₁₋₆ alkyl, C₁₋₆ alkoxy or halo C₁₋₆ alkoxy;
or a pharmacologically acceptable salt thereof.

7. The compound according to claim 1, which is represented by Formula (IV): 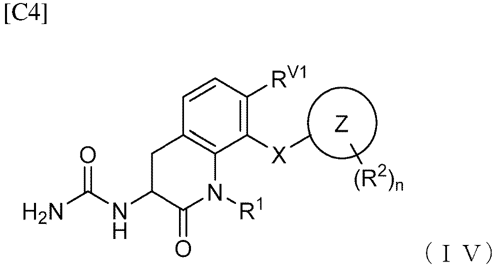 [wherein,
the ring Z is C₆₋₁₀ aryl, 5- or 6-membered heteroaryl or C₃₋₈ cycloalkyl;
X is -CHR^{X}- or -O-;
R^{X} is a hydrogen atom or C₁₋₆ alkyl;
R^{V1} is a hydrogen atom, a halogen atom, a hydroxy group, C₁₋₆ alkyl, halo C₁₋₆ alkyl or C₆₋₁₀ aryl C₁₋₆ alkoxy;
R¹ is C₁₋₆ alkyl;
R² is a halogen atom, a cyano group, C₁₋₆ alkyl, halo C₁₋₆ alkyl, C₁₋₆ alkoxy or halo C₁₋₆ alkoxy;
n is an integer of 0 to 3; and
when n is 2 or 3, respective R²s may be the same as or different from each other]
or a pharmacologically acceptable salt thereof.

8. The compound according to claim 7:
wherein the ring Z is C₆₋₁₀ aryl;
or a pharmacologically acceptable salt thereof.

9. The compound according to claim 8:
wherein X is -O-;
or a pharmacologically acceptable salt thereof.

10. A compound selected from the group consisting of the following compounds: 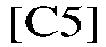 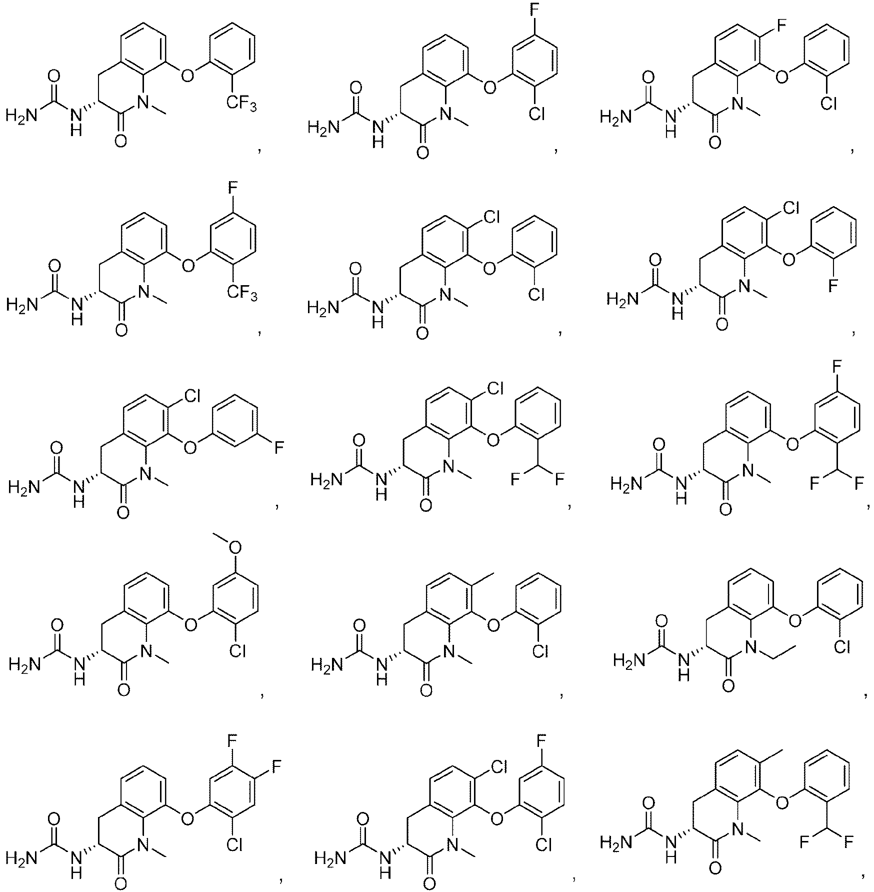 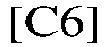 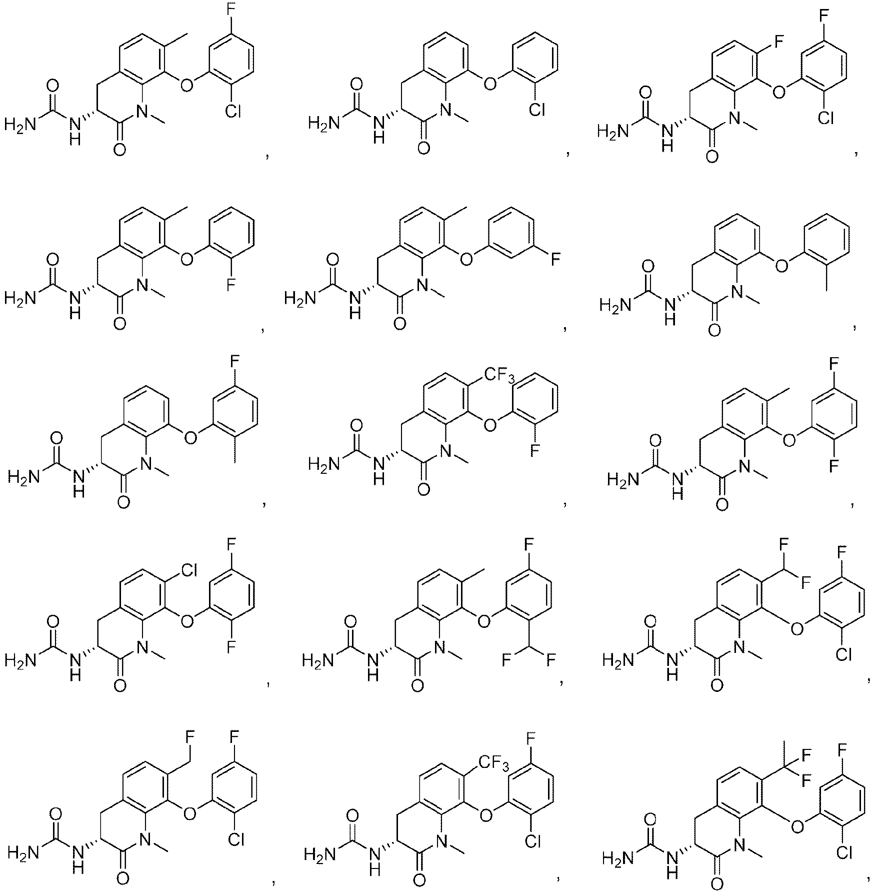 and 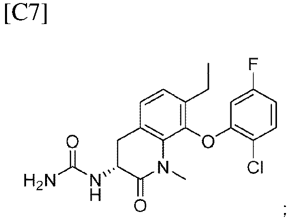 or a pharmacologically acceptable salt thereof.

11. The compound according to claim 8:
wherein X is -CHR^{X}-;
R^{X} has the same meaning as in claim 7;
or a pharmacologically acceptable salt thereof.

12. A compound selected from the group consisting of the following compounds: 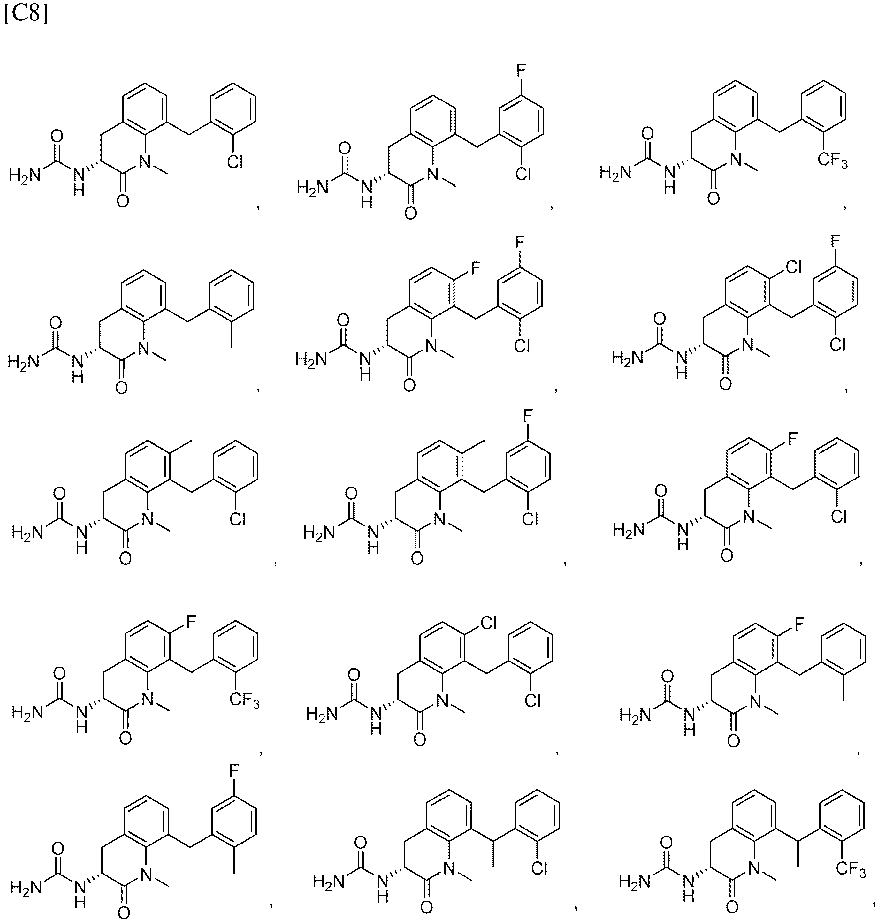 and 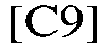 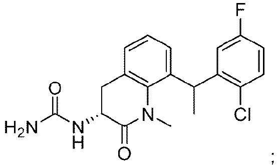 or a pharmacologically acceptable salt thereof.

13. A pharmaceutical composition comprising the compound according to any one of claims 1 to 12 or a pharmacologically acceptable salt thereof, and a pharmaceutical additive.

14. The pharmaceutical composition according to claim 13, which is a pharmaceutical composition for treating thyroid-related diseases.

15. The pharmaceutical composition according to claim 14,
wherein the thyroid-related diseases are hyperthyroidism, Graves' disease, or thyroid eye disease.
